# EUROPEAN PATENT APPLICATION

(11) **EP 4 678 144 A2**
(43) Date of publication of application: **14.01.2026**
(21) Application number: 25210104.3
(22) Date of filing: 04.01.2019
(51) Int. Cl.: A61F 2/24

(54) **HEART VALVE PROSTHESIS AND DELIVERY**

(30) Priority: 07.01.2018 US 201862614489 P; 06.11.2018 US 201862756556 P; 18.12.2018 US 201862781537 P
(62) Divisional of application: 19736146.2
(71) Applicant: JC Medical, Inc., Burlingame, CA 94010 (US)
(72) Inventor: ZHANG, Ji, Burnaby, V3N 5A7 (US); YANG, Cheng Yong, Foster City, 94404 (US); ZHU, Jinhua, San Francisco, 94110 (US); MCMAHON, Dennis Michael, Windsor, 95492 (US); WALSH, Brandon G., Kaysville, 84037 (US)
(74) Representative: Cohausz & Florack

(57) **Abstract**

A delivery system for delivering a prosthetic heart valve prosthesis to a native valve sinus of a patient, the valve prosthesis comprising a mesh frame and a sinus locator, the delivery system comprising: a grasper configured to be coupled to a proximal portion of the sinus locator of the valve prosthesis; and a distal sheath configured to receive a distal portion of the mesh frame, the distal sheath having a proximal edge that overlaps the mesh frame; and a proximal sheath configured to receive a proximal portion of the mesh frame, the proximal sheath having a distal edge that overlaps the mesh frame and that is spaced apart from the proximal edge of the distal sheath to form a longitudinal gap that permits a section of the mesh frame to be exposed therethrough between the proximal and distal edges, wherein the delivery system supports the prosthesis in a delivery configuration wherein (i) the sinus locator is engaged with the grasper and (ii) the proximal and distal sheaths support the mesh frame in a collapsed state with a medial portion of the mesh frame being exposed through the longitudinal gap, the delivery system being configured to permit expansion of the sinus locator and to subsequently permit expansion of the mesh frame within the sinus locator upon separation of the proximal sheath from the distal sheath, the grasper being configured to disengage from the proximal portion of the sinus locator for releasing the valve prosthesis.

## Description

### CROSS-REFERENCE TO RELATED APPLICATIONS

The present application claims the benefit of and priority to U.S. Provisional Application No. 62/614,489, filed on January 7, 2018, U.S. Provisional Application No. 62/756,556, filed on November 6, 2018, and U.S. Provisional Application No. 62/781,537, filed on December 18, 2018, the entireties of each of which are incorporated herein by reference.

### TECHNICAL FIELD

The present disclosure relates to devices and methods for the percutaneous delivery and implantation of a cardiac valve prosthesis. The valve prosthesis can be delivered in a compressed state within a sheath to the defective native valve and released in situ.

### BACKGROUND

Prosthetic heart valves are used to replace damaged or diseased heart valves. In vertebrate animals, the heart is a muscular organ with four pumping chambers: the left and right atria and the left and right ventricles, each provided with its own one-way valve. The natural heart valves are identified as the aortic, mitral (or bicuspid), tricuspid and pulmonary valves. Prosthetic heart valves can be used to replace any of these naturally occurring valves, although repair or replacement of the aortic or mitral valves is more common since they reside in the left side of the heart where pressures are the greatest.

A conventional heart valve replacement surgery involves accessing the heart in the patient's thoracic cavity through a longitudinal incision in the chest. For example, a median sternotomy requires cutting through the sternum and forcing the two opposing halves of the rib cage to be spread apart, allowing access to the thoracic cavity and heart within. The patient is then placed on cardiopulmonary bypass which involves stopping the heart to permit access to the internal chambers. Such open-heart surgery is particularly invasive and involves a lengthy and difficult recovery period.

The foregoing examples of the related art and limitations related therewith are intended to be illustrative and not exclusive. Other limitations of the related art will become apparent to those of skill in the art upon a reading of the specification and a study of the drawings.

### SUMMARY

The present disclosure relates to heart valve prostheses, delivery devices, and actuation handles that can facilitate delivery of a heart valve prosthesis to a defective native valve structure in a patient, such as the aortic valve. In some embodiments, the delivery can be performed using a transcatheter approach.

The delivery devices and actuation handles can enable a clinician to more easily maneuver and advance the delivery device through blood vessels leading to the heart, as well as through tortuosities of such vessels, using a transvascular approach, such as a transfemoral approach. Indeed, some embodiments disclosed herein enable components of the heart valve prosthesis to be advanced in tandem, as an axially displaced unit (with or without partial or full overlapping between the components), while still being movably connected, movably attached, flexibly connected, displaceably connected, linked, or coupled to each other, thereby minimizing a passing profile or cross section of the delivery device. Optionally, the distance from which the components of the heart valve prosthesis may be serially displaced may be variable, such that various components are adjacent or potentially inches or feet away. Further, the interconnection of components of the heart valve prosthesis can allow different degrees of motion and can be set into an engaged or retained position that provides a limited range of motion. In some embodiments, the engaged position can also provide a preset relative positioning of the components of the heart valve prosthesis to facilitate proper placement and release of the heart valve prosthesis. Additionally, some embodiments can provide a clinician with a high degree of control and enhance the maneuverability of the heart valve prosthesis when implanting the heart valve prosthesis at the target location.

In accordance with some embodiments, a procedure is provided for a transcatheter aortic valve implantation (TAVI) and/or a transcatheter aortic valve replacement (TAVR). For example, in the TAVI procedure, a clinician can anchor the anchoring component of the heart valve prosthesis relative to the aortic valve annulus to guide the placement of the prosthetic leaflet structure. The valve prosthesis can comprise prosthetic leaflets, an anchoring component, a valve frame component, and a tethering component, which allows the anchoring component and the frame component to be placed serially in a delivery device in order to reduce the overall crossing profile of the delivery device. The tethering component can be coupled to the anchoring component and the frame component to permit a range of motion and in some embodiments, to restrict other motion. The tethering component can be slidable relative to the anchoring component between a released position and a retained position. In the retained position, the tethering component can allow relative movement of the valve frame component and a preset or predetermined position which the valve frame component is optimally located relative to the anchoring component, which can facilitate placement and release of the valve prosthesis.

For example, in some embodiments, the interconnection can be implemented using a novel approach of looping the tethering component around "U-shaped" members of the anchoring component. The tethering component can slide along the anchoring component until reaching the end of the travel on the anchoring component. The clinician can exert tension on the tethering component until the tethering component is seated in the engagement area. This action can ratchet the tethering component and engage it to the engagement area of the anchoring component. Thereafter, the tethering component establishes a fixed range of longitudinal travel of the valve frame component relative to the anchoring component, and subsequently a proper position of the valve frame component in the anatomy, based only on the clinician placing the anchoring component into the aortic sinus region (the clinician can see under fluoroscopy and can "feel" the placement).

Thus, some embodiments disclosed herein advantageously provide a delivery device that has a reduced passing profile or cross section, thereby enabling delivery of a heart valve prosthesis in a safer, less invasive manner than traditional approaches. As such, open-heart surgery can be avoided because the heart valve prosthesis can be advanced to the heart using a catheter via an access point in the blood vessel, such as the femoral artery. This provides enormous benefits to patients, including less trauma to the patient, greater ease of recovery, and potentially fewer surgical risks, to name a few.

Further, although the in-series arrangement of the anchoring component and the valve frame component overcomes the challenge of creating a low-profile delivery device, the advantageous arrangement of the interconnection overcomes yet another critical challenge: how to optimally position the valve prosthesis within the native valve structure and to reliably anchor it in place. Indeed, some embodiments disclosed herein address this challenge and teach structures and methods for using a tethering component to operatively couple the anchoring component to the valve frame component in a delivery device.

The delivery device can comprise a proximal sheath that can house at least a portion of the anchoring component and a distal carrier assembly that can house at least a portion of the valve frame component. The tethering component can extend between the anchoring component and the valve frame component when the valve prosthesis is loaded onto the delivery device. The valve prosthesis can be released from the delivery device in a component-by-component manner that allows the clinician to maneuver and position the anchoring component first, followed by the valve frame component.

In some embodiments, the anchoring component can be coupled to an engagement member or grasper of the delivery device that allows the clinician to push or pull the anchoring component. The grasper can be released from engagement with the anchoring component when the anchoring component is properly seated relative to the native valve annulus.

In addition, in some embodiments, the distal carrier assembly of the delivery device can comprise two components or be referred to as a two-part nose cone assembly. In accordance with some embodiments is the realization that if a single tubular member or nose cone is used to sheath most of the valve frame component, various problems can arise due to the expansive force and corresponding compressive force required to maintain the valve frame component in its compressed configuration during delivery to a target valve structure. Because the delivery device can be quite long (for example, in some embodiments, up to about 4 to 6 feet or more, although the length can be less than 4, 3, or 2 feet), these forces can create a much stiffer distal section of the delivery device. Further, these forces can require a high degree of longitudinal force to release the valve frame component due to the high frictional forces due to the radial force of the valve implant

Thus, the radial and frictional forces of such configurations can cause problems of matching handle actuation and make precise positioning of the distal end of the delivery device quite difficult. For example, the friction tends to be a variable friction that makes it difficult for a clinician to position the components of the valve prosthesis relative to each other, which can lead to unpredictable and/or imprecise component positioning or deployment. Thus, some embodiments herein include the realization that by separating the distal carrier or nose cone assembly into two components (such as a proximal and distal enclosure), the components can cover less surface area of the valve frame component, thus reducing the radial forces exerted on a single component and the resultant friction that would need to be overcome in order to actuate or release the valve frame component. As such, the problems associated with a single tubular member are much more manageable.

Additionally, in some embodiments, a two-part distal carrier assembly can also enable the clinician to release the valve frame component in an advantageous sequence. For example, during testing and development of the valve prostheses, deployment systems, and handle actuators disclosed herein, some embodiments demonstrate advantageous characteristics by permitting a distal end portion of the valve frame component to open first, before a proximal end portion of the valve frame component is released. In some embodiments, the valve frame component can have one or more anchors at its distal end portion that can supplement the outward expansive force (due to self-expansion of the valve frame component) and its resultant frictional engagement. By opening the distal end portion first (by actuation of distal nose cone or enclosure), the distal end portion can "flower" out and engage with the native valve structure to secure a longitudinal position of the valve frame component relative to the native valve structure. Thereafter, the self-expanding radial outward force of the valve frame component can cause the proximal end portion of the valve frame component to become disengaged and released from the proximal nose cone or enclosure.

Some embodiments can also provide self-aligning features to allow the components of the delivery assembly to be moved from a releasing state (where the components of the valve prosthesis are released from engagement with the delivery assembly) to a nested or stowed state in which outer surfaces of portions of the delivery assembly are aligned or in an abutting position at a seam. This alignment, abutment, or positioning can provide a smoother outer profile that can reduce the likelihood of having the delivery assembly snag or become entangled with the prosthetic valve after being released or with other vasculature as the delivery assembly is retrieved from the patient's vasculature.

For example, in some embodiments, the distal carrier or nose cone assembly can include an internal plunger or piston mechanism. The plunger mechanism can be compressed when the valve frame component is loaded into the delivery device. As the valve frame component is released, a spring of the plunger mechanism can push a plunger head to a predetermined position relative to the distal carrier assembly. In accordance with some embodiments, in the predetermined position, the plunger head can be exposed partially from the distal enclosure and be configured to engage with the proximal enclosure to align the proximal and distal enclosures relative to each other in an abutting relationship. The plunger head can therefore engage with both the proximal and distal enclosures to reduce the likelihood of catching or snagging of the delivery device with the prosthetic valve or other vasculature during retrieval of the delivery device. Additionally, such features can also aid in proximal retraction of the delivery device into an introducer sheath. Moreover, the plunger head can also provide a proximal surface that can be in contact with the distal end portion of the valve frame component and not catch or snag with the intricate mesh of the valve frame component, thereby ensuring that the valve frame component can flower open without catching on the delivery device. Accordingly, some embodiments can include one or more of these advantageous features that address the problem of having the valve prosthesis and/or the delivery device catch or snag on each other or surrounding anatomy.

Furthermore, due to the reduced cross-sectional profile of the delivery device, retrograde delivery of a valve prosthesis through the blood vessel (such as femoral artery in a transfemoral retrograde approach) can be possible with reduced risk of trauma to the surrounding vasculature. For example, retrograde delivery of the valve prosthesis through the femoral artery has been associated with aortofemoral artery injury and/or rupture, and carries a potential risk of stroke as the delivery involves crossing the aortic arch. However, the various features and advantages achieved using some embodiments disclosed herein provide a valve prosthesis and delivery device that minimizes damage along the delivery path of device while also minimizing the invasive nature of the implantation procedure.

Additional embodiments of the present devices and methods, and the like, will be apparent from the following description, drawings, examples, and claims. As can be appreciated from the foregoing and following description, each and every feature described herein, and each and every combination of two or more of such features, is included within the scope of the present disclosure provided that the features included in such a combination are not mutually inconsistent. In addition, any feature or combination of features may be specifically excluded or omitted from any embodiment of the present disclosure. Additional aspects and advantages of the present disclosure are set forth in the following description and claims, particularly when considered in conjunction with the accompanying examples and drawings.

Additional features and advantages of the subject technology will be set forth in the description below, and in part will be apparent from the description, or may be learned by practice of the subject technology. The advantages of the subject technology will be realized and attained by the structure particularly pointed out in the written description and embodiments hereof as well as the appended drawings.

Certain features of valve prostheses, delivery devices, actuation handles, other devices, systems, and methods which can be implemented with the valve prostheses, delivery devices, actuation handles, other devices, systems, and methods discussed in the present disclosure, can implement features of and/or be used in combination with other features of valve prostheses, delivery devices, actuation handles, other devices, systems, and methods described for example in International Application No. (Docket No.: 122271-5044), entitled HEART VALVE PROSTHESIS, filed on January 4, 2019, by Ji Zhang, Brandon G. Walsh, Cheng Yong Yang, Jinhua Zhu, and Dennis Michael McMahon, and in International Application No. (Docket No.: 122271-5048), entitled PROSTHETIC HEART VALVE DELIVERY SYSTEM, filed on January 4, 2019, by Ji Zhang, Brandon G. Walsh, and Cheng Yong Yang, the entirety of each of which is incorporated herein by reference.

It is to be understood that both the foregoing general description and the following detailed description are exemplary and explanatory and are intended to provide further explanation of the subject technology.

### BRIEF DESCRIPTION OF THE DRAWINGS

Various features of illustrative embodiments of the inventions are described below with reference to the drawings. The illustrated embodiments are intended to illustrate, but not to limit, the inventions The drawings contain the following figures:
Figure 1 shows a valve prosthesis, according to some embodiments.
Figures 2A-2H are views of valve anchors, according to some embodiments.
Figures 3A-3C are enlarged views of progressive movement of a link mechanism along an engagement area of a valve prosthesis, moving from a position in which the link mechanism is coupled to a tension member to a position in which the link mechanism is retained in the engagement area of the valve prosthesis, according to some embodiments.
Figures 4A-4G show different embodiments of a link mechanism for the valve prosthesis of Figure 1, in which the valve prosthesis is loaded onto a valve delivery device, according to some embodiments.
Figure 5 is a side cross-sectional view of the valve prosthesis loaded onto a valve delivery device, taken along section lines 5-5 of Figure 4A, according to some embodiments.
Figure 6A is a side view of the valve prosthesis and delivery device of Figure 4A, showing a proximal sheath retracted from over a valve anchor, according to some embodiments.
Figure 6B is an enlarged detail view of the valve prosthesis and delivery device of Figure 6A, according to some embodiments.
Figure 6C is an enlarged detail view of the valve prosthesis and delivery device of Figure 4B, showing a proximal sheath retracted from over a valve anchor, according to some embodiments.
Figure 6D is an enlarged detail view of a valve prosthesis having a valve anchor with a link motion limiter, according to some embodiments.
Figure 6E is an enlarged detail view of another valve prosthesis having a valve anchor with another link motion limiter, according to some embodiments.
Figures 7A-7D are views of a valve delivery device having a grasper mechanism for engaging a valve anchor, according to some embodiments.
Figures 7E and 7F are side and top views of a grasper mechanism, according to some embodiments.
Figures 7G and 7H are views of a valve delivery device having a grasper mechanism with a grasper alignment hub, according to some embodiments.
Figure 7I is a view of the grasper alignment hub assembly of Figure 7H, according to some embodiments.
Figures 7J and 7K are views of a valve delivery device having a grasper mechanism for engaging a valve anchor, according to some embodiments.
Figures 8A-8D are side and end cross-sectional views illustrating aspects and operation of a distal carrier assembly and a proximal enclosure of a delivery device, according to some embodiments.
Figure 8E is a cross-sectional view taken along section lines 8E-8E of Figure 8D illustrating a structure of a proximal enclosure of the distal carrier assembly of the delivery device of Figure 4A, according to some embodiments.
Figure 9A is a perspective view of a nose cone protector, according to some embodiments.
Figures 9B-9F are side cross-sectional views illustrating operation of a distal carrier assembly of the delivery device with the nose cone protector of Figure 9A, according to some embodiments.
Figures 10A and 10B are perspective views illustrating a structure of a proximal enclosure of the delivery device of Figure 4A, according to some embodiments.

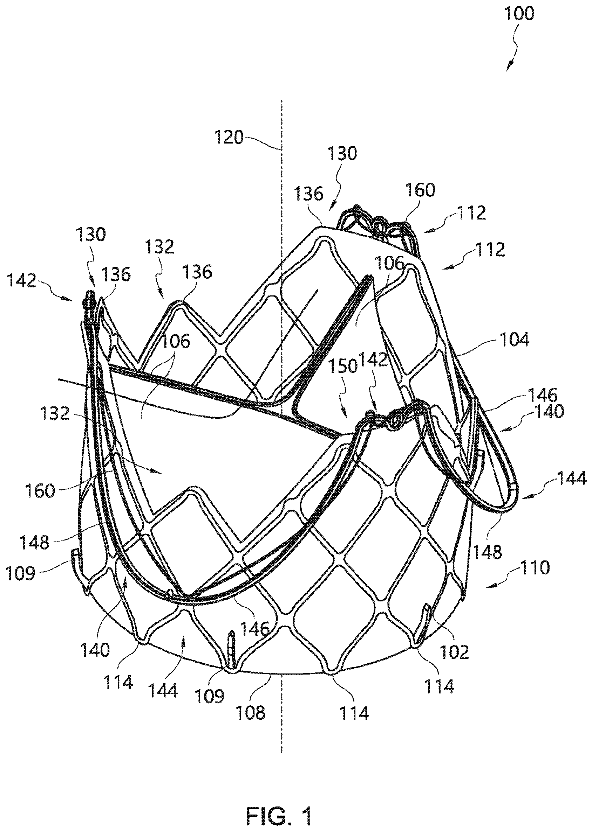

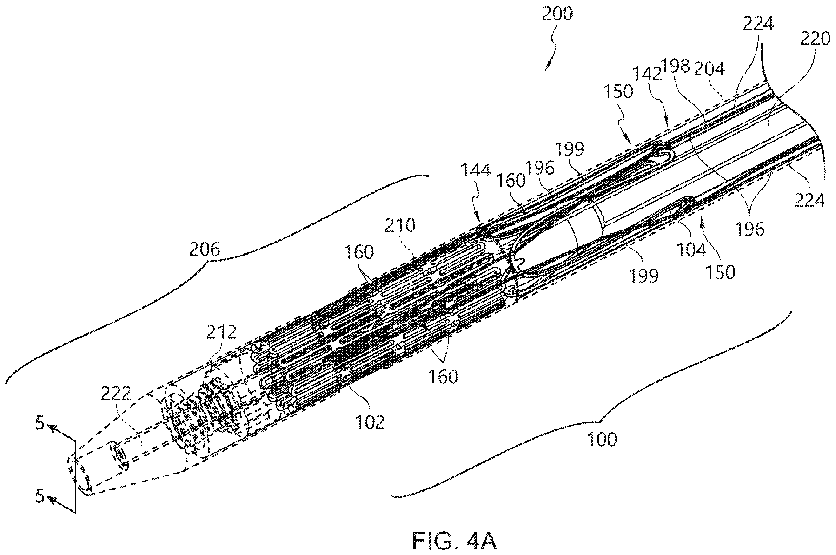

Figures 1 1A-11F illustrate delivery stages of a method for delivering the valve prosthesis of Figure 1 using the delivery device of Figure 4A using a transfemoral retrograde approach, according to some embodiments.

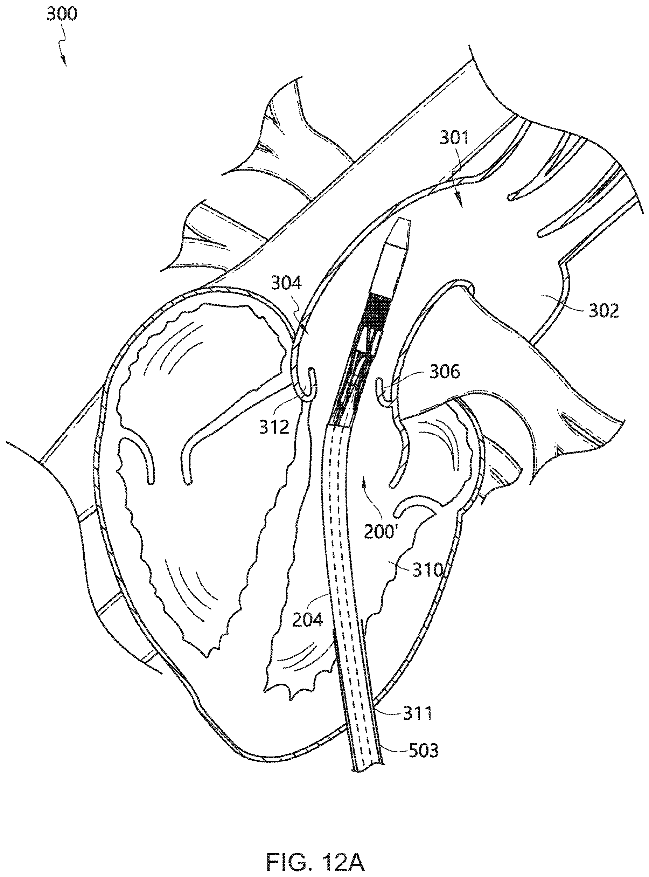

Figures 12A-12F illustrate delivery stages of a method for delivering the valve prosthesis of Figure 1 using the delivery device of Figure 4A using a transapical antegrade approach, according to some embodiments.

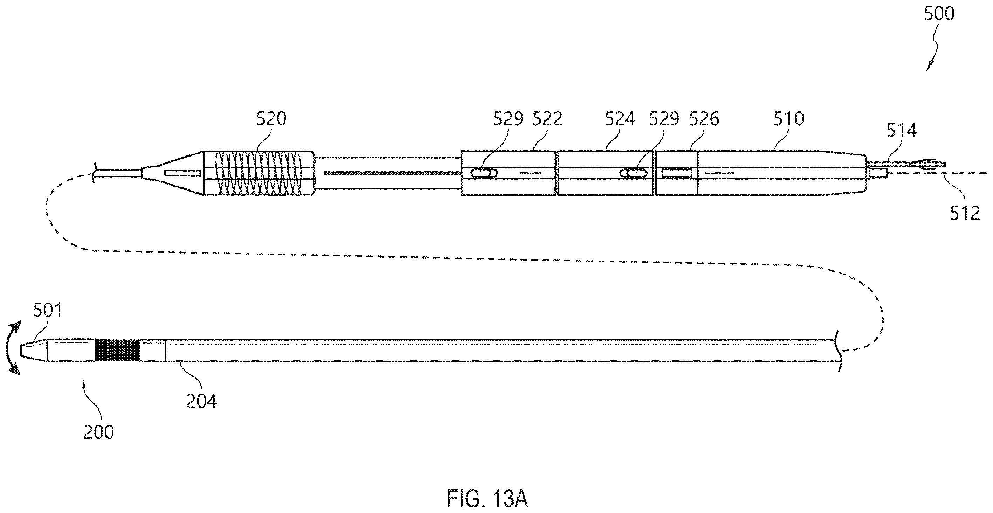

Figures 13A-13H illustrate operation of a handle actuator to control aspects of a valve delivery device, according to some embodiments.

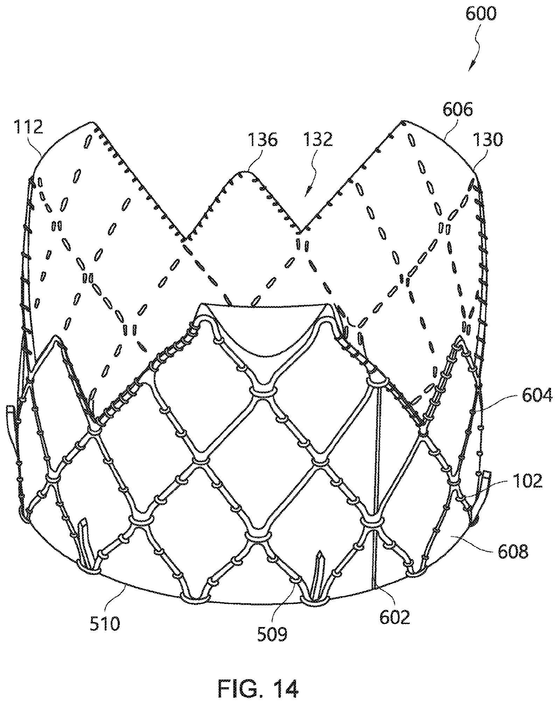

Figure 14 shows a frame component of a valve prosthesis, according to some embodiments.

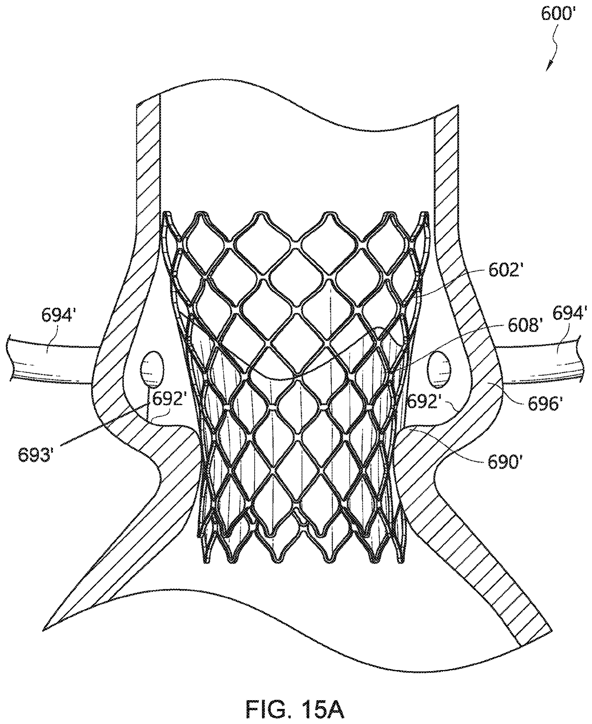

Figure 15A shows a prior art valve prosthesis and its typical blockage of coronary ostia.

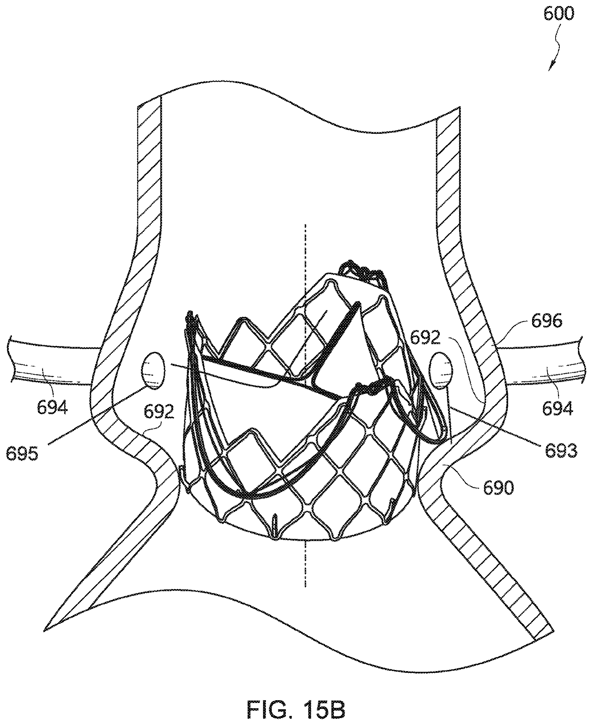

Figure 15B shows the valve prosthesis of Figure 14 positioned relative to a native valve structure, which can advantageously permit blood flow through one or more coronary ostia, according to some embodiments.

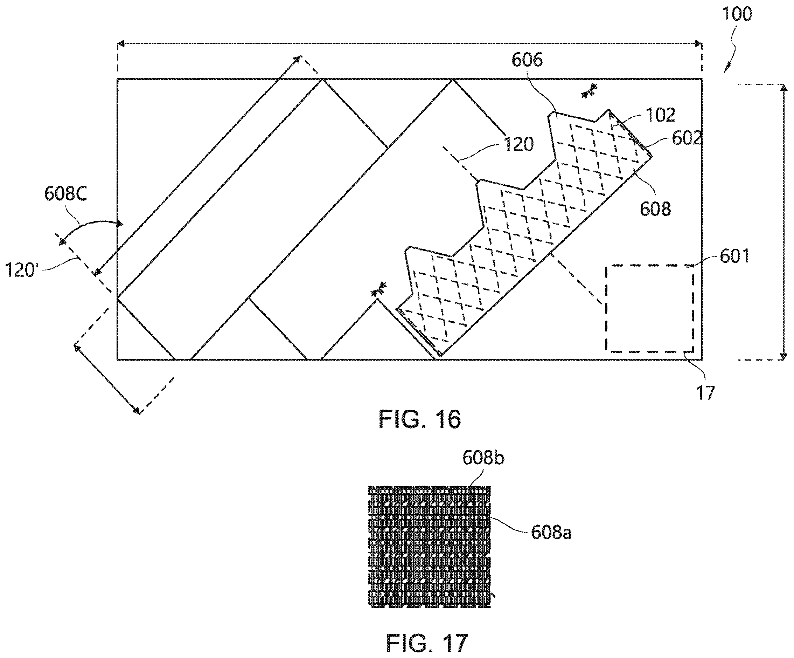

Figure 16 illustrates aspects of a method for forming the membrane of the valve prosthesis, according to some embodiments.
Figure 17 is an enlarged view of a portion of the membrane fabric of Figure 16.

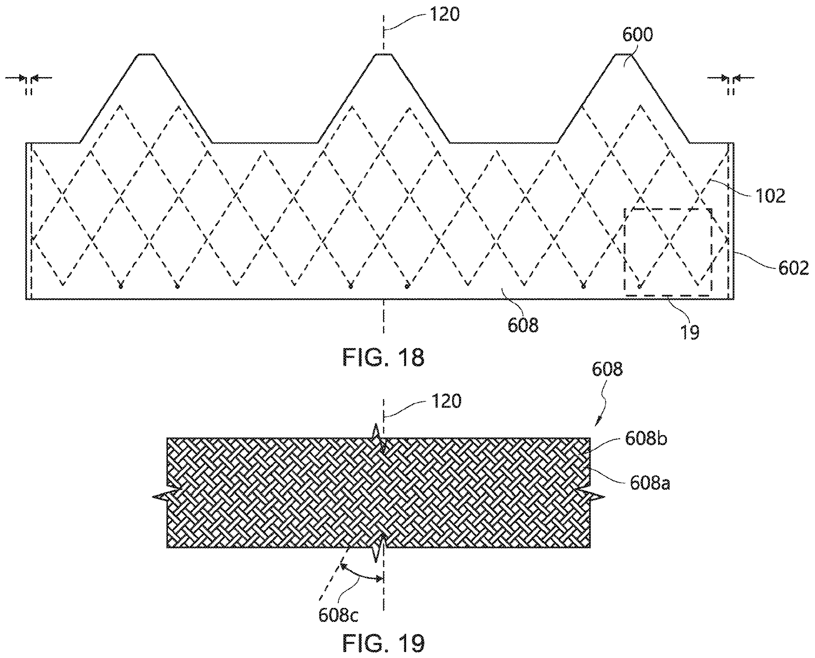

Figure 18 illustrates the membrane of the valve prosthesis of Figure 14, according to some embodiments.
Figure 19 is an enlarged view of a portion of the membrane of Figure 18, according to some embodiments.

### DETAILED DESCRIPTION

In the following detailed description, numerous specific details are set forth to provide a full understanding of the subject technology. It should be understood that the subject technology may be practiced without some of these specific details. In other instances, well-known structures and techniques have not been shown in detail so as not to obscure the subject technology.

Further, while the present disclosure sets forth specific details of various embodiments, it will be appreciated that the description is illustrative only and should not be construed in any way as limiting. Additionally, it is contemplated that although particular embodiments of the present disclosure may be disclosed or shown in the context of aortic valve prostheses, such embodiments may be used in other cardiac valve prosthesis applications. Furthermore, various applications of such embodiments and modifications thereto, which may occur to those who are skilled in the art, are also encompassed by the general concepts described herein.

Various embodiments will now be described more fully hereinafter. Such embodiments may, however, be embodied in many different forms and should not be construed as limited to the embodiments set forth herein; rather, these embodiments are provided so that this disclosure will be thorough and complete, and will fully convey its scope to those skilled in the art. Thus, one or more features shown or otherwise disclosed in an embodiment herein may be interchangeably used or incorporated into another embodiment that may not expressly show or disclose such feature(s). Further, one or more features shown or otherwise disclosed for an embodiment herein may be excluded from such embodiment, unless expressly indicated, using skill in the art.

As with all cardiac valves, a healthy aortic valve will open to allow blood flow and close to prevent backflow of blood. However, disease and dysfunction of the valve can result in regurgitation or decreased blood flow (stenosis). In such cases, a replacement aortic valve prosthesis must be used to perform the functions of a healthy aortic valve.

Minimally invasive surgical techniques are evolving, where a valve prosthesis can be introduced into a patient using a catheter that is introduced via a small incision that provides access to, for example, a femoral artery or directly to the heart. These implantation techniques have shown promising results in providing treatment options for patients who are poor open surgical candidates. Nevertheless, challenges still remain in such catheter-based delivery of prosthetic valves.

For example, in according with an aspect of at least one embodiment disclosed herein is the realization that advancing a conventional tubular delivery device through a vessel exerts stress against the vessel walls and carries the risk of damaging the vessel walls. Further, in according with an aspect of at least one embodiment disclosed herein is the realization that transcatheter prosthetic valves may not be able to treat patients with aortic regurgitation. Additionally, in according with an aspect of at least one embodiment disclosed herein is the realization that conventional prosthetic valves may be difficult to position, may require rapid ventricular pacing, and may have limited expansion. Accordingly, implantation and use of conventional prosthetic valves may result in complications, such as vascular damage, moderate to severe paravalvular leakage, valve thrombosis/migration, coronary artery blockage, and excessive stress due to excessive radial force.

The present disclosure describes various aspects of heart valve prostheses that can be delivered to a defective heart valve in a patient. The valve prostheses can comprise at least one valve anchor or clasper, which is movably connected, movably attached, flexibly connected, displaceably connected, linked, or coupled to a radially-expandable valve support or frame. The valve frame can comprise prosthetic valve leaflets or cusps and provide the functionality of the native heart valve. Certain features of valve prostheses, which can be implemented with the prostheses discussed in the present disclosure, are also further described for example, in U.S. Pat. No. 8,366,768, the entirety of which is incorporated herein by reference.

Thus, the present disclosure provides a variety of features that can be optionally incorporated or excluded from any of the embodiments explicitly discussed or illustrated herein. These modifications and combinations of features can be performed by a person of skill to achieve advantages and benefits discussed herein. Further, certain modifications or combinations are indicated or suggested herein, but it is contemplated that a person skill can implement or exclude certain aspects or features disclosed herein in developing a suitable embodiment or implementation of these teachings. Advantageously, various embodiments described herein allow for treating patients with aortic regurgitation, permit precise axial, angular, and radial positioning of the valve prosthesis, minimize valve migration and paravalvular leakage while avoiding damage to the valve annulus, minimize the need for a pacemaker, and decrease the likelihood of blocking the coronary artery.

Referring now to Figures 1-3, a valve prosthesis 100 and components thereof are shown in various configurations. The valve prosthesis 100 can be delivered to a patient using a suitable delivery device, including embodiments of the delivery devices disclosed herein. The valve prosthesis 100 can comprise a support frame 102 and an anchoring component or valve anchor 104 to which the support frame 102 is movably connected, movably attached, flexibly connected, displaceably connected, linked, or coupled.

The valve prosthesis 100 can be configured such that components of the valve prosthesis 100 to be advanced in series while still being movably connected, movably attached, flexibly connected, displaceably connected, linked, or coupled to each other, thereby minimizing a passing profile or cross section of the delivery system. The interconnection of components of the valve prosthesis 100 can allow different degrees of motion and can be set into an engaged or retained position that provides a limited range of motion. In some embodiments, the engaged position can also provide a preset relative positioning of the components of the valve prosthesis 100 to facilitate proper placement and release of the valve prosthesis 100. Additionally, some embodiments can provide a clinician with a high degree of control and enhance the maneuverability of the valve prosthesis 100 when implanting the valve prosthesis 100 at the target location.

In some embodiments, the valve anchor 104 can be coupled to the support frame 102 when the support frame 102 is in the compact configuration prior to delivery and expansion. In some embodiments, the valve anchor 104 is not fixed to the support frame 102. Further, the valve anchor 104 can be separate from the support frame 102 or formed separately from and later coupled to the support frame 102. Thus, although a least a portion of the valve anchor 104, e.g., the anchoring leg, may be in contact with or otherwise reversibly attached or connected to the support frame 102, no part of the valve anchor 104 is fixed, e.g., welded or otherwise irreversibly adhered, to the support frame 102. Alternatively stated, the valve anchor 104, which may be in contact with or otherwise reversibly attached to the support frame 102, is not irreversibly fixed to the support frame 102.

Further, upon reaching the target location, the valve anchor 104 can be movably coupled to the support frame 102 in a manner that prevents the entire valve anchor 104 from being radially displaced from the support frame 102 when the valve anchor 104 is initially expanded. For example, portions of the valve anchor 104 can be radially displaced from the support frame during initial "landing" of the valve anchor 104 against the native valve structure at the target location. In some embodiments, the support frame 102 can be deployed or expanded within the native heart valve structure, and the valve anchor 104 can become sandwiched between the support frame and the native valve tissue, becoming at least partially, and possibly fully, immobilized. The valve anchor 104 can function to hold the expanded support frame 102 in place within the native valve structure.

Optionally, the support frame 102 may be referred to as a valve frame or valve support frame. Figure 1 illustrates the support frame 102 aligned with and expanded within the valve anchor 104, in a configuration that is achieved when the prosthesis 100 is released and expanded within the native valve structure. The native valve structure includes the valve annulus or leaflets. This expanded configuration, serves to secure the valve prosthesis 100 within the native valve annulus by engaging the native valve structure. In some embodiments, the expanded configuration of the valve prosthesis 100 may reduce reliance on securing the valve prosthesis 100 with radial force exerted by the support frame 102 and the valve anchor 104 via the sandwiching or compression of the native valve leaflets between the support frame 102 and the valve anchor 104 of the valve prosthesis 100. Further, as discussed further herein, during implantation of the valve prosthesis 100, the support frame 102 and the valve anchor 104 can be movable relative to each other in expanded and/or compressed states in order to facilitate proper positioning of the prosthesis 100 relative to the native valve annulus and surrounding structures. Indeed, various advantages made possible by the prosthesis 100 and delivery device disclosed herein allow a clinician to achieve a higher degree of precision in placing the prosthesis 100, as well as making such increased precision easier to achieve.

Referring to Figure 1, the support frame 102 can comprise an outer or external surface and defines a central orifice about a longitudinal axis 120. The longitudinal axis 120 corresponds to an inflow-outflow axis of the prosthesis 100. In some embodiments, the valve prosthesis 100 further comprises a plurality of prosthetic valve leaflets or cusps 106 that are coupled to the support frame 102. The support frame 102 can provide a structural support for the valve leaflets 106. The valve leaflets 106 can have surfaces defining a reversibly sealable opening for unidirectional flow of a liquid through the prosthesis 100. The prosthesis 100 can include three valve leaflets 106 for a tri-leaflet configuration. As appreciated, mono-leaflet, bi-leaflet, and/or multi-leaflet configurations are also possible. For example, the valve leaflets can be coupled to the support frame 102 to span and control fluid flow through the lumen of the prosthesis 100. The prosthetic leaflets 106 can comprise one or more synthetic materials, engineered biological tissues, biological valvular leaflet tissues, pericardial tissues, cross-linked pericardial tissues, aortic root tissue, chemically or biologically processed/treated tissue, or combinations thereof. In some embodiments, the pericardial tissue is selected from but not limited to the group consisting of bovine, equine, porcine, ovine, human tissue, or combinations thereof.

Furthermore, in some embodiments, the valve prosthesis 100 can comprise a sealing component or membrane 108 that can be attached to an inside surface, an outside surface, and/or enclose the support frame 102, such as by being laminated onto inner and outer surfaces of the support frame 102. Thus, the valve leaflets 106 can be coupled to the support frame 102 and/or the membrane 108. In some embodiments, the membrane 108 can restrict blood flow in areas around the valve leaflets 106 so that blood flow occurs only between the valve leaflets 106 through the lumen of the prosthesis 100, as in a healthy native heart valve.

The support frame 102 and/or the valve anchor 104 can comprise a braided frame, a wire frame, or a laser-cut frame (e.g., laser-cut tubular mesh), as shown in Figure 1. In some embodiments, the support frame 102 and/or the valve anchor 104 can comprise a shape-memory metal, which can change shape at a designated temperature or temperature range or by inducing stress. Alternatively, the self-expanding frames can include those having a spring-bias. The material from which either the support frame 102 and/or the valve anchor 104 is fabricated can allow the support frame 102 and/or the valve anchor 104 to automatically expand to its functional size and shape when deployed but also allows the support frame 102 and/or the valve anchor 104 to be radially compressed to a smaller profile for delivery through the patient's vasculature. Examples of suitable materials for self-expanding components described herein (e.g., support frames, valve anchors, locking members) include, but are not limited to, medical grade nickel titanium alloys, tantalum, platinum alloys, niobium alloys, cobalt alloys, alginate, or combinations thereof. Shape memory alloys having superelastic properties generally made from ratios of nickel and titanium, commonly known as Nitinol, are preferred materials. In some embodiments, self-expanding components described herein can include materials including, but not limited to shape memory plastics, polymers, and thermoplastic materials which are inert in the body. In an alternative embodiment, either the support frame 102 and/or the valve anchor 104 is not self-expanding, and may be expanded, for example, using a balloon catheter as is well known in the art. Examples of suitable materials for components described herein include, but are not limited to, stainless steel and titanium. Optionally, either the support frame 102 and/or the valve anchor 104 can comprise radiopaque materials to allow visualization under fluoroscopy or other imaging techniques.

Optionally, the support frame 102 can comprise one or more hooks 109 that can engage with tissue of the native valve annulus, the aortic root, or any other portion of the native valve when the support frame 102 is expanded within the native valve annulus. The hooks 109 can be engaged with the native valve annulus to secure the prosthesis 100 and mitigate any downstream or antegrade migration of the prosthesis 100 during operation.

The support frame 102 can comprise a first end portion 110 and a second end portion 112. The first end portion 110 can be positioned upstream of the second end portion 112 when the prosthesis 100 is released within the native valve annulus. As illustrated in Figure 1, the first end portion 110 of the support frame 102 can be shaped as a generally flat end of a cylinder, where first apices 114 of the support frame 102 lie generally in a common plane, which can be oriented substantially perpendicular relative to a longitudinal axis 120 of the prosthesis 100. Further, the second end portion 112 can be shaped to include a series of peaks 130 and valleys 132, where second apices or minor peaks 136 of the support frame 102 collectively form contours of the peaks 130 and valleys 132. The peaks 130 and valleys 132 of the second end portion 112 can be positioned downstream of the first end portion 110 when the prosthesis is seated within the native valve annulus.

In accordance with some embodiments, the prosthetic leaflets 106 can be coupled relative to the support frame 102 at locations circumferentially aligned with the peaks 130 of the second end portion 112, as shown in Figure 1. In some embodiments, the prosthetic leaflets 106 can be coupled to the membrane 108 using ultra-high molecular weight polyethylene sutures. This unique configuration can advantageously enable the prosthesis 100 to more fully approximate the native valve structures, permit a more natural blood flow without limiting or otherwise constraining movement of the valve leaflets 106, and more seamlessly integrate with surrounding architecture of the heart. In some embodiments, the prosthetic leaflets 106 can comprise features, including, but not limited to, planar features, flat features, three-dimensional features, Bézier curves, or other suitable shapes. Optionally, the prosthetic leaflets 106 can be shaped through fixation on a leaflet-shaped mandrel.

The valve anchor 104 can comprise at least one U-shaped member, valve clasper, sinus locator, valve positioner, or valve hanger 140 that extends about a longitudinal axis of the valve anchor 104. As illustrated in Figure 1, the valve anchor 104 can comprise a plurality of lobes or U-shaped members 140, such as three U-shaped members 140, but can have fewer or more. In some embodiments, U-shaped members 140 can be configured to engage with or fit inside the posterior aortic sinus, the left aortic sinus, and the right aortic sinus of a native aortic valve. The U-shaped members 140 can each have a peak portion 142 and a base portion 144. The U-shaped members 140 can each comprise first and second legs 146, 148. The first and second legs 146, 148 of the adjacent U-shaped members 140 can be interconnected at the peak portions 142 thereof. Further, the U-shaped members 140 can comprise shapes other than a U-shape, such as a wave-shape, V-shape, W-shape, or zig-zag. Optionally, multiple valve anchors 104 can each comprise one or more U-shaped members 140, wherein the multiple valve anchors 104 cooperatively engage with the aortic sinus to anchor the valve prosthesis as described herein.

The valve prosthesis 100 can include a link mechanism that interconnects the support frame 102 to the valve anchor 104. The link mechanism can comprise a single, continuous strand of material or multiple, independent strands of material that interconnects the support frame 102 to the valve anchor 104. Further, the link mechanism can attach in a sliding, engaged, or fixed manner to one or more locations on the support frame 102 and/or on the valve anchor 104.

In accordance with some embodiments, the valve anchor 104 may optionally define one or more engagement areas in one or more portions of the valve anchor 104, where a link mechanism may engage with the one or more engagement areas to restrict relative motion between the support frame 102 and the valve anchor 104.

For example, at the interconnection of the respective peak portions, the valve anchor 104 can define an engagement area 150. The engagement area 150 may also be referred to as a peak portion engagement area.

As illustrated in Figure 1, the support frame 102 can be flexibly coupled to the valve anchor 104 via one or more tethering components or link mechanisms 160. The link mechanism 160 can be coupled to the support frame 102 and to the valve anchor 104, permitting relative movement between the support frame 102 and the valve anchor 104. However, the link mechanism 160 can be configured to limit relative movement between the support frame 102 and to the valve anchor 104. In some embodiments, the engagement area 150 of the valve anchor 104 can be used to further restrict relative motion of the support frame 102 with respect to the valve anchor 104 when the link mechanism 160 is engaged in the engagement area 150, as discussed herein.

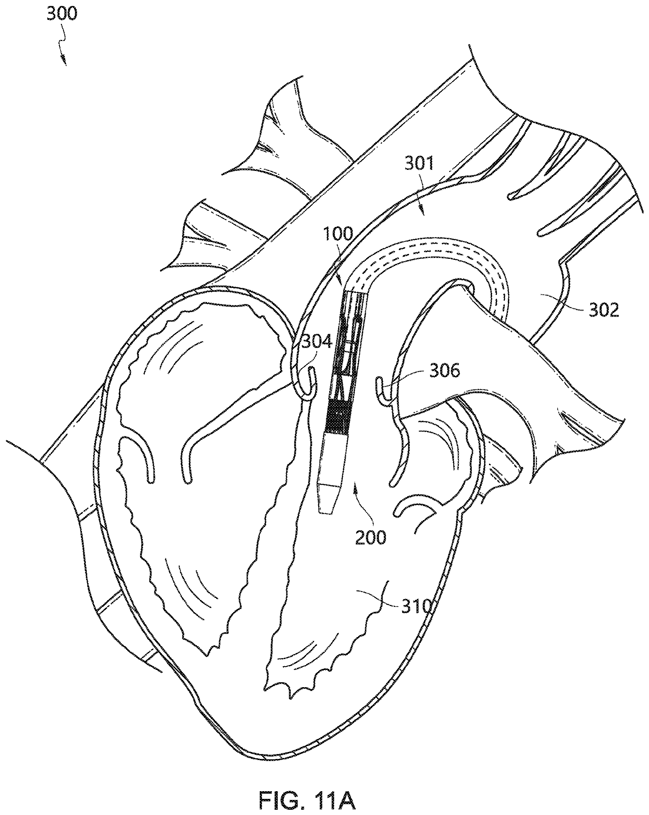

The valve anchor 104 can thus be coupled to the support frame 102 to permit the valve anchor 104 to be moved axially or longitudinally relative to the support frame 102 while still remaining coupled to the support frame 102. This advantageous feature of some embodiments can allow a clinician to independently position the valve anchor 104 relative to the support frame 102. For example, in a transcatheter aortic valve replacement, the clinician can independently position the valve anchor 104 in order to fit the base portions 144 of the valve anchor 104 into the aortic sinus. Portions of the of aortic sinus may include the posterior aortic sinus, the left aortic sinus, and/or the right aortic sinus, of a native aortic valve. In some embodiments, the valve anchor 104 can rotate to be aligned in the respective aortic sinuses. In some embodiments, the interconnection of the valve anchor 104 to the support frame 102 can allow the valve anchor 104 to self-rotate to be aligned in the aortic sinus. Thereafter, with the valve anchor 104 "landed" in the respective aortic sinuses, the interconnection of the valve anchor 104 to the support frame 102 further enables the support frame 102 to translated along the longitudinal axis 120 of the valve prosthesis 100. In some embodiments, during the delivery procedure, the valve anchor 104 can be moved at least axially from a proximal position relative to the support frame 102, to a distal position relative to the support frame 102, or from either of such positions to a position in which the support frame 102 at least partially longitudinally overlaps with or is concentric within the valve anchor 104. A range of various positions are illustrated, for example, in Figures 11A-1 1 F.

For example, when the support frame 102 is nested within the valve anchor 104, as shown in Figure 1, the base portions 144 of the valve anchor 104 can be longitudinally spaced apart from first end portion 110 of the support frame 102 along the longitudinal axis 120 at a distance which is about 10% to about 100%, about 25% to about 75%, about 33% to about 100%, about 33% to about 66%, about 25% to about 75%, about 50% to about 75%, or about 60% to about 70% of a length of the support frame 102. In some embodiments, the support frame 102 can be contained or otherwise fully overlapping the valve anchor 104. In some embodiments, the support frame 102 can have minimal or no overlap with the valve anchor 104. The support frame 102 can move along the longitudinal axis 120 to overlap the valve anchor 104 by about 10% to about 100%, about 25% to about 75%, about 33% to about 100%, about 33% to about 66%, about 25% to about 75%, or about 50% to about 75% of the length of the support frame 102. In accordance with some embodiments, the U-shaped members 140 of the valve anchor 104 can be in nested positions within the aortic sinuses, and the base portions 144 of the valve anchor 104 can be about longitudinally adjacent to, coplanar with, or spaced apart from the first end portion 110 of the support frame 102. For example, the valve anchor 104 can be in a nested position when at least one base portion 144 of the valve anchor 104 is in contact with or adjacent to the basal attachments of the native aortic valvar leaflets. Further, the first end portion 110 of the support frame 102 can be longitudinally adjacent to, coplanar with, or spaced apart from the native valve structure (or a virtual ring formed by the basal attachments of the native aortic valvar leaflets) or with the ventriculo-aortic junction.

The link mechanism 160 can allow rotational and longitudinal movement of the valve anchor 104 relative to the support frame 102. Thus, despite the presence of the link mechanism 160, the valve anchor 104 can move rotationally with respect to the support frame 102. Further, in some embodiments, the link mechanism 160 can be fixedly attached or coupled to the support frame 102 and fixedly or slidably attached to the valve anchor 104. When the support frame 102 is moved relative to the valve anchor 104, the link mechanism 160 can slide along the U-shaped members 140. In some embodiments, the U-shaped members 140 have a generally arcuate or convex shape (as illustrated with the U-shaped members of Figure 1) that allows unrestricted movement of the link mechanism 160 along the geometry of the first and second legs 146, 148 of the U-shaped members 140. When the link mechanism 160 is allowed to slide along the first and second legs 146, 148 of the U-shaped members 140, the valve prosthesis 100 can be in a position referred to as a "slidable" state. In the slidable state, the range of longitudinal and/or rotational movement of the support frame 102 relative to the valve anchor 104 is variable and may be its greatest because the link mechanism 160 can move along the first and second legs 146, 148 of the U-shaped members 140.

In some embodiments, the link mechanism 160 can be fixedly attached or coupled to the support frame 102 and fixedly attached to the valve anchor 104. When the support frame 102 is moved relative to the valve anchor 104, the link mechanism 160 can stretch, flex, deform elastically and/or plastically. As the link mechanism 160 deforms, the range of longitudinal and/or rotational movement of the support frame 102 relative to the valve anchor 104 is variable as allowed by the deformation of the link mechanism 160.

In some embodiments, the link mechanism 160 can have multiple link members, where each link member is coupled to and intermittently spaced about a circumference of the support frame 102. Each link member may be slidably coupled to a respective one of the U-shaped members 140. Further, the link mechanism 160 can have multiple link members that are coupled together in an end-to-end manner. Moreover, the link mechanism 160 can have multiple link members that are individually coupled at one and to the support frame 102 and at another and to the valve anchor 104. Each of the link members can be slidable along the valve anchor 104, as disclosed similarly herein and not described again herein for brevity.

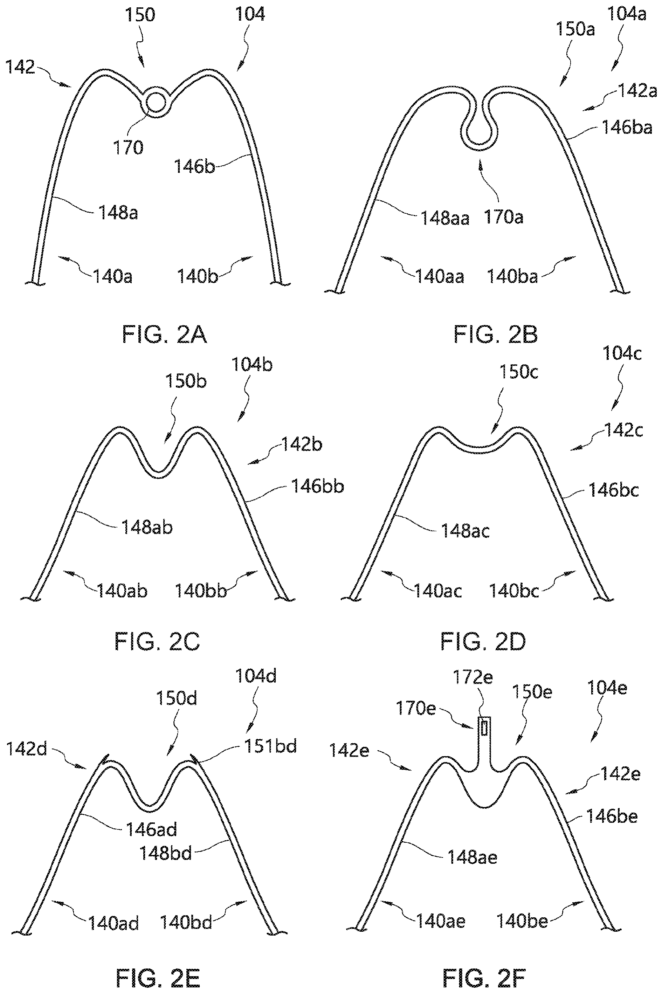

As noted above, however, the valve anchor 104 can also comprise engagement areas 150 that can engage with the link mechanism 160 in order to restrict relative motion between the support frame 102 and the valve anchor 104. The engagement areas 150 can include one or more local concavities or other geometric shapes that can engage or trap the link mechanism 160 once the link mechanism 160 passes into the engagement area 150. Figures 2A-2G illustrate various embodiments of engagement areas 150 that can be used to permit the slidable link mechanism 160 to enter into the engagement area 150, but restrict the link mechanism 160 from exiting the engagement area 150.

For example, Figure 2A illustrates an embodiment of the valve anchor 104 having an engagement area 150 that is formed between first and second U-shaped members 140a, 140b. The first U-shaped member 140a comprises a second leg 148a, and the second U-shaped member 140b comprises a first leg 146b. The engagement area 150 can be formed or disposed between the first leg 146b and the second leg 148a.

The engagement area 150 can comprise an eyelet or anchor retention component 170, and the first and second legs 146b, 148a can extend in a direction toward the peak portions 142 thereof. The first and second legs 146b, 148a can also each comprise a bend or curve that causes the first and second legs 146b, 148a to bend in a direction away from the peak portions 142 and to converge at the anchor retention component 170. This configuration creates a local concavity or cove whereinto the link mechanism 160 can slide and be engaged. Further, as discussed further below, the anchor retention component 170 can be used to engage with the control member or a grasper to facilitate movement and control of the positioning of the valve anchor 104 during delivery. As shown in Figure 2A, in some embodiments, the anchor retention component 170 may be in a shape of an eyelet, aperture, or hole that extends through the engagement area 150 of the valve anchor 104.

Further, as illustrated, the anchor retention component 170 can allow for the control member or the grasper to engage with the valve anchor 104 for a retrograde approach (as shown in Figures 11A-11F) or an antegrade approach (as shown in Figures 12A-12F). For example, the anchor retention component 170 can receive a control member or a grasper approaching the valve anchor 104 proximal to the peak portions 142 to allow the valve anchor 104 to be used for a retrograde (e.g., transfemoral retrograde) approach in delivering the valve anchor 104. Further, the anchor retention component 170 can receive the control member or the grasper approaching the valve anchor 104 proximal to the first and second U-shaped members 140a, 140b to allow the valve anchor 104 to be used for an antegrade, apical, or transapical approach in delivering the valve anchor 104.

Similar to Figure 2A, Figure 2B illustrates an embodiment of a valve anchor 104a having an engagement area 150a formed between first and second U-shaped members 140aa, 140ba. The first U-shaped member 140aa comprises a second leg 148aa, and the second U-shaped member 140ba comprises a first leg 146ba. The engagement area 150a is formed or disposed between the first leg 146ba and the second leg 148aa. The first and second legs 146ba, 148aa can extend in a direction toward the peak portions 142a thereof. The first and second legs 146ba, 148aa can also each comprise a bend or curve that causes the first and second legs 146ba, 148aa to bend in a direction away from the peak portions 142a and to converge to form a keyhole shape or narrow-waisted cove 170a. The keyhole shape or narrow-waisted cove 170a may be used as an anchor retention component to engage with the control member or the grasper. Again, this configuration creates a local concavity or cove whereinto the link mechanism 160 can slide and be engaged.

Further, Figures 2C and 2D illustrate additional embodiments of valve anchors 104b, 104c having engagement areas 150b, 150c. Similar to the embodiments shown in Figures 2A and 2B, the details of which will not be repeated for brevity (but can optionally be incorporated into the embodiments of Figures 2C and 2D, as well), first and second U-shaped members 140ab, 140bb, 140ac, 140bc can have bends or curves which form the respective engagement areas 150b, 150c. These configurations can be referred to as collectively providing a "triple switchback" design that is created by the first and second legs 146bb, 148ab and 146bc, 148ac, respectively. The engagement area 150b is deeper than the engagement area 150c, and the depth can be varied in order to facilitate capture of the link mechanism 160 therein.

Figure 2E illustrates an additional embodiment of valve anchor 104d having engagement area 150d. Similar to the embodiments shown in Figures 2A and 2B, the details of which will not be repeated for brevity (but can optionally be incorporated into the embodiment of Figure 2E, as well), first and second U-shaped members 140ad, 140bd can have bends or curves which form the engagement area 150d. Similarly, this configuration can also be referred to as providing a "triple switchback" design that is created by the first and second legs 146bd, 148ad.

In addition, as shown in Figure 2E, in some embodiments, the engagement area 150d can include one or more barbs 151d to facilitate capture of the link mechanism 160 therein. In some embodiments, the barbs 151d can deflect downward to allow the link mechanism 160 to pass over the barb 151d, while preventing the link mechanism 160 from returning over the barb 151d.

Figure 2F illustrates an additional embodiment of valve anchor 104e having engagement area 150c. Similar to the embodiments shown in Figures 2A and 2B, the details of which will not be repeated for brevity (but can optionally be incorporated into the embodiment of Figure 2F, as well), first and second U-shaped members 140ae, 140be can have bends or curves which form the engagement area 150e. The first and second legs 146be, 148ae can also each comprise a bend or curve that causes the first and second legs 146be, 148ae to bend in a direction away from the peak portions 142e and to converge at towards the clasper tang 170e to create a local concavity or cove whereinto the link mechanism 160 can slide and be engaged.

In addition, as shown in Figure 2F, in some embodiments, the engagement area 150e can also comprise a central grasping portion or clasper tang 170e that extends from the engagement area 150e. As discussed below, the clasper tang 170e can be used as an anchor retention component to engage with a control member or grasper of the delivery device to facilitate movement and control of the positioning of the valve anchor 104 during delivery. In some embodiments, the clasper tang 170e can comprise an engagement structure, socket, aperture, or protrusion 172. The engagement structure 172 can be positioned along the length or body of the clasper tang 170e. As illustrated, the engagement structure 172 can be positioned centrally at a distal end portion of the clasper tang 170e.

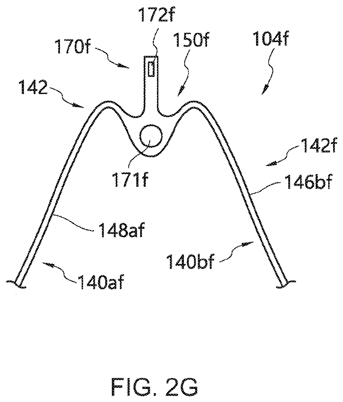

Figure 2G illustrates an additional embodiment of valve anchor 104f having engagement area 150f. Similar to the embodiment shown in Figures 2A and 2B, the details of which will not be repeated for brevity (but can optionally be incorporated into the embodiment of Figure 2G, as well), first and second U-shaped members 140af, 140bf can have bends or curves which form the engagement area 150f. The first and second legs 146bf, 148af can also each comprise a bend or curve that causes the first and second legs 146bf, 148af to bend in a direction away from the peak portions 142f and to converge at towards the clasper tang 170f to create a local concavity or cove whereinto the link mechanism 160 can slide and be engaged.

In addition, as shown in Figure 2G, in some embodiments, the engagement area 150f can also comprise an eyelet or anchor retention component 171f and a central grasping portion or clasper tang 170f that extends from the engagement area 150f. Similar to the embodiment shown in Figure 2A, the anchor retention component 171f may be in a shape of an eyelet, aperture, or hole that extends through the engagement area 150f of the valve anchor 104f. Further, similar to the embodiment shown in Figure 2F, the clasper tang 170f can be used as another anchor retention component to engage with a control member or grasper of the delivery device to facilitate movement and control of the positioning of the valve anchor 104f during delivery.

As illustrated, the clasper tang 170f can allow for the control member or the grasper to engage with the valve anchor 104f for a transfemoral retrograde approach (as shown in Figures 11A-11F), and the anchor retention component 171f can allow for the control member or the grasper to engage with the valve anchor 104f for an antegrade approach (as shown in Figures 12A-12F). For example, the clasper tang 170f can receive a control member or a grasper approaching the valve anchor 104f proximal to the peak portions 142f to allow the valve anchor 104 to be used for a retrograde (e.g., a transfemoral retrograde) approach in delivering the valve anchor 104f. Further, the anchor retention component 171f can receive the control member or the grasper approaching the valve anchor 104f proximal to the first and second U-shaped members 140af, 140bf to allow the valve anchor 104f to be used for an antegrade, apical, or transapical approach in delivering the valve anchor 104f.

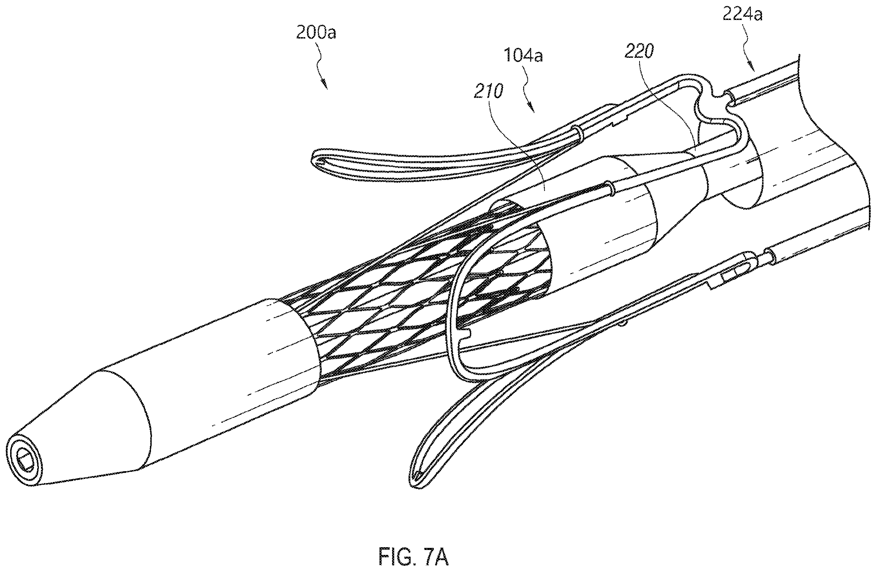

Further, as also illustrated and discussed later in Figures 7A-7D, the engagement structure 172f can comprise an aperture that can receive a respective protrusion of a distal end portion of a grasper member for coupling the clasper tang 170f to the grasper. However, some embodiments can be provided in which the engagement structure 172 of the clasper tang 170f uses a protrusion and the grasper member uses a corresponding aperture.

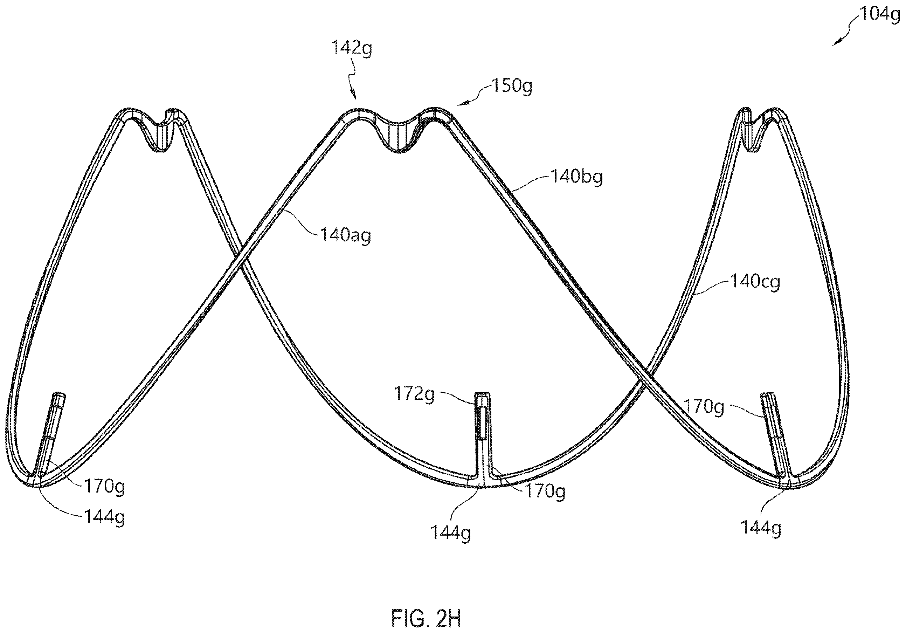

Figure 2H illustrates an additional embodiment of valve anchor 104g having engagement area 150g. Similar to the embodiment shown in Figures 2C and 2D, the details of which will not be repeated for brevity (but can optionally be incorporated into or omitted from the embodiment of Figure 2H), the first and second U-shaped members 140ag, 140bg can have bends or curves which form the engagement area 150g. These configurations can be referred to as collectively providing a "triple switchback" design that is created by the first and second U-shaped members 140ag, 140bg, respectively.

In addition, as shown in Figure 2H, in some embodiments, the U-shaped members 140ag, 140bg, 140cg, can optionally comprise a central grasping portion or clasper tang 170g that extends from a medial location of the base portion of the U-shaped members 140ag, 140bg, 140cg. Optionally, multiple valve anchors 104g can each comprise one or more U-shaped members 140ag, 140bg, 140cg, wherein the multiple valve anchors 104g cooperatively engage with the aortic sinus to anchor the valve prosthesis as described herein.

For example, the clasper tang 170g can extend from a base portion 144g of the U-shaped member 140ag, 140bg in an axial direction toward the engagement areas of the valve anchor 104g or radially inside the bend of the U-shaped members 140ag, 140bg of the valve anchor 104g.

As discussed below, the clasper tang 170g can be used as an anchor retention component. For example, the clasper tang 170g can engage with a control member or grasper of the delivery device to facilitate movement and control of the positioning of the valve anchor 104g during delivery. This engagement can advantageously provide greater control over the articulation of the U-shaped members, as well as to function as a link motion limiter. Such features and benefits are discussed further below.

In some embodiments, by locating the clasper tang 170g at the base portion of the U-shaped members 140ag, 140bg, 140cg, the valve anchor 104g may have an increased bending stiffness at the grasper attachment points during delivery. In some applications, the valve anchor 104g may advantageously be less likely to invert during delivery due to the increased bending stiffness. Further, control of the base portions 144g can permit a clinician to specifically control the articulation and placement of the U-shaped members relative to the sinus structure of the native valve during delivery and placement of the valve anchor 104g.

In some embodiments, the clasper tang 170g can comprise an engagement structure, socket, aperture, or protrusion 172g. The engagement structure 172g can be positioned along the length or body of the clasper tang 170g. As illustrated, the engagement structure 172g can be positioned centrally at a distal end portion of the clasper tang 170g.

As illustrated and discussed in regard to other aspects of this disclosure (see, e.g., Figures 7A-7F), the clasper tang 170g can allow for the control member or the grasper to engage with the valve anchor 104g for a retrograde approach (as shown in Figures 11A-11F) or for an antegrade approach (as shown in Figures 12A-12F).

For example, the clasper tang 170g can receive a control member or a grasper approaching the valve anchor 104g proximal to the peak portions 142g to allow the valve anchor 104g to be used for a retrograde approach (e.g., a transfemoral retrograde approach) in delivering the valve anchor 104g. Further, the clasper tang 170g can receive the control member or the grasper approaching the valve anchor 104g proximal to the first and second U-shaped members 140ag, 140bg to allow the valve anchor 104g to be used for an antegrade approach (e.g., an apical or transapical approach) in delivering the valve anchor 104f.

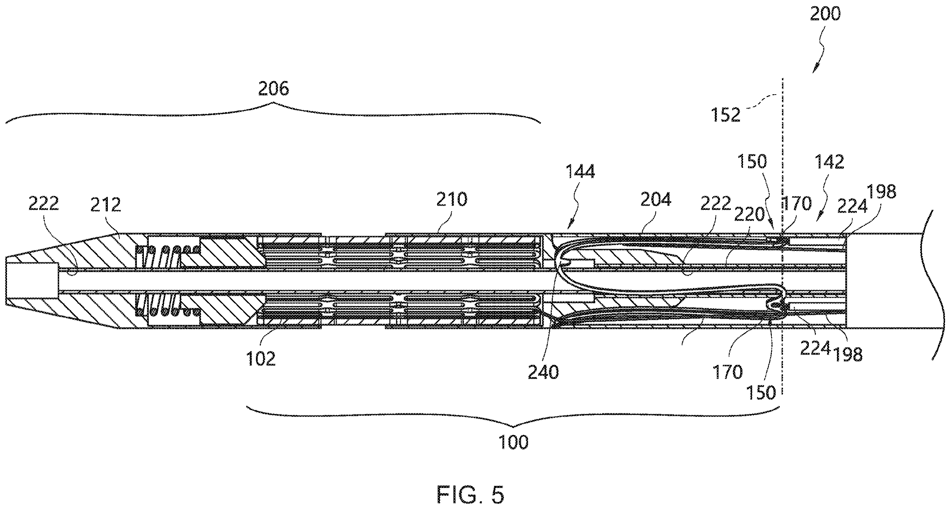

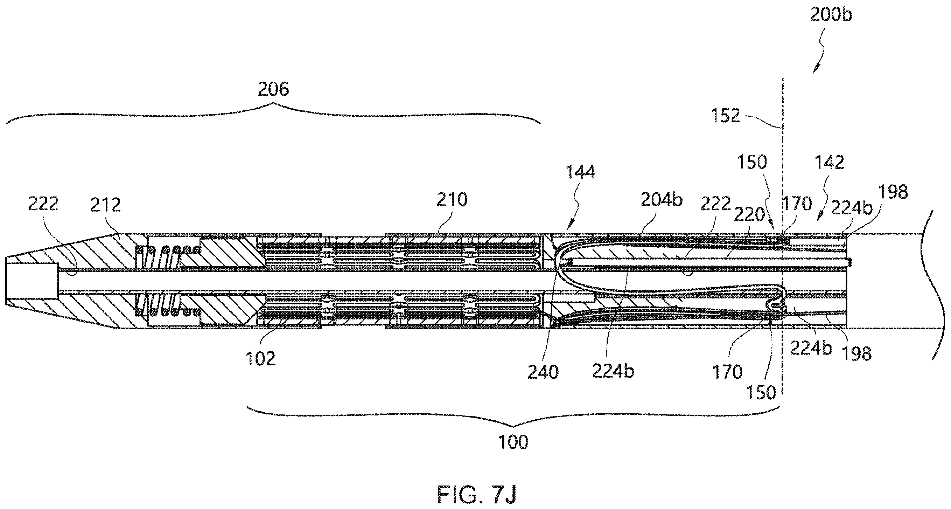

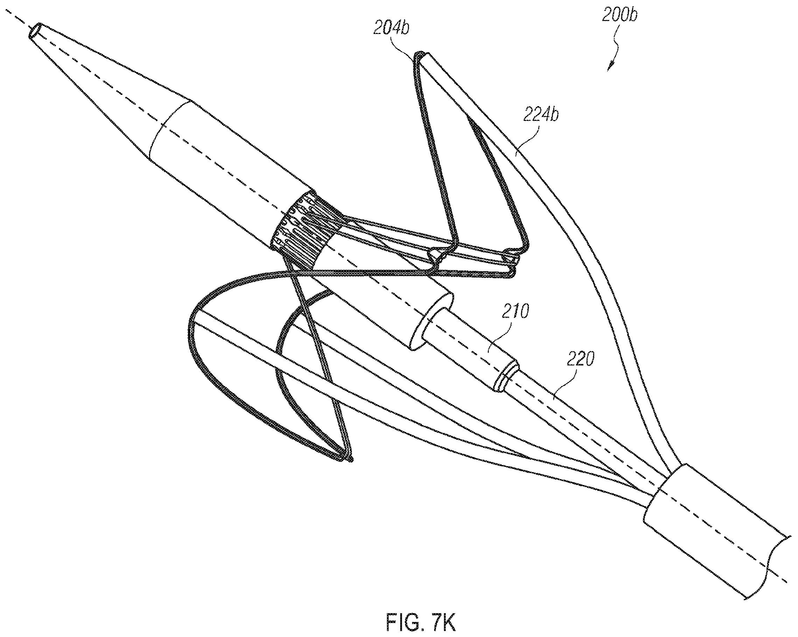

Optionally, as noted above, the clasper tang 170g (collectively, with the grasper 224b, as shown in the example of Figures 7J and 7K) can also serve as a link motion limiter, as described herein with regard to Figures 5-6E, to provide an enlarged profile as to restrict or prevent motion of the link mechanism as the link mechanism slides along the U-shaped members 140ag, 140bg, 140cg of the valve anchor 104g.

These various designs are examples of engagement areas that can be used in some embodiments disclosed herein. Further, in any of the embodiments disclosed in Figures 2A-2D, the engagement area can comprise barbs or hooks over which the link mechanism 160 can pass. The barbs or hooks can permit one-way motion of the link mechanism 160 - once the link mechanism 160 crosses the barbs or hooks, the barbs or hooks will prevent reverse movement of the link mechanism 160 over the barbs or hooks. The illustrated embodiments provide double peaks or coves that can tend to capture the link mechanism during the stages of prosthesis delivery, as discussed herein. When the link mechanism is so captured, the valve prosthesis 100 can be in a position referred to as a "retained" position.

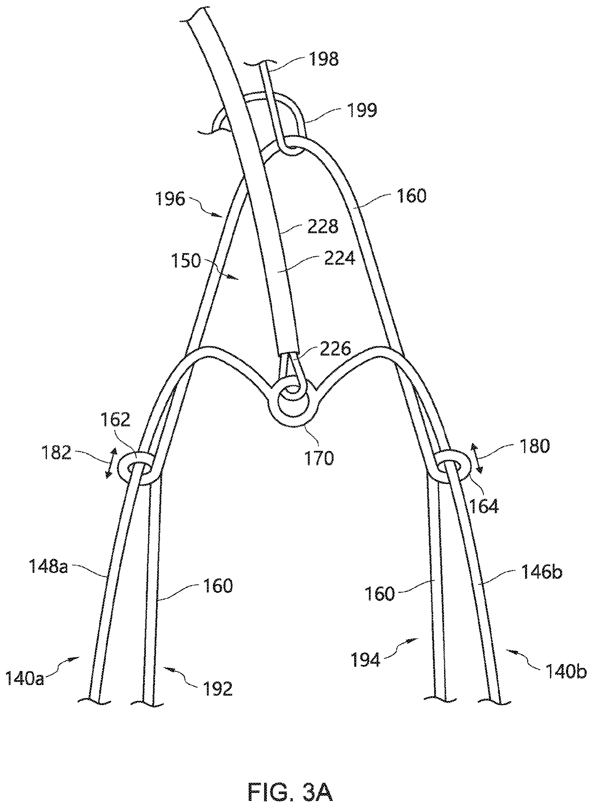

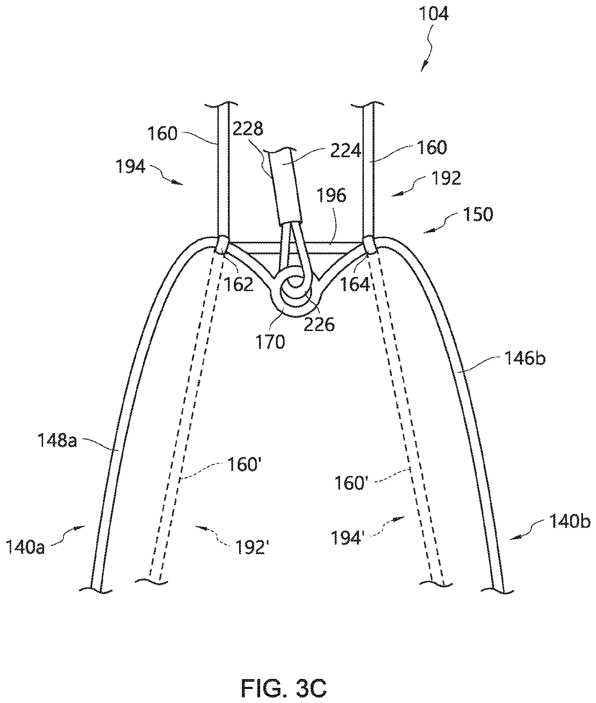

Figures 3A-3C illustrate movement of the link mechanism along legs of the U-shaped members and capture of the link mechanism within the engagement area, according to some embodiments. Figure 3A illustrates an embodiment of the valve anchor 104 having first and second U-shaped members 140a, 140b and a link mechanism 160 that is in the slidable position. Figure 3C illustrates an embodiment of the valve anchor 104 with the link mechanism 160 in the retained position.

As illustrated in Figure 3A, the link mechanism 160 is in a slidable position and therefore, can slide freely along the first and second legs 146b, 148a of the first and second U-shaped members 140a, 140b, as indicated by the arrows 180, 182. The link mechanism 160 has a loop 162 that loops around the second leg 148a of the first U-shaped member 140a, and a loop 164 that loops around the first leg 146b of the second U-shaped member 140b. The loops 162, 164 can be shaped in mirror images of each other (i.e., the pathway or course of the link mechanism 160 around the second leg 148a and the first leg 146b can be mirror images of each other), as shown.

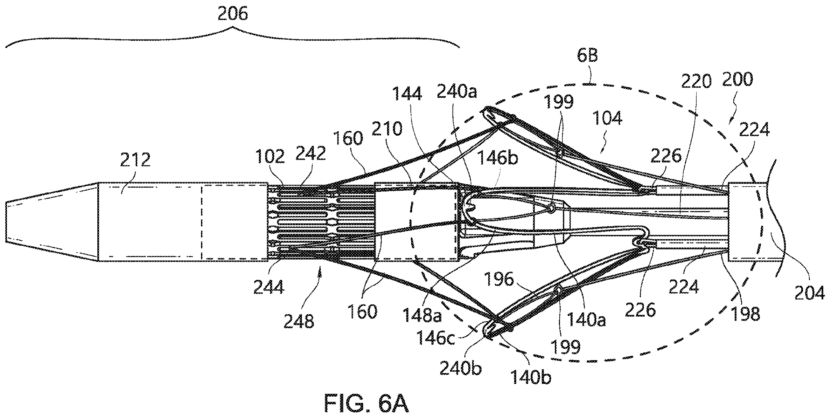

As also generally illustrated in Figure 3A, the link mechanism 160 can be woven between the U-shaped member 140 and corresponding circumferential attachment points or positions at or on the support frame 102. For example, a first segment 192 of the link mechanism 160 can loop around the second leg 148a and extend towards a first attachment point on the support frame. Further, a second segment 194 of the link mechanism 160 can loop around the first leg 146b and extend towards a second attachment point on the support frame, different from the first attachment point. This weaving pattern is also generally shown in Figures 4A and 6A.

Furthermore, with the first and second segments 192, 194 extending away from the engagement area 150, it is also apparent that in Figure 3A, the support frame is positioned somewhat distally relative to the valve anchor 104, thus causing the link mechanism 160, when taut, to extend toward the support frame in a direction away from the engagement area 150. Furthermore, an intermediate segment 196 of the link mechanism 160 may have some slack and be relatively long (compared to its status and length shown in Figure 3C, which is explained below).

In some embodiments, the slack and length in the intermediate segment 196 of the link mechanism 160, when the link mechanism 160 is in the slidable position, allow the intermediate section 196 to be engaged by a tension member 198. As shown, the tension member 198 can comprise a flexible loop 199 that can be engaged with the intermediate section 196 such that the intermediate section 196 passes through the flexible loop 199. As discussed further below, the tension member 198 can extend proximally relative to the prosthesis 100 and assist in maintaining the intermediate section 196 in a tucked or compact configuration, so that the link mechanism 160 does not catch or become tangled during advancement of the prosthesis 100 to the target valve annulus during the procedure.

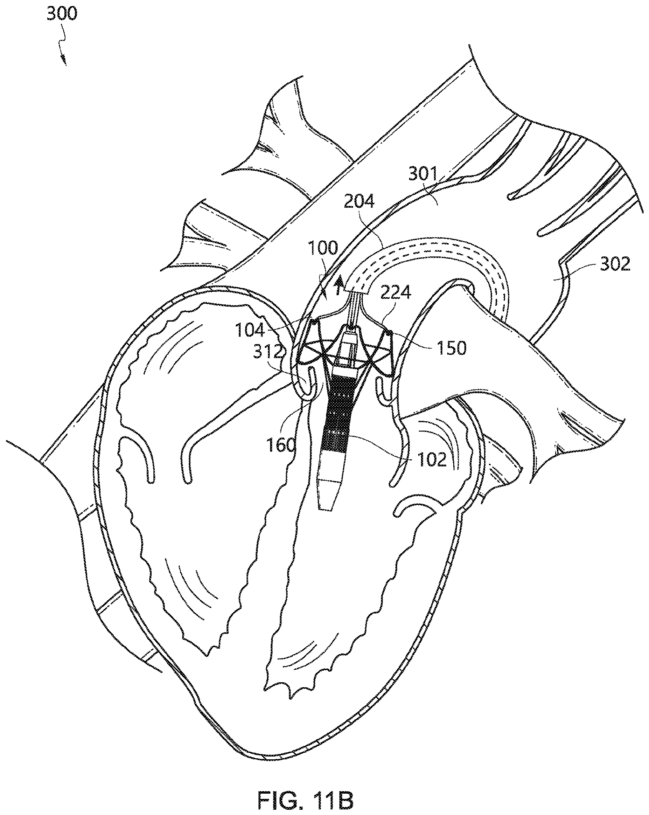

For example, in use, the tension member 198 can be maintained at a position that generally allows the intermediate section 196 to be pulled proximally to remove or reduce slack in the link mechanism 160 when the valve anchor 104 is in the compressed configuration. In some embodiments, the tension members 198 can be positioned in a fixed position relative to the grasper 224, and the flexible loop 199 can be longitudinally positioned distal or proximal to distal ends, pinchers, or hooks 226 of the grasper 224. However, during relative movement of the support frame 102 and the valve anchor 104, when the support frame 102 is distally advanced relative to the valve anchor 104 (when the anchor 104 is expanded at the target location), the slack in the intermediate section 196 will lessen and eventually, the flexible loop 199 of the tension member 198 will bend and release the intermediate section 196, thus disengaging the tension member 198 from the link mechanism 160. The general arrangement of the prosthesis 100, including the when loaded onto the delivery device, as shown in Figures 4A-6B and in the delivery stages of Figures 11A and 11B.

Further, various embodiments of the link mechanism 160 (e.g., 160a, 160b, 160c, 160d, 160e, and/or combinations thereof) are illustrated in Figures 4A-4G. In accordance with some embodiments, it is contemplated that any version of the link mechanism can be interchangeably used with the prosthesis or features thereof.

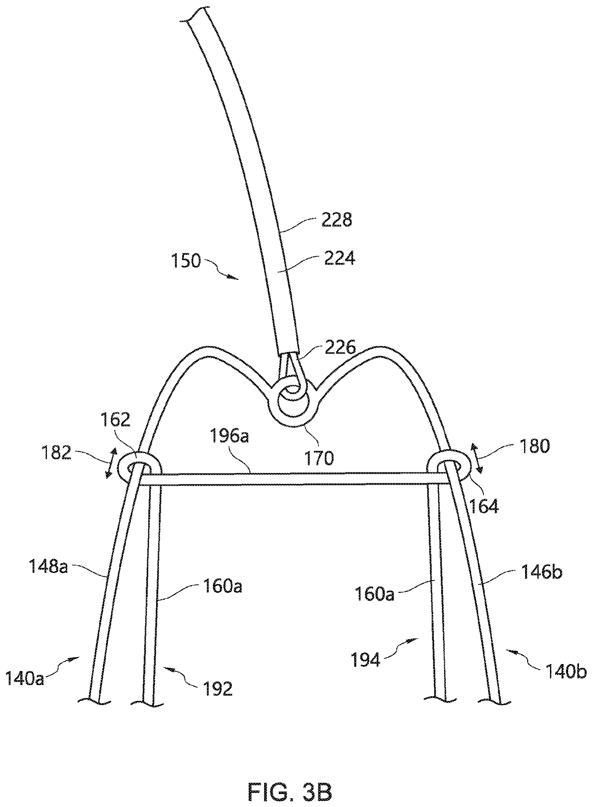

As illustrated in Figure 3B, some embodiments of the delivery device can use a link mechanism 160a without a tension member. Thus, the link mechanism 160a can be attached to the support frame 102 and the valve anchor 104 and have a length configured such that in a delivery state (such as shown in Figure 4A), the link mechanism 160a is generally taut. As shown, an intermediate segment 196a of the link mechanism 160a may have less slack and length between the loops 162 and 164 compared to the link mechanism 160 shown in Figure 3A. In some embodiments, advantageously, the length of the intermediate segment 196a and taut state thereof may avoid the use of a tension member while still maintaining the intermediate section 196a in a tucked or compact configuration. Thus, the link mechanism 160a does not catch or become tangled during the advancement of the prosthesis 100 to the target valve annulus during the procedure.

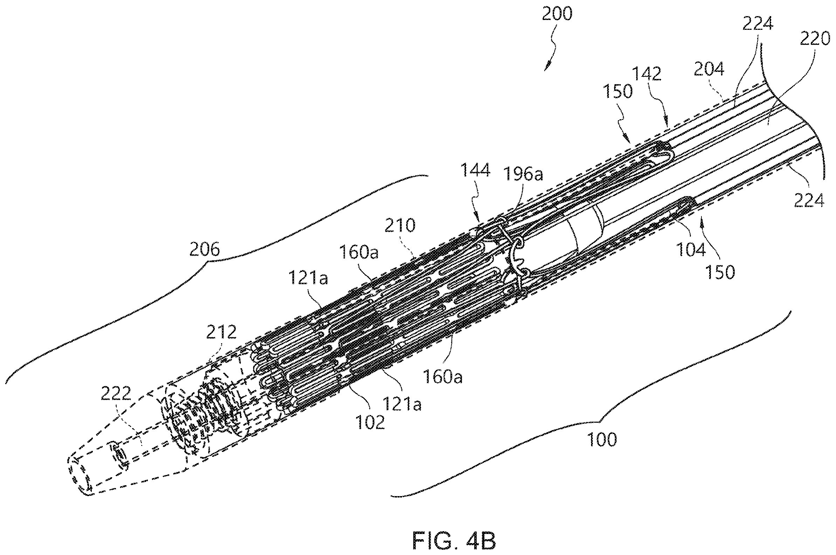

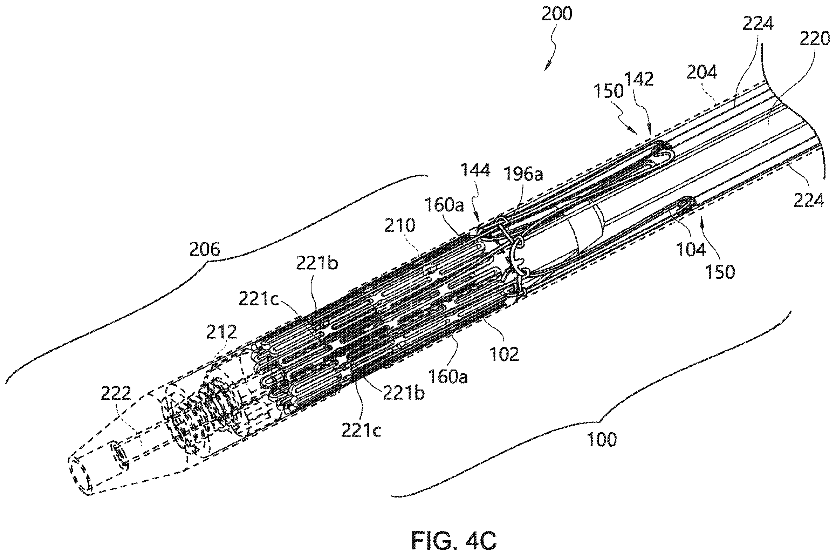

As shown in Figure 3B, the link mechanism 160a can be woven between the U-shaped member 140 and corresponding circumferential attachment points or positions at or on the support frame 102. For example, a first segment 192 of the link mechanism 160a can loop around the second leg 148a and extend towards a first attachment point on the support frame. Further, a second segment 194 of the link mechanism 160a can loop around the first leg 146b and extend towards the first attachment point on the support frame (Figure 4B) or a second attachment point on the support frame, different from the first attachment point (Figure 4C). In some embodiments, the link mechanism 160a can be tied, looped, and/or wound to the attachment points of the support frame. As illustrated, an intermediate segment 196a of the link mechanism 160a may interconnect the loop around the first leg 146b and the loop around the second leg 148a. In some embodiments, the use of the link mechanism 160a without a tension member may facilitate the serial "stacked" arrangement of the valve anchor 104 and the support frame 102.

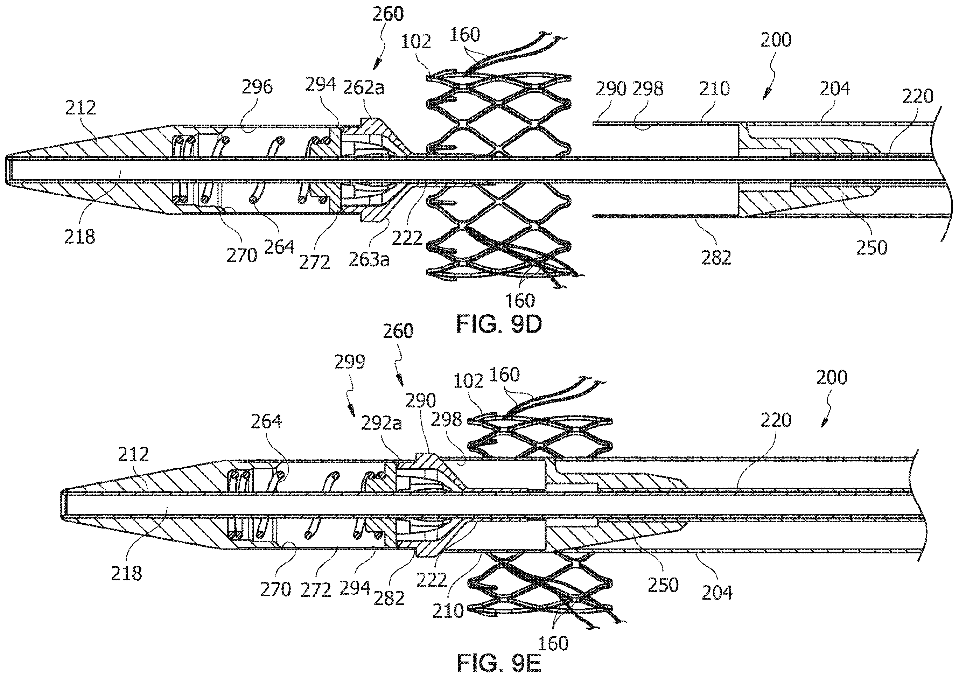

When the support frame is retracted proximally relative to the valve anchor 104, the link mechanism 160 can slide along the U-shaped members 140 toward the engagement area 150. Thus, moving from the position of the link mechanism 160 in Figure 3A (or similarly, from the position of the link mechanism 160a and Figure 3B) to the position of the link mechanism 160 in Figure 3C, the first and second segments 192, 194 are now pulled in a direction proximal to the engagement area 150. This proximally oriented pulling of the link mechanism 160, 160a has drawn the first and second loops 162, 164 distally into the engagement area 150. Further, in embodiments utilizing the tension member 198, during this movement (or when the support frame 102 is moved distally relative to the valve anchor 104), the tension member 198 will eventually disengage from the link mechanism 160. Additionally, because the link mechanism 160 can slide around the legs 146b, 148a via the loops 162, 164, the relative length of the intermediate segment 196 decreases and is much smaller in Figure 3C due to the proximally oriented pulling of the link mechanism 160 (which causes more of the length of the link mechanism 160 to be drawn into the first and second segments 192, 194 than in the intermediate segment 196). Accordingly, a clinician can reduce the length of the intermediate segment 196 by pulling the support frame proximally relative to the valve anchor 104 until the first and second segments 192, 194 of the link mechanism 160 are taut. This general arrangement in illustrated in the delivery stage of Figure 9D.

Thereafter, as shown by the dashed lines in Figure 3C, the support frame can later be advanced distally relative to the valve anchor 104 and longitudinally overlap with the valve anchor 104, thus pulling the first and second segments 192', 194' of the link mechanism 160' in a distal direction relative to the engagement area 150. However, in some embodiments, due to the reduced length of the intermediate segment 196, which can be less than a width of the engagement area 150 (which can be represented by a peak-to-peak width of the curves of the second leg 148a and the first leg 146b), one or both of the loops 162, 164 will tend to remain captured within the engagement area 150 when the support frame is advanced distally relative to the valve anchor 104. In this manner, the engagement of the loops 162, 164 within the engagement area 150 can restrict or prevent the loops 162, 164 of the link mechanism 160' from sliding distally along either the first or second legs 146b, 148a. The prosthesis 100 is thus in the retained position, and the range of longitudinal and/or rotational movement of the support frame 102 relative to the valve anchor 104 is fixed as the link mechanism 160 is retained within the engagement area 150.

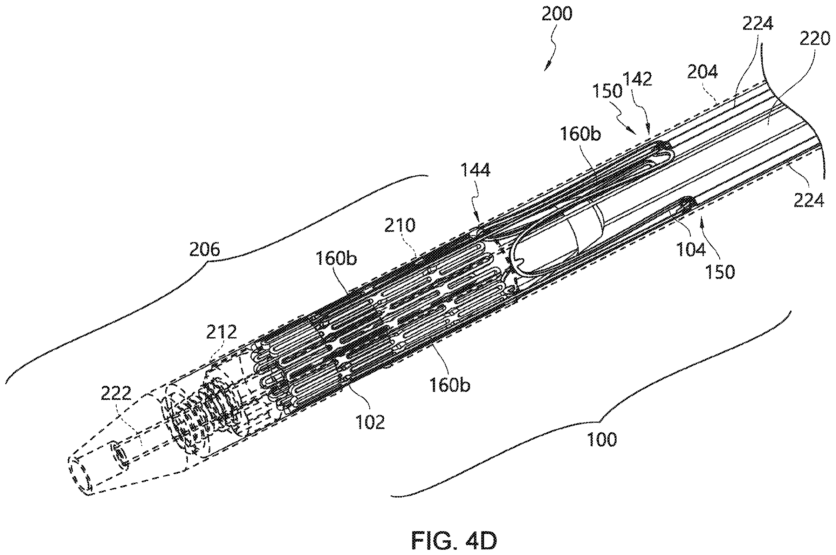

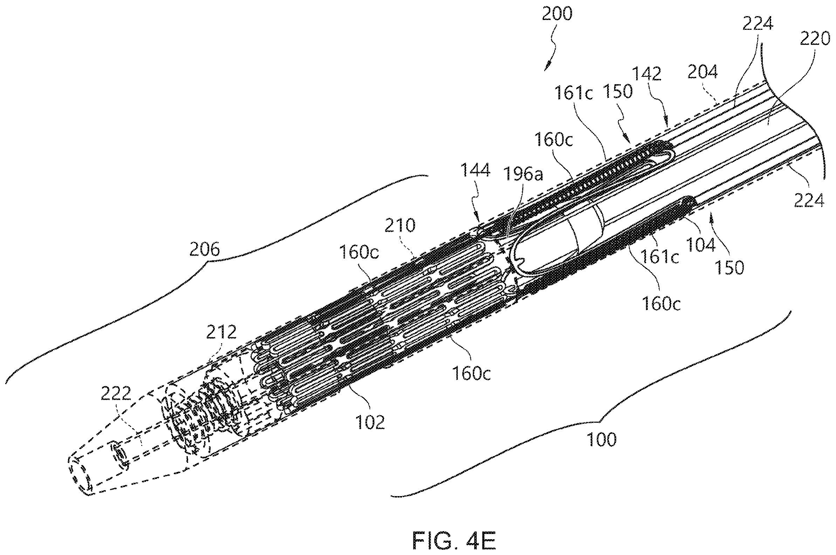

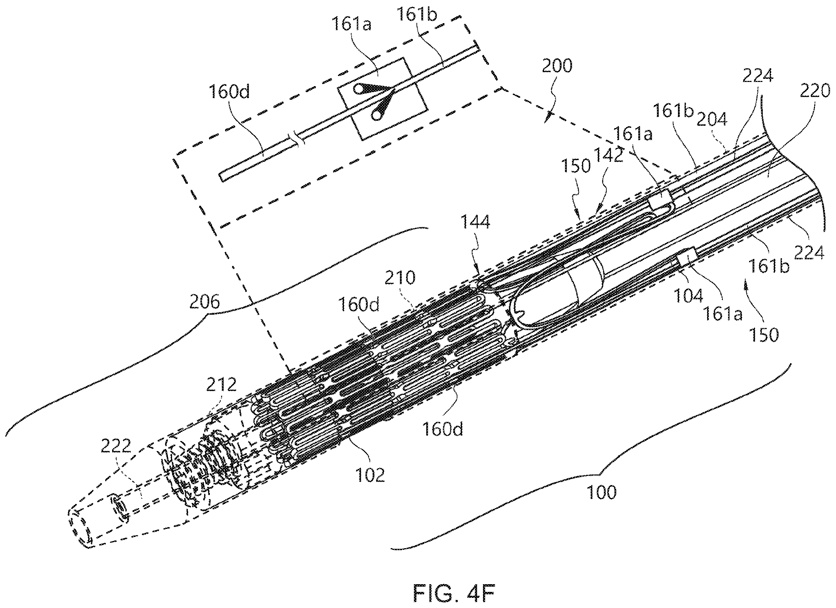

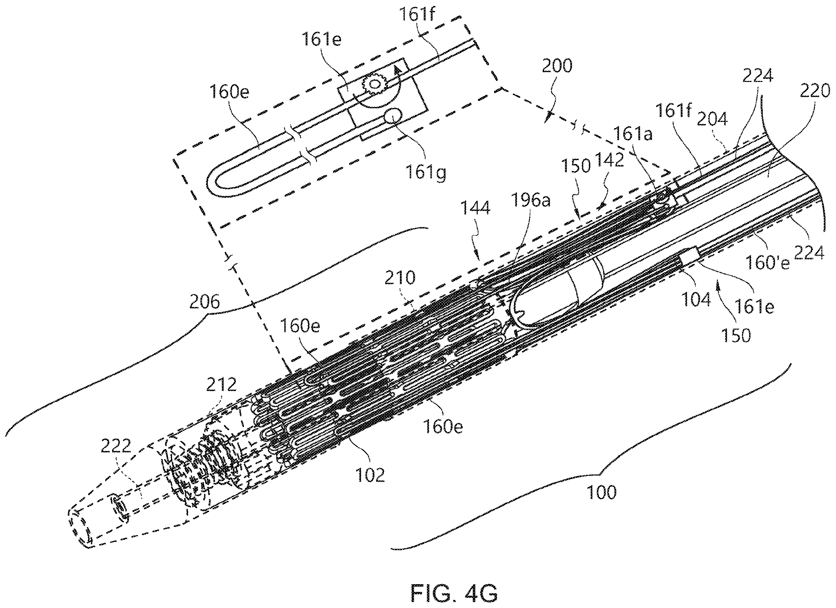

Figures 3A-3C also illustrate an embodiment of a control member or grasper of a delivery device (e.g., as shown further below in Figure 4A) that can be used to engage the anchor retention component 170 of the engagement area 150. It is noted that, the example embodiments illustrated in Figures 2A and 3A-3C show that the anchor retention component 170 is disposed at the peak portion 142. However, in other embodiments, the anchor retention component 170 may be disposed in one or more other portions of the U-shaped member 140. Further, although the anchor retention component 170 is illustrated as being positioned in a valley between two peaks formed by the looping ends of the first leg 146b of the second U-shaped member 140b and the second leg 148a of a first U-shaped member 140a, the valve anchor 104 can be configured to comprise a rounded, single-peak section (i.e., without dual peaks or an intermediate valley) at which the anchor retention component 170 is formed between the first leg 146b and the second leg 148a. The function and structure of the grasper as discussed further herein, for example, with respect to Figures 4-6B.

In accordance with some embodiments, the link mechanism 160 can have a fixed length. The fixed length of the link mechanism 160 can restrict longitudinal, circumferential (i.e., rotational), and/or radial movement that the valve anchor 104 relative to the support frame 102 when the prosthesis 100 is in either the slidable or retained positions. Thus, the link mechanism 160 can comprise a material that is not stretchable. In some embodiments, the link mechanism 160 can comprise a suture, made from materials known in the art. The fixed length of the link mechanism 160 can be configured to ensure that when in the retained position, the support frame 102 is advanced to a maximum distal position relative to the valve anchor 104 that provides an optimal placement of the support frame 102 relative to the valve anchor 104.

Further, in some embodiments, the link mechanism 160 can comprise a single, continuous strand of material that is interwoven with the support frame 102 and the valve anchor 104 at multiple locations, as illustrated in Figure 1. In such embodiments, the single, continuous strand can be tied at its ends to a coupling point(s) of the support frame 102 and looped or wound around other coupling points of the support frame 102 and the valve anchor 104 at (many or all) other locations. When looped or wound around a coupling point of the support frame 102 or the valve anchor 104, the continuous-stranded link mechanism 160 can slide or move relative to the support frame 102 or valve anchor 104, which provides various advantages that are discussed herein.

In some embodiments using a single, continuous link mechanism 160 and a single U-shaped member of the valve anchor, a loop length of the continuous link mechanism 160 can be greater than the sum of (i) a compressed circumference of the support frame and (ii) two times a longitudinal length of the support frame. Further, in some embodiments using a single, continuous link mechanism 160 and a plurality of U-shaped members, a loop length of the continuous link mechanism 160 can be between about 80% to about 120% of a sum of (i) a compressed circumference of the support frame and (ii) four times a longitudinal length of the support frame. For example, in some embodiments, a single, continuous link mechanism 160 can be interwoven with three U-shaped members of the valve anchor, and a loop length of the continuous link mechanism 160 can be between about 80% to about 120% of a sum of (i) a compressed circumference of the support frame and (ii) six times a longitudinal length of the support frame.

In some embodiments, the link mechanism 160 can comprise multiple, individual lengths of material that are coupled at one end to the support frame 102 and at another end to the valve anchor 104. For example, the link mechanism 160 can be fixedly attached (i.e., so as to not move or slide) at one end to the valve anchor 104 and slidably attached at the other end to the support frame 102 to provide the movement and capturing of the link mechanisms 160 within the engagement areas 150, as discussed above.

Whether the link mechanism 160 comprises a single, continuous strand or multiple, individual lengths of material, a longitudinal length or extent of the link mechanism(s) 160 can be between about 110% and about 170%, such as less than 170%, 160%, 150%, 140%, 130%, 120%, or 110%, of a longitudinal length of the valve anchor when the valve anchor and the support frame are in the compressed configuration. Further, a longitudinal length or extent of the link mechanism(s) 160 can be between about 40% and about 130%, such as less than 130%, 120%, 110%, 100%, 90%, 80%, 70%, 60%, 50%, or 40% of a longitudinal length of the valve anchor when the valve anchor and the support frame are in the expanded configuration.

The present disclosure also describes various aspects of a delivery device for, e.g., transfemoral delivery of a valve prosthesis. The delivery device can support a valve prosthesis, such as the prosthesis 100 discussed above. The delivery device can comprise a proximal sheath component, a distal carrier assembly, and a control unit, wherein the distal carrier assembly is distal to the proximal sheath component, and the proximal sheath component is distal to the control unit. In some embodiments, the distal carrier assembly can comprise a conical or tapered end portion. The proximal sheath component and the distal carrier assembly can at least partially enclose the support frame and the valve anchor of the valve prosthesis prior to and during delivery of the valve prosthesis. The configuration of the delivery device with respect to the proximal sheath component and the distal carrier assembly, the support frame, and the valve anchor can allow the valve anchor and the support frame to be loaded or positioned serially along the longitudinal axis in a compact condition, thus enabling the delivery device to achieve a minimal crossing profile to reduce any difficulty in advancing the delivery device to the target location within the patient. The distance from which the valve anchor may be serially displaced from the support frame is highly variable. This may allow the user to minimize the radius of the delivery device which must be advanced through, for example, arteries and veins. Further, the valve anchor can be expanded and positioned independent of the support frame prior to positioning and releasing the support frame, as described in greater detail below. Additionally, a link mechanism that interconnects the valve anchor to the support frame can advantageously facilitate reliable positioning of the support frame relative to the valve anchor.

Referring now to Figures 4A-8D, the prosthesis 100 is illustrated loaded onto a delivery device 200. Using the delivery device 200, the valve prosthesis 100 can be delivered and expanded component by component in order to achieve the expanded configuration illustrated in Figure 1. During this component-by-component expansion process (illustrated in the delivery stages of Figures 11A-11F), the ability of the link mechanism 160 to move along the U-shaped members of the valve anchor 104 provides several distinct benefits. For example, one of these benefits is illustrated in Figures 4A-6E, which is that during delivery of the valve prosthesis 100, it is advantageous to have the valve anchor 104 positioned serially from the support frame 102 in order to achieve a minimal outer profile for the delivery device. This serial positioning allows the delivery device 200 to have a minimized outer diameter, which can allow the delivery device 200 to be more easily advanced through blood vessels.

As shown in Figures 4A-6E, the delivery device can carry the support frame, the valve anchor, and the link mechanism of the valve prosthesis. For example, Figure 4A shows that the delivery device 200 can carry the support frame 102, the valve anchor 104, and the link mechanism 160 of the valve prosthesis 100. The delivery device 200 can comprise one or more elongate core members that extend along a longitudinal axis of the delivery device 200.

The delivery device 200 can also comprise a proximal sheath component 204 (shown in dashed lines in Figure 4A to illustrate the underlying components and features of the prosthesis 100 and the delivery device 200) and a distal carrier assembly 206 (also shown in dashed lines in Figure 4A to illustrate the underlying components and features of the prosthesis 100 and the delivery device 200). The proximal sheath component 204 can be coupled to and extend distal to a control unit (illustrated in Figures 13A-13H), through which the clinician can control movement of the various components of the delivery device 200.

The distal carrier assembly 206 can be a two-part component that is configured to house at least one of the valve anchor or the support frame. The distal carrier assembly 206 comprise a proximal enclosure 210 (shown in dashed lines) and a distal enclosure 212 (shown in dashed lines). The proximal enclosure 210 can be coupled to a first core member 220, and the distal enclosure 212 can be coupled to a second core member 222. In some embodiments, the distal enclosure 212 can be threadedly and/or adhesively coupled or bonded to the second core member 222. For example, the second core member 222 may include a hollow shaft. The first and second core members 220, 222 can allow a clinician to manipulate the relative positions of the proximal and distal enclosures 210, 212. Together, the proximal sheath component 204 and the distal carrier assembly 206 can collectively house the valve anchor 104 and the support frame 102, respectively, during delivery of the valve prosthesis 100 to a target location within the body (e.g., discussed herein as the aortic valve annulus) and be actuated by the clinician to position and release the valve prosthesis 100. Further, the distal enclosure 212 can comprise a conical or tapered anterior or distal portion to facilitate movement through the vasculature. In some embodiments, the distal carrier assembly 206 can be referred to as a two-part distal enclosure or a split nose cone assembly. Optionally, the distal enclosure 212 can comprise, be formed from, or include features that comprise a radiopaque material such as platinum.

Figures 4A-6E show the delivery device 200 prior to delivery of the prosthesis 100, in a loaded configuration. As shown, the first and second core members 220, 222 extend through the delivery device 200 and are coupled at their distal ends to proximal and distal enclosures 210, 212, respectively, of the distal carrier assembly 206. As illustrated in these figures, the second core member 222 can be disposed within a lumen of the first core member 220 and slidable therewithin. Accordingly, the proximal enclosure 210 of the distal carrier assembly 206, as well as the proximal sheath component 204, can be slidable relative to the second core member 222 and the distal enclosure 212.

As also shown, the proximal sheath component 204 can extend distally over the valve anchor 104 to enclose the valve anchor 104 within a lumen of the proximal sheath component 204 and maintain the valve anchor 104 in a compressed state. The lumen of the proximal sheath component 204 may also be referred to as a proximal sheath lumen. The proximal sheath component 204 can be retracted relative to the valve anchor 104 in order to permit the base portions of the U-shaped members of the valve anchor 104, thereafter to expand and later be maneuvered into position within the aortic sinuses.

As also illustrated in Figures 4-6E (see also Figures 3A-3C), the delivery device 200 can comprise at least one grasper 224 that can engage with and control positioning of the valve anchor 104. The grasper 224 can comprise distal ends, pinchers, or hooks 226 at its distal end that can be coupled to the peak portions of the U-shaped members of the valve anchor 104. For example, the pinchers 226 of the grasper 224 can be coupled to the anchor retention component 170 at the engagement area of the valve anchor 104 to cause the grasper 224 to be engaged with the valve anchor 104. The number of graspers 224 preferably equals the number of engagement areas 150 or U-shaped members 140 of the valve anchor 104. Each of the graspers 224 can comprise a tubular enclosure 228 through which a pair of wires, which terminate in the pinchers or hooks 226, passes. The wires can be pulled proximally relative to the tubular enclosure 228 in which the wires are housed in order to tighten the pinchers 226 around the anchor retention component 170, thus engaging the valve anchor 104. In order to release the pinchers 226, the wires can be shifted distally relative to the tubular enclosure 228 thereby allowing the pinchers 226 to spring open radially and release the anchor retention component 170. The distal end of each of the graspers 224 can enclose or be coupled to a hook of the valve anchor 104.

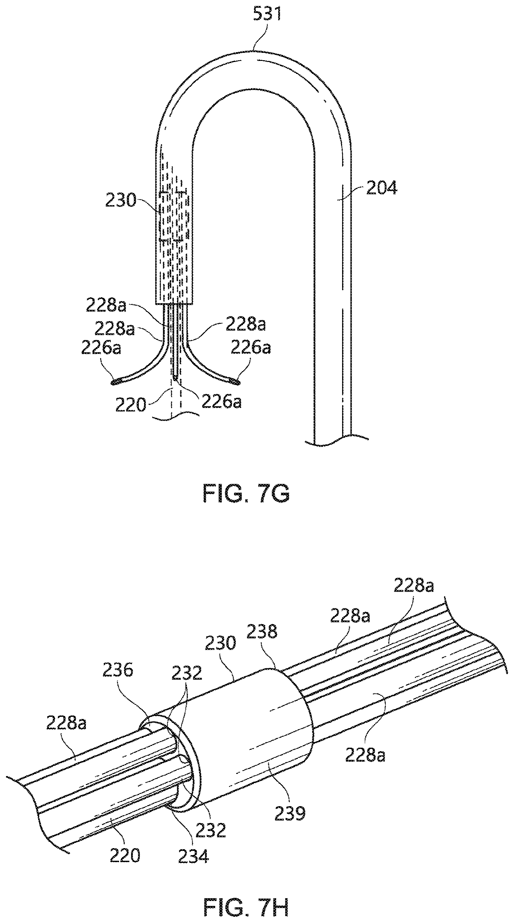

The interconnection between the distal ends, pinchers, or hooks 226 of the graspers 224 and the valve anchor 104 can permit the valve anchor 104 of the support frame 102 to be held in a stationary and/or compressed position relative to or within the proximal sheath component 204. As discussed further below, for example, with regard to Figures 7G-7I, this engagement can maintain the engagement areas 150 in a common plane 152, oriented generally perpendicular relative to the longitudinal axis of the delivery device 200. Additionally, when the proximal sheath component 204 is proximally retracted relative to the distal ends of the graspers 224, the valve anchor 104 can begin to expand, however, the engagement between the graspers 224 and the engagement areas 150 can allow a clinician to push, pull, or rotate the valve anchor relative to the delivery device 200 before fully releasing the valve anchor 104 from engagement with the delivery device 200. For example, this can allow the clinician to rotate or push the base portions 144 of the valve anchor 104 into the nested position within the aortic sinuses, as discussed above. Thereafter, once in the nested position, the engagement areas 150 of the valve anchor 104 can be released from the pinchers 226 of the graspers 224, and the valve anchor 104 can fully expand and be released into apposition with the native valve annulus.

In some embodiments, as illustrated in Figures 4B and 4C, the delivery device 200 can include a link mechanism 160a that interconnects the support frame 102 and the valve anchor 104 without a tension member. As illustrated in Figure 4B, a link mechanism 160a can be woven between the support frame 102 and the valve anchor 104. In the depicted example of Figure 4B, the link mechanism 160a can be coupled to an attachment point 221a on the support frame 102, extend toward and loop around a first leg of the valve anchor 104, then toward a second leg of the valve anchor 104, and then extends back toward the attachment point 221a. The link mechanism 160a can be tied, looped, or wound around the support frame 102 at the attachment point 221a. In some embodiments, one or more of the ties, loops, or windings of the link mechanism 160a can be fixed relative to or capable of sliding along the leg(s) of the valve anchor 104.

In some embodiments, as illustrated in Figure 4C, the link mechanism 160a can attach to the support frame 102 at two different attachment points 221b, 221c. For example, as shown in Figure 4C, the link mechanism 160a can be coupled to the support frame 102 at a first attachment point 221b of the support frame 102, extend toward and loop around the first leg of the valve anchor 104, then extend toward and loop around the second leg of the valve anchor 104, and then extend toward a second attachment point 221c of the support frame 102. An intermediate segment 196a can interconnect the loops around the first leg and the second leg of the valve anchor 104.

In some embodiments, as illustrated in Figures 4D and 4E, the delivery device 200 can include a link mechanism 160b that interconnects the support frame 102 and the valve anchor 104 while being fixed or coupled relative to the leg(s) of the valve anchor 104. As illustrated in Figure 4D, the link mechanism 160b can be coupled to an attachment point on the support frame 102 and extend toward an engagement area 150 of the valve anchor 104. The link mechanism 160b can be tied, looped, wound or otherwise attached to the support frame 102 and the valve anchor 104 at the attachment points.

In the depicted example, the link mechanism 160b can be an elastic interconnect formed from silicone, polyurethane, or any other suitable elastic material. When the support frame 102 is moved relative to the valve anchor 104, the link mechanism 160b can stretch, flex, or otherwise elastically stretch. As the link mechanism 160b stretches, the support frame 102 can move longitudinally and/or rotationally relative to the valve anchor 104.

In some embodiments, as illustrated in Figure 4E, the delivery device 200 can include a link mechanism 160c that interconnects the support frame 102 and the valve anchor 104. The link mechanism 160c can include a resilient or coiled portion 161c. For example, as shown in Figure 4E, the coiled portion 161c is proximal to the engagement area 150. The coiled portion 161c can allow for the link mechanism 160c to stretch to a longer length and/or minimize length of the link mechanism 160c in an unstretched state. In some embodiments the coiled portion 161c can be a spring mechanism and may include laser cut patterns.

In some embodiments, as illustrated in Figures 4F and 4G, the delivery device 200 can include a link mechanism 160d that interconnects the support frame 102 and the valve anchor 104 and a one-way interconnect to maintain tension on the link mechanism 160a.

As illustrated in Figure 4F, a link mechanism 160d can be coupled to the support frame 102, e.g., at an attachment point thereof, and extend toward an engagement area 150 of the valve anchor 104. The link mechanism 160d can be received by a one-way interconnect 161a to permit movement of the link mechanism 160d in a proximal direction through the interconnect 161a while restricting or preventing distal advancement or return of the link mechanism 160d through the interconnect 161a. In some embodiments, the link mechanism 160d can comprise a proximal actuation portion 161b that is coupled or extends to the link mechanism 160d. For example, the link mechanism 160d and the proximal actuation portion 161b can be sections of a continuous line, wire, or suture that passes through the one-way interconnect 161a. Further, the link mechanism 160d and the proximal actuation portion 161b can be flexible or rigid.

In accordance with some embodiments, the one-way interconnect 161a can comprise a ratcheting mechanism. For example, as illustrated in the inset figure of Figure 4F, the interconnect 161a can comprise a body having an aperture through which the link mechanism 160d can pass, and the ratcheting mechanism can have a toothed structure extending into the aperture that permits movement of the link mechanism 160d in one direction, but engages the link mechanism 160d to restrict movement in an opposite direction.

In use, for example, the clinician can distally advance the valve anchor 104 relative to the support frame 102 (using a motion and features of the embodiments disclosed herein), which can result in some slack in the link mechanism 160d. The clinician can then grasp and pull the proximal actuation portion 161b, which will cause the link mechanism 160d to be drawn proximally through (e.g., ratcheted through) the interconnect 161a, thereby reducing the length of the link mechanism 160d between the interconnect 161a and the attachment point on the support frame 102. Accordingly, the clinician can draw the support frame 102 and the valve anchor 104 together upon exerting a proximally oriented force on the proximal actuation portion 161b while maintaining the valve anchor 104 stationary. This motion and ratcheting of the link mechanism 160d can restrict motion of the valve anchor 104 relative to the support frame 102. In this manner, as the valve anchor 104 is drawn towards the support frame 102, the relative longitudinal positions of these components can be restricted or fixed in a desirable relative position in anticipation of releasing the prosthesis at the implantation site.

In some embodiments, as illustrated in Figure 4G, a link mechanism 160e can be coupled to the support frame 102, e.g., at an attachment point thereof, and extend toward an engagement area 150 of the valve anchor 104. The link mechanism 160e can be woven between the support frame 102 and the valve anchor 104 (e.g., the link mechanism 160e can extend from the valve anchor 104 and be looped through the support frame 102, similar to or such as by a pulley-type configuration).

In the depicted example of Figure 4G, the link mechanism 160e can be coupled to the one-way interconnect 161e, extend toward and loop around the support frame 102 and then toward the one-way interconnect 161e disposed proximal to the engagement area 150 of the valve anchor 104 in a pulley mechanism. Similarly, the link mechanism 160e can be received by the one-way interconnect 161e to permit movement of the link mechanism 160e in a distal direction through the interconnect 161e while preventing the proximal movement of the link mechanism 160e through the interconnect 161e. In some embodiments, the link mechanism 160e can comprise a proximal actuation portion 161f that is coupled or extends to the link mechanism 160e. For example, the link mechanism 160e and the proximal actuation portion 161f can be sections of a continuous line, wire, or suture that passes through the one-way interconnect 161e. Further, the link mechanism 160e and the proximal actuation portion 161f can be flexible or rigid. In some embodiments, an end 161g of the link mechanism 160e can be coupled to the interconnect 161e to secure the link mechanism 160e thereto, thus enabling the clinician to draw the support frame 102 and the valve anchor 104 together upon exerting a proximally oriented force on the proximal actuation portion 161f while maintaining the support frame 102 or the valve anchor 104 stationary.

In accordance with some embodiments, the one-way interconnect 161e can comprise a rotating ratcheting mechanism. For example, as illustrated in the inset figure of Figure 4G, which illustrates the pulley mechanism of the interconnect 161e, the interconnect 161e can comprise a body having an aperture through which the link mechanism 160e can pass, and the ratcheting mechanism can have a toothed wheel structure extending into the aperture that permits movement of the link mechanism 160e in one direction, but engages the link mechanism 160e to restrict movement in an opposite direction.

In use, for example, the clinician can distally advance the valve anchor 104 relative to the support frame 102 (using a motion and features of the embodiments disclosed herein), which can result in some slack in the link mechanism 160e. The clinician can then grasp and pull the proximal actuation portion 161f, which will cause the link mechanism 160e to be drawn proximally through (e.g., ratcheted through) the interconnect 161e, thereby reducing the length of the link mechanism 160e between the interconnect 161e and the attachment point on the support frame 102. Alternatively, the clinician can simply pull proximally on the proximal actuation portion 161f to draw or force distal advancement of the valve anchor 104 relative to the support frame 102, which can reduce the length of the link mechanism 160e between the interconnect 161e and the attachment point on the support frame 102. Either of these types of motion and ratcheting of the link mechanism 160e can restrict motion of the valve anchor 104 relative to the support frame 102. In this manner, as the valve anchor 104 can be drawn towards the support frame 102, the relative longitudinal positions of these components can be restricted or fixed in a desirable position in anticipation of releasing the prosthesis at the implantation site.

Referring now to Figure 5, a side cross-sectional view is provided of the valve prosthesis 100 loaded onto the delivery device 200, taken along section lines 5-5 of Figure 4A, according to some embodiments. Among the many features illustrated in Figure 5, Figure 5 shows that the proximal enclosure 210 extends over both the valve anchor 104 and the support frame 102. Thus, in accordance with some embodiments, in the compressed or delivery configuration shown in Figure 5, the link mechanism can extend between the valve anchor 104 and the support frame 102 and be at least partially enclosed within the proximal enclosure 210 (depending on the attachment point of the link mechanism with the support frame 102 and the longitudinal extent of the proximal enclosure 210).

Although not all of the various types of link mechanisms are illustrated in Figure 5, any of the link mechanisms disclosed herein, such as those illustrated in Figures 4A-4G, can be enclosed within the proximal enclosure 210 in this manner. This can advantageously protect the link mechanism from any damage or entanglement during advancement of the delivery device 200 to the target area.

In addition, Figure 5 illustrates that the valve anchor 104 can comprise a link motion limiter 240. The link motion limiter 240 can provide an enlarged profile of the wireframe structure of the valve anchor 104 so as to restrict or prevent motion of the link mechanism as the link mechanism slides along the U-shaped member of the valve anchor 104.

In alternative embodiments of the delivery device 200, the valve anchor 104 and the support frame 102 can both be enclosed within the proximal sheath component 204 prior to and during delivery prior to releasing the valve anchor 104. For example, in some embodiments, the valve anchor 104 can be distal to the support frame 102 wherein the valve anchor 104 is near the distal end of the proximal sheath component 204 and the support frame 102 can be approximately adjacent to the valve anchor 104 (in a serial configuration) and is proximal to the valve anchor 104. In some embodiments of the delivery device 200, the valve anchor 104 and the support frame 102 can both be enclosed within the proximal sheath component 204, with the support frame 102 near the distal end of the proximal sheath component 204 and the valve anchor 104 being approximately adjacent to the support frame 102 and proximal to the support frame 102.

Further, in alternative embodiments of the delivery device 200, the valve anchor 104 can be enclosed within the distal carrier assembly 206 and the support frame 102 can be enclosed within the proximal sheath component 204 prior to and during delivery of the valve prosthesis. For example, in some embodiments of the delivery device 200, both the valve anchor 104 and the support frame 102 can be enclosed within the distal carrier assembly 206 and the support frame 102 can be enclosed within the proximal sheath component 204 prior to and during delivery of the valve prosthesis. In this configuration, the valve anchor 104 and the support frame 102 can be approximately adjacent to one another (in a serial configuration) and the valve anchor 104 can be positioned proximal to the support frame 102.

Some embodiments can advantageously facilitate realignment and repositioning of the proximal sheath component 204 after the valve anchor 104 has been released from the delivery device 200.

For example, after the valve anchor 104 has been positioned within the native valve sinuses and released from the graspers (thereby releasing the valve anchor 104 from the delivery device 200), the proximal sheath component 204 can be advanced distally relative to the distal carrier assembly 206 until a distal end portion 208 of the proximal sheath component 204 is pushed into contact with a proximal abutment surface 214 of the proximal enclosure 210.

Figure 6A-6E illustrate the various positions and actuation of link mechanisms during a deployment sequence of the valve prosthesis. The link mechanism attachment pattern can vary from that discussed and illustrated with respect to other embodiments disclosed herein; however, the embodiments shown in Figure 6A-6E illustrated movement and positioning of the link mechanism and related features that can be incorporated into one or more embodiments disclosed herein.

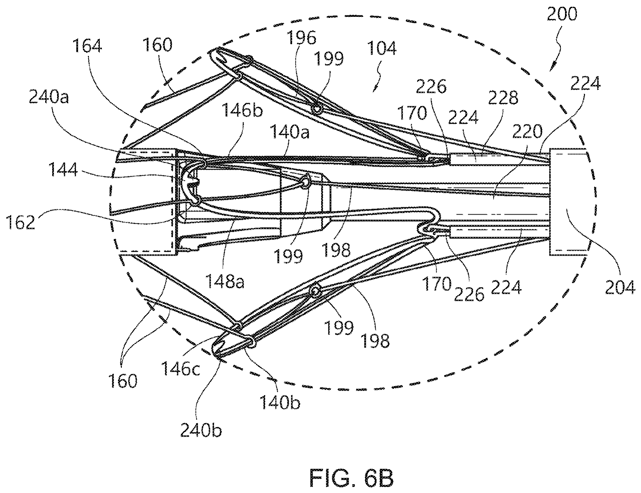

For example, Figures 6A and 6B illustrate the delivery device 200 in a configuration in which the proximal sheath component 204 is proximally retracted relative to the valve anchor 104, which permits the valve anchor 104 to begin to expand from a compressed, loaded configuration. As illustrated in Figures 6A and 6B, the valve anchor 104 can optionally be configured such that the U-shaped member's base portion 144 comprises a link motion limiter 240a (see also 240b) that extends from a medial location of the base portion 144. The link motion limiter 240a, 240b can provide an enlarged profile of the wireframe structure of the valve anchor 104 that restricts or prevents movement of a loop (see e.g., loops 162 and 164, also shown in Figure 3A) of the link mechanism 160 as the loop slides along the base portion 144 of the valve anchor 104.

For example, the link motion limiter 240a can tend to prevent the loops of the link mechanism 160 from both sliding onto a single leg of the U-shaped member 140. This will allow the loops (e.g., loops 162, 164) of the link mechanism 160 to move only toward the respective engagement areas of the valve anchor 104, thereby properly positioning the link mechanism 160 within the respective engagement areas and ensuring proper motion and deployment of the valve anchor 104.

In some embodiments, each of the first, second, and third U-shaped members of the valve anchor 104 can comprise a respective link motion limiter. Further, the link mechanism 160 can be coupled to each of the respective U-shaped members on opposing sides of the respective link motion limiters (see e.g., link motion limiter 240a and loops 162, 164 in Figure 6B).

Furthermore, in some embodiments, the link motion limiter 240a, 240b can extend from the base portion 144 in a proximal direction toward the peak portions of the valve anchor 104, as shown in Figures 6A and 6B. However, the link motion limiter 240a, 240b can also extend from the base portion 144 in a distal direction away from the peak portions of the valve anchor 104. As can be appreciated, while the link motion limiter 240a, 240b extends from the base portion 144, the U-shape members of the valve anchor 104 generally retain and can be considered "u-shaped." Optionally, the link motion limiter can include features to enable functionality as a latch, clasper tang or other attachment point for a grasper component, as described herein.

Figures 6A and 6B also illustrate that the link mechanism 160 can define a weave pattern that interconnects the valve anchor 104 with the support frame 102. For example, the link mechanism 160 extends (i) from a first circumferential attachment position 242 on the support frame 102, (ii) to a first leg 146b of a first U-shaped member 140a (on a first side of the link motion limiter 240a of the first U-shaped member 140a), (iii) then hooking into the flexible loop 199 of the tension member 198 and then to a second leg 148a of the first U-shaped member 140a (on a second side of the link motion limiter 240a of the first U-shaped member 140a), (iv) then to a second circumferential attachment position 244 on the support frame 102, and (v) then to a first leg 146c of a second U-shaped member 140b on a second side of the link motion limiter 240b. The link mechanism can continue in this pattern and extend to a second leg of a third U-shaped member and then to a third circumferential attachment position on the support frame 102 (not shown).

As also illustrated in Figure 6A, the first and second circumferential attachment positions 242, 244 can be located adjacent to, within, or along a medial portion of the support frame 102. In particular, the first and second circumferential attachment positions 242, 244 can be exposed through a window or gap 248 between the proximal and distal enclosures 210, 212 when the prosthesis 100 is in the compressed or delivery configuration. However, the first and second circumferential attachment positions 242, 244 can be located adjacent to, within, or along a proximal or distal end portion of the support frame 102 such that the first and second circumferential attachment positions 242, 244 are covered by one of the proximal or distal enclosures 210, 212 during delivery to the target location.

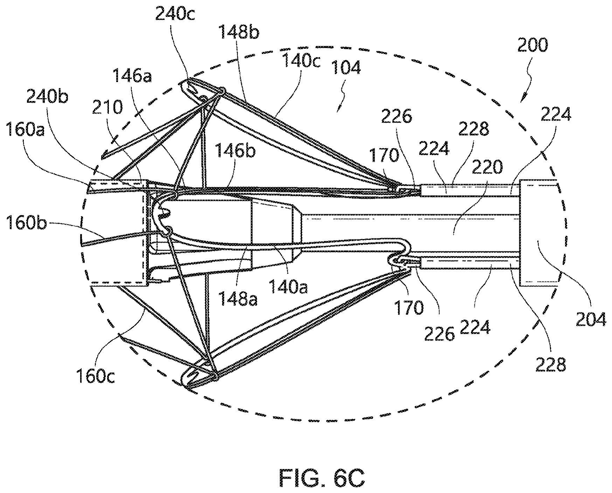

Figure 6C is an enlarged detail view of the valve prosthesis and delivery device of Figure 4B. Figure 6C illustrates a delivery device 200 with link mechanisms 160a, 160b without tension members. Each link mechanism 160a can define a weave pattern that interconnects the valve anchor 104 with the support frame 102. For example, the link mechanism 160a extends (i) from a first circumferential attachment position (e.g., 242, as in Figure 6A) on the support frame 102, (ii) to a first leg 146b of a first U-shaped member 140a (on a first side of the link motion limiter 240b of the first U-shaped member 140a), (iii) then extending directly to a second leg 148b of a second U-shaped member 140c (on a second side of a link motion limiter 240c of the second U-shaped member 140c), (iv) then back to the first circumferential attachment position (e.g., 242, as in Figure 6A) on the support frame 102 (or alternatively, to a different circumferential attachment point on the support frame 102).

Additional link mechanisms 160b, 160c can weave a similar pattern between the U-shaped members and circumferential attachment positions on the support frame 102.

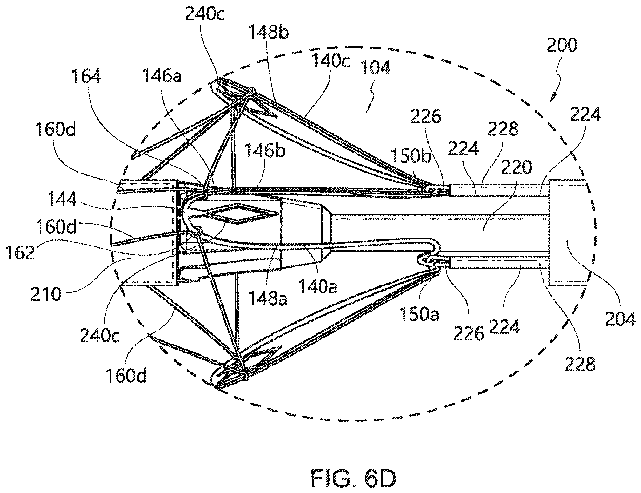

Figure 6D illustrates a delivery device 200 with a diamond shaped link motion limiter 240c that extends from a medial location of the base portion 144. In the depicted example, the link motion limiter 240c can provide an elongated diamond shaped profile of the wireframe structure of the valve anchor 104 that restricts or prevents movement of a loop 162, 164 of the link mechanism 160d sliding along the base portion 144 of the valve anchor 104. For example, the link motion limiter 240c can tend to prevent the loops 162, 164 of the link mechanism 160d from both sliding onto a single leg of the U-shaped member 140, while allowing the loops 162, 164 of the link mechanism 160d to freely move toward the respective engagement areas of the valve anchor 104, thereby properly positioning the link mechanism 160d within the respective engagement areas 150 and ensuring proper motion and deployment of the valve anchor 104.

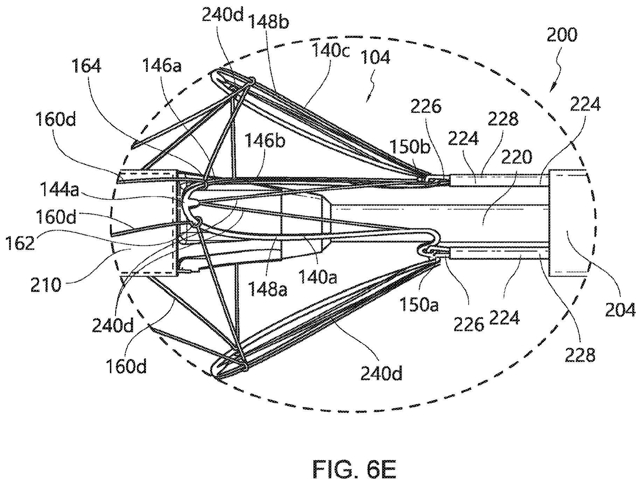

Figure 6E illustrates a delivery device 200 with a barrier suture 240d for maintaining a loop (e.g., 162, 164) of a link mechanism on a single leg of a U-shaped member. The barrier suture 240d can be a strand of material that is attached to the valve anchor 104. However, the barrier suture 240d can be formed as a single, continuous portion of the valve anchor 104, for example, as a single, continuous piece of material.

In Figure 6E, the valve anchor 104 comprises a plurality of barrier suture 240d, which extend from medial locations 144a of the U-shaped members 140a, 140b, 140c toward respective legs thereof adjacent to the engagement area of each U-shaped member. For each U-shaped member, for example, a barrier suture 240d can extend from a first location, adjacent to a first engagement area 150b on a first leg 146b, proximal to a medial location 144a of the base portion and, whether as a single, continuous strand or as a second, separate strand, the barrier suture 240d can extend from the medial location 144a to a second location on a second leg 148a, proximal to the second engagement area 150a.

In the depicted example of Figure 6E, the barrier suture 240d restricts or prevents movement of loops 162, 164 of the link mechanism 160d from sliding past or beyond the medial location 144a of the valve anchor 104 onto a different leg 146b, 148a of the U-shaped member 140a. For example, the barrier suture 240d can tend to prevent the loops 162, 164 from both sliding onto the same leg 146b, 148a of the U-shaped member 140a, while allowing the loops 162, 164 to freely move toward the respective engagement areas 150a, 150b of the valve anchor 104, thereby properly positioning the link mechanism 160 within the respective engagement areas and ensuring proper motion and deployment of the valve anchor 104.

Figures 7A-7K illustrate optional aspects of a delivery device, according to at least one embodiment. These figures do not illustrate all of the components of the delivery device that can be incorporated into an embodiment. However, the features illustrated in these figures can be incorporated into embodiments of the delivery device to facilitate engagement with the valve anchor and/or facilitate delivery and control of the valve anchor during implantation and release of the valve anchor at the target location.

For example, Figures 7A-7D illustrate an embodiment of a delivery device 200a that comprises a grasper mechanism. The grasper mechanism can be used to securely couple a portion of the valve anchor with the delivery device to permit the clinician to control movement, operation, and deployment of the valve anchor. The grasper mechanism can engage one or more portions or structures of the valve anchor using a variety of coupling mechanisms, which can use attachment means including mechanical engagement, dissolvable structures, chemically reactive degradable structures, electrolytically degradable structures, and the like.

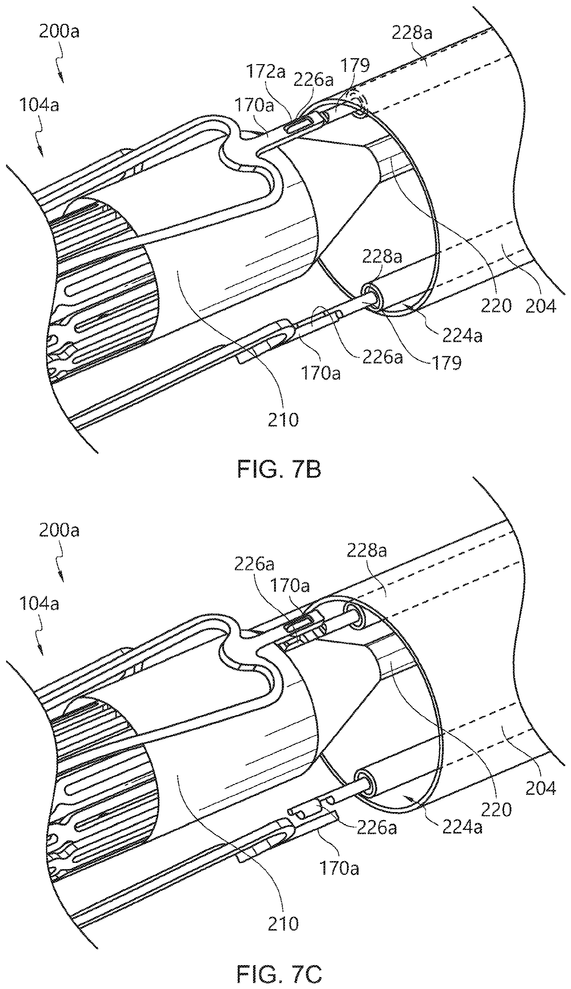

In some embodiments, the grasper mechanism can be a tubular grasper mechanism. The delivery device 200a, shown in Figure 7A, can comprise a grasper 224a that can engage with and control the longitudinal position of the valve anchor 104a, as shown in Figure 7A. Figures 7B and 7C illustrate states of disengagement and features of components of the grasper 224a, which are hidden in Figure 7A due to the grasper 224a and the valve anchor 104a being in an engaged configuration in Figure 7A.

As shown in Figures 7B and 7C, the grasper 224a of the delivery device 200a can comprise an engagement wire 179 that is movable within a lumen of a tubular enclosure 228a. The valve anchor 104a can be configured to comprise a clasper tang 170a extending from an engagement area 150d of the valve anchor 104a. The engagement wire 179 can comprise a distal end portion that includes pins, ridges, or protrusions 226a that can be coupled to the engagement structure 172a of the clasper tang 170a at the engagement area of the valve anchor 104a.

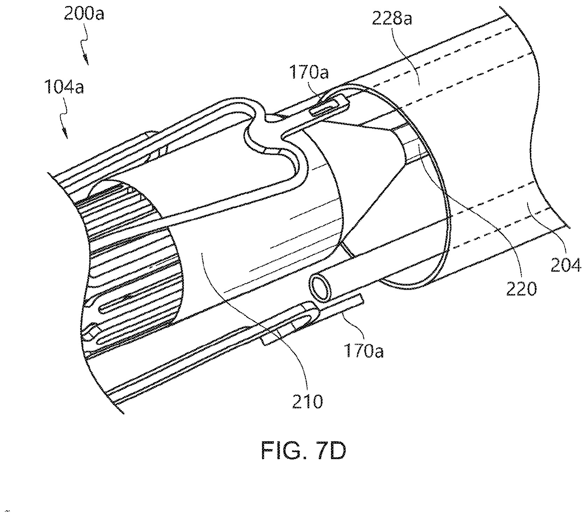

When engaged together, the engagement wire 179 and the clasper tang 170a can be proximally drawn into the lumen of the tubular enclosure 228a, which secures the engagement wire 179 and the clasper tang 170a relative to each other in both radial and longitudinal directions. However, when the engagement wire 179 and the clasper tang 170a are moved outside of the lumen of the tubular enclosure 228a, as shown in Figure 7D, the engagement wire 179 and the clasper tang 170a can be disengaged as the valve anchor 104a and the clasper tang 170a expand radially, thereby disengaging the clasper tang 170a from the engagement wire 179.

Referring to Figure 7A, each wire 179 can be pulled or positioned proximally within or relative to the lumen of its tubular enclosure 228a in which the wires 179 are housed. In some embodiments, the engagement area of the valve anchor 104a is positioned within the tubular enclosure 228a, securing the ridges or protrusions 226a within the openings of the clasper tangs 170a by limiting the relative motion of the ridges or protrusions 226a with respect to the openings of the clasper tangs 170a, thus engaging the valve anchor 104a.

In some embodiments, the clasper tangs 170a can be coupled along a radial outer region of the engagement wires 179, as shown in Figure 7B. Further, the cross-sectional area of the tubular enclosure 228a is small enough to prevent the protrusions 226a from disengaging from the clasper tangs 170a in a radial direction while allowing the collective cross-section of the engaged valve anchor 104a to move within or relative to the tubular enclosure 228a while maintaining the longitudinal locking or engagement between the engagement wire 179 and the clasper tang 170a.

In accordance with at least one embodiment, the graspers 224a can permit a clinician to push, pull, or rotate the valve anchor 104a relative to the delivery device 200a before fully releasing the valve anchor 104a from engagement with the delivery device 200a.

Referring again to Figure 7B, each wire 179 can be advanced distally within or relative to the lumen of its tubular enclosure 228a such that the ridges or protrusions 226a and the openings of the clasper tangs 170a are outside of the lumen of the tubular enclosure 228a. By advancing each wire 179 relative to its tubular enclosure 228a, so that the protrusions 226a and the clasper tangs 170a are outside of the lumens of the tubular enclosures 228a, the clasper tangs 170a can begin to self-expand and radially separate from the protrusions 226a, thereby detaching from the protrusions 226a to release the valve anchor 104a, as shown in Figure 7C. Upon release of the valve anchor 104a, the wires 179 can be retracted into the tubular enclosure 228a, as shown in Figure 7D.

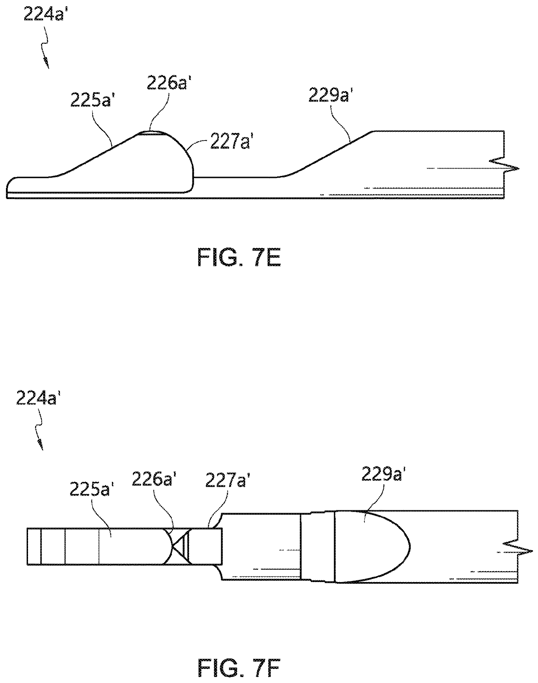

Figures 7E and 7F illustrate aspects of a grasper 224'. As illustrated, the grasper 224' has a profile to facilitate a connection with the valve anchor 104a when loading the prosthesis onto the delivery device. In some embodiments, the grasper 224a' has a generally smooth profile. As illustrated in Figures 7E and 7F, the tang of the grasper 224a' has a protrusion 226a' with an angled distal portion 225a' and a rounded proximal portion 227a' to facilitate interconnection with the valve anchor 104a.

For example, in some embodiments, the tang of the grasper 224a' can further include an angled distal portion 229a'. Advantageously, the angled and rounded features of the profile of the grasper 224a' can promote smooth engagement and disengagement with the valve anchor 104a. In some embodiments, the engagement surfaces of the grasper 224a' can be electropolished.

In accordance with at least one embodiment disclosed herein is the realization that when the delivery device is moved through a blood vessel, the delivery device may be bent or curved as it passes through a tortuosity of the blood vessel. When this happens, the graspers may tend to move radially within the proximal sheath component, which may cause the distal or engagement ends of the graspers to become longitudinally displaced or misaligned relative to each other. This may cause bending or misalignment of the valve anchor during delivery, which may make placement more challenging.

More specifically, in at least one embodiment, the grasper mechanisms may extend from a handle actuator (see e.g., Figures 13A-13H) from a common plane (that may be oriented generally perpendicular relative to a longitudinal axis of the delivery device) and have approximately equal longitudinal lengths. Additionally, each of the grasper mechanisms may originate from a given radial or circumferential position, sector, or quadrant within the proximal sheath component. If the delivery device extends along a straight path, the distal or engagement ends of the graspers will also be aligned in a common plane that is oriented generally perpendicular relative to the longitudinal axis of the delivery device thereby engaging with the areas of the valve anchor, as shown in Figure 5.

However, in use, if the proximal sheath component bends (as shown in Figure 7G), e.g., due to a tortuosity of the blood vessel, each of the grasper mechanisms may be moved out of its original circumferential or radial position, sector, or quadrant within the proximal sheath component. This occurs because each individual grasper mechanism will tend to extend in a straight, linear path within the proximal sheath component and only bend if the grasper mechanism hits the inner wall of the proximal sheath component. As a result, the grasper mechanisms may converge against an outer wall of a bend in the proximal sheath component, which can change the relative positioning of the distal or engagement ends of the graspers. Accordingly, instead of being aligned or positioned within a common plane that is oriented generally perpendicular relative to a longitudinal axis of the proximal sheath component, the distal or engagement ends of the graspers may be positioned out of plane or simply out of the common plane that extends perpendicular relative to the longitudinal axis of the proximal sheath component. Such a misalignment can create stress, misalignment, unintentional disengagement, or bending of the valve anchor, whose engagement areas are most preferably maintained within a common plane (that is oriented generally perpendicular relative to a longitudinal axis of the proximal sheath component) during delivery.

Therefore, at least one embodiment can be configured to include an alignment hub that can maintain or fix a longitudinal position of at least a portion of the grasper mechanisms along distal portion of the grasper mechanisms. In this manner, although intermediate portions of the grasper mechanisms may be radially misaligned or displaced from their original radial or circumferential position, sector, or quadrant within the proximal sheath component, the alignment hub can advantageously serve to reset or realign the positions of the grasper mechanisms so that the longitudinal positions of the can be located within substantially the same or common plane positioned perpendicularly relative to a longitudinal axis of the proximal sheath component.

For example, Figures 7G-7I illustrate a distal hub or grasper alignment hub 230 that may optionally be used in at least one embodiment of the delivery device. As illustrated, the grasper alignment hub 230 can be utilized to fix or maintain the distal ends of the graspers 226a in a desirable common plane to allow for desired and/or predictable engagement and disengagement of the graspers 226a with the valve anchor 104. In particular, the grasper alignment hub 230 can allow the distal ends of the graspers 226a to be maintained in a common plane despite bending of a proximal sheath component 204 530 as is manipulated through or past tortuosities of a blood vessel, e.g., at a bend.

As illustrated in Figures 7G and 7H, the grasper alignment hub 230 can be bonded or secured relative or directly to the tubular enclosures 228a of the graspers 226a near the distal section of the delivery device. In some embodiments, the grasper alignment hub 230 can be secured to the tubular enclosures 228a at least approximately 1 inch, 2 inches, 3 inches, 3.5 inches, or 4 inches from the distal end of the delivery device.

Referring to Figure 7H, the graspers 226a and the corresponding tubular enclosures 228a extend through the grasper alignment hub 230, between a first or proximal end 236 to a second or distal end 238 through tubular enclosure passages 232 formed through the grasper alignment hub 230. The tubular enclosures 228a can be bonded or mechanically coupled to the tubular enclosure passages 232 so as to engage or secure the tubular enclosures 228a relative to the grasper alignment hub 230. Even though the tubular enclosures 228a may be constrained from longitudinal movement along the distal section of the delivery device relative to the grasper alignment hub 230, in some embodiments, the graspers 226a may still be permitted to move within the tubular enclosures 228a to facilitate engagement and/or disengagement with the valve anchor. However, because the tubular enclosures 228a are constrained from sliding movement, the distal ends of the graspers 226a may also be restricted from being longitudinally displaced relative to each other when the proximal sheath component 204 moves through or is moved into a curve or tortuosity. tend to be longitudinally displaced relative to each other less than in an embodiment

As illustrated, the core member 220 can pass through the first or proximal end 236 to the second or distal end 238 through a core member passage 234 formed through the grasper alignment hub 230. The core member 220 can be movable through the core member passage 234. For example, in some embodiments, the grasper alignment hub 230 can move relative to the core member 220 while the tubular enclosures 228a are secured to the grasper alignment hub 230.

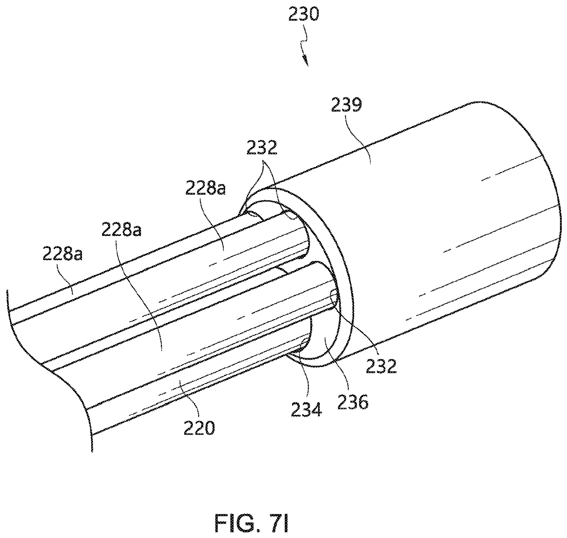

Referring to Figure 7I, the outer surface 239 of the grasper alignment hub 230 can have a generally cylindrical shape. In some embodiments, the cylindrical shape of the outer surface 239 can allow the grasper alignment hub 230 to move freely within the catheter 530. As illustrated, the tubular enclosure passages 232 and the core member passage 234 can be arranged to maintain a desired alignment between the tubular enclosures 228a and the core member 220 as the catheter 530 is manipulated during introduction and delivery Further, in at least one embodiment, the cross-sectional arrangement of the tubular enclosure passages 232 and the core member 220 at the grasper alignment hub 230 can be substantially similar to their cross-sectional arrangement at the handle actuator.

Additionally, in accordance with at least one embodiment, the arrangement of the tubular enclosure passages 232 and the core member passage 234 and the grasper alignment hub 230 can further reduce or avoid twisting or misalignment of the tubular enclosures 228a and the core member 220 relative to the longitudinal axis of the lumen of the catheter 530.

As illustrated, in at least one embodiment, the tubular enclosure passages 232 may be disposed adjacent to each other, offset from a central axis of the grasper alignment hub 230, along an arc bordering a periphery of the grasper alignment hub 230. The core member passage 234 can have a cross-sectional diameter larger than the respective cross-sectional diameters of the tubular enclosure passages 232. The core member passage 234 can be offset from the central axis of the grasper alignment hub 230 and be spaced apart from the tubular enclosure passages 232. Such a configuration can allow the grasper alignment hub 232 have a minimal cross-sectional diameter while permitting each of the tubular enclosure passages 232 and the core member passage 234 to be circumscribed therewithin.

However, in at least one embodiment, the tubular enclosure passages 232 may be disposed circumferentially around the core member passage 234. Optionally, the tubular enclosure passages 232 may be equidistantly disposed.

Figures 7J and 7K illustrate an embodiment of a delivery device 200b that comprises a grasper mechanism that may engage with a valve anchor 204b. In some embodiments, valve anchor 204b may comprise a clasper tang located at the base portion of the U-shaped members, as discussed above with respect to Figure 2H. Optionally, the valve anchor 204b may be similar or identical to the valve anchor 104g described in Figure 2H, the features of which are not discussed again here for brevity, but may be realized in such a combination.

With reference to Figure 7J, the delivery device 200b is shown with proximal sheath extending over both the valve anchor 204b and the support frame 102. Thus, in accordance with some embodiments, in the compressed or delivery configuration shown in Figure 7J, the link mechanism can extend between the valve anchor 204b and the support frame 102 and be at least partially enclosed within the proximal enclosure 210. In alternative embodiments of the delivery device 200b, the valve anchor 204b and the support frame 102 can both be enclosed within the proximal sheath component prior to and during delivery prior to releasing the valve anchor 204b.

For example, the delivery device 200b, shown in Figure 7J, can comprise a grasper 224b that can extend across the valve anchor 204b to engage with and control the base portion of the valve anchor 204b. Optionally, the proximal enclosure 210 can be recessed or have a reduced diameter portion to permit the valve anchor 204b and the graspers 224b attached thereto to have a lower profile in a compressed or undeployed configuration.

With reference to Figure 7K, the delivery device 200b is shown with the valve anchor 204b in a deployed configuration. In some embodiments, by engaging the base portion of the valve anchor 204b, the grasper 224b can precisely engage with and control the longitudinal position of the valve anchor 104b. Thus, the graspers 224b can be used to control the articulation of the valve anchor 204b as desired by the clinician.

Further, in some embodiments, by allowing the graspers 224b to engage the base portion of the valve anchor 204b, the motion of the link mechanism 160 can be limited to the desired portion of the valve anchor 204b. Thus, the link mechanism 160 will not slide along an undesired path or leg of the U-shaped member, thus tending to ensure that the link moves as intended.

Advantageously, the grasper mechanism can be used to securely couple a portion of the valve anchor with the delivery device. This can beneficially improve maneuverability and permit the clinician to control movement, operation, and deployment of the valve anchor while preventing inversion of the valve anchor 204b.

Accordingly, in at least one embodiment, a clinician can manipulate the valve anchor by engaging or coupling an engagement portion or protrusion of a grasper with a clasper tang of a valve anchor. The engagement portion and the clasper tang can be restricted from relative radial movement (to thereby remain longitudinally engaged and secured relative to each other) by enclosing the engagement portion and the clasper tang within a tubular enclosure. In order to disengage the engagement portion and the clasper tang, the clinician can relatively advance the engagement portion distally beyond an end of the tubular enclosure. Once in this position, the clasper tang can tend to be pulled radially outwardly as the valve anchor expands radially, thereby disengaging the clasper tang from the engagement portion. Thereafter, the engagement portion can be retracted or withdrawn into the tubular enclosure. In some embodiments, the engagement portion engages a window or protrusion of the clasper tang. In some embodiments, the engagement portion is a pin or slot. In some embodiments, each grasper can include a plurality of engagement portions or protrusions.

During use, after the valve anchor has been released from within the proximal sheath and after the valve anchor and the valve frame have been released from the delivery device, the delivery device can be configured to be compactly reassembled and withdrawn into the introducer sheath in order to minimize any damage to the blood vessel through which the delivery device was advanced.

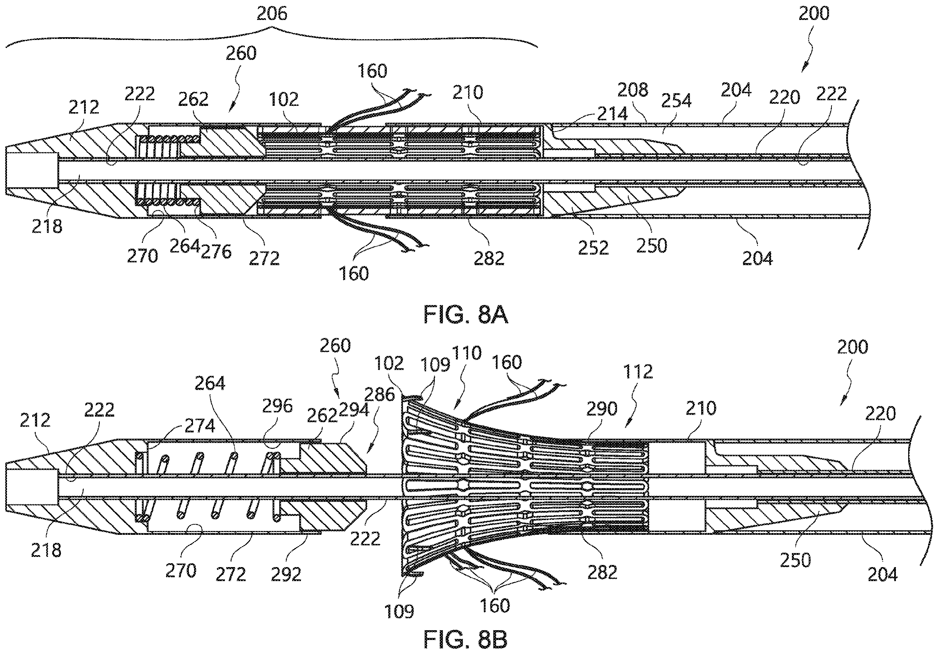

For example, in at least one embodiment, as illustrated in Figure 8A, the proximal enclosure 210 can comprise a proximal section 250 to facilitate realignment (e.g., radial realignment) of the distal end portion 208 of the proximal sheath component 204 with the proximal enclosure 210.

As illustrated in Figure 8A, the proximal section 250 can be coupled to the core member 220. Further, the proximal section 250 can optionally be conical or tapered in a proximal direction and/or have circumferential nodes 252 and/or circumferential cavities 254 that can facilitate realignment of the proximal sheath component 204 relative to the proximal enclosure 210 along a longitudinal axis of the delivery device 200. The tapering of the proximal section 250 can allow the distal end portion 208 of the proximal sheath component 204 to smoothly advance distally over the proximal section 250, and the circumferential nodes 252 can contact an inner surface of the distal end portion 208 of the proximal sheath component 204 as the distal end portion 208 approaches the proximal abutment surface 214.

For example, as illustrated in Figure 8A, the circumferential nodes 252 may gradually taper from the proximal abutment surface 214 in the proximal direction. With such a configuration, as the proximal sheath component 204 slides distally toward the proximal enclosure 210, the circumferential nodes 252 can advantageously guide the distal end portion 208 of the proximal sheath component 104 distally toward the proximal abutment surface 214 of the proximal enclosure 210 so that the outer surface of the proximal sheath component 204 is aligned with an outer surface of the proximal enclosure 210. Thus, the outer surfaces of the proximal enclosure 210 and the proximal sheath component 204 can provide a smooth outer profile for the delivery device 200 that can advantageously reduce the likelihood that the delivery device 200 catches or otherwise damages tissue within a body lumen as the delivery device 200 is moved therewithin.

Optionally, the proximal section 250 can comprise three circumferential nodes 252 and three circumferential cavities 254. The circumferential nodes 252 may extend proximally from the proximal abutment surface 214. The three circumferential cavities 254 can correspond to the number of U-shaped members of the valve anchor that are housed within the proximal sheath component 204 between the proximal sheath component 204 and the proximal section 250 of the proximal enclosure 210.

This advantageous feature of some embodiments can allow the distal enclosure 212 to be properly positioned along the delivery device 200 in order to ensure that distal enclosure 212 does not snag or become caught on any structure during retrieval of the delivery device 200.

As also shown in Figures 4-8B, the proximal and distal enclosures 210, 212 can collectively house the support frame 102. The first and second core members 220, 222 can be actuated to separate the proximal and distal enclosures 210, 212, thereby permitting the support frame 102 to self-expand when in position within the valve anchor 104.

For example, by pushing or pulling the first core member 220, the second core member 222, and/or the proximal sheath component 204 relative to each other along the longitudinal axis of the delivery device 200, a clinician can control longitudinal movement of each of these components to permit the release of the support frame 102 and the valve anchor 104 of the valve prosthesis 100.

Further, in some embodiments, to facilitate delivery of the delivery device 200 to the target location, as shown in Figures 8A-8D, the second core member 222 can include a lumen 218 to permit the delivery device 200 to move along a guidewire, which can extend through the lumen 218 of the second core member 222.

Figures 8A-8D further illustrate positions of the proximal and distal enclosures 210, 212 during the release of the support frame 102. After separating the proximal and distal enclosures 210, 212 from the position illustrated in Figure 8A to the position illustrated in Figure 8B, the first end portion 110 of the support frame 102 can begin to expand from the compressed configuration to an expanded configuration. In some embodiments, the support frame 102 can have one or more anchors 109 (see also Figure 1) at its first end portion 110 that, when engaged with the native valve structure can supplement the outward expansive force (due to self-expansion of the support frame 102) and its resultant frictional engagement, to mitigate downstream migration of the support frame 102 relative to the native valve structure. Thus, by opening the first end portion 110 first (before the second end portion 112, and via relative movement of the proximal and distal enclosures 210, 212), the first end portion 110 can "flower" out to facilitate release of the support frame and/or to engage with the native anatomy, such as the valve structure itself, to secure a longitudinal position of the support frame 102 relative to the native valve structure. Thereafter, the second end portion 112 of the support frame 102 can be controlled and released to become disengaged and released from the proximal enclosure 210.

In some embodiments, the first end portion 110 and the second end portion 112 can open simultaneously, at the same or different rates. For example, in some embodiments, the first end portion 110 and the second end portion 112 can open simultaneously, but with the first end portion 110 opening at a faster rate than the second end portion 112.

Advantageously, the use of the proximal enclosure 210 and the distal enclosure 212 allows for greater control and enhanced operation of the support frame 102. For example, by controlling the position and rate of separation of the proximal enclosure 210 and the distal enclosure 212, the opening of the support frame 102 at both the first end portion 110 and the second end portion 112 can be controlled. Further, by controlling the movement of the distal enclosure 212, the timing and rate of opening of the first end portion 110 can be controlled relative to the timing and rate of opening of the second end portion 112 (which may be controlled by the movement of the proximal enclosure 210).

Additionally and advantageously, by having separate proximal and distal enclosures 210, 212, the delivery device 200 may experience reduced frictional forces and minimize travel of the enclosures 210, 212 relative to the support frame 102.

In particular, in accordance with some embodiments, the distal carrier assembly 206 can comprise a plunger mechanism 260 that can facilitate expansion of the support frame 102. The plunger mechanism 260 can expand from a compressed state (shown in Figure 8A) to an extended state (shown in Figure 8B). The plunger mechanism 260 can be biased by a spring or other device in order to move automatically from the compressed state to the extended state. However, the plunger mechanism 260 can also be manually actuated by the clinician in some embodiments.

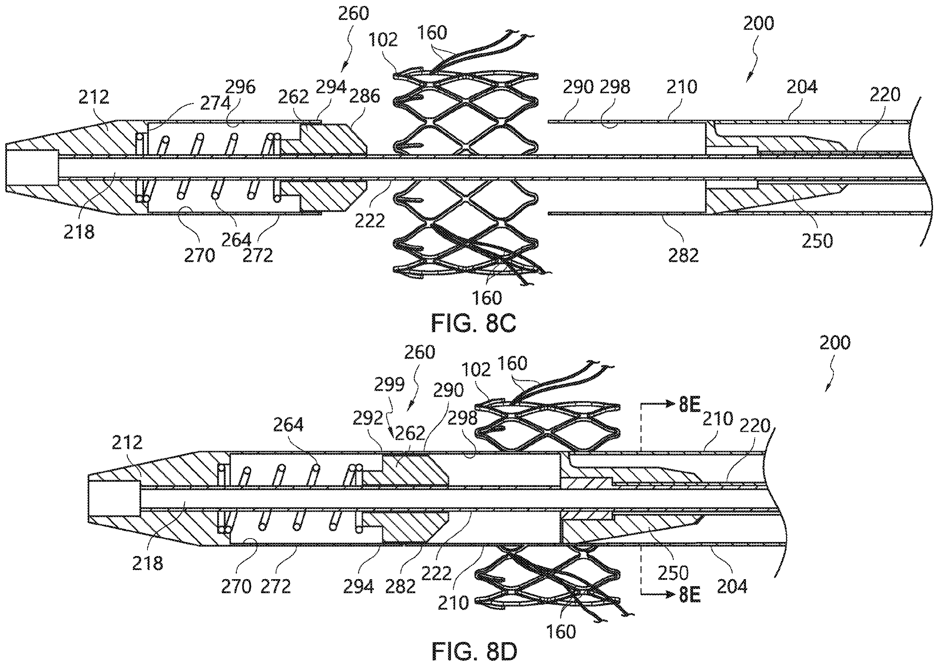

As illustrated, the plunger mechanism 260 can comprise a plunger head 262 and a biasing means 264. Further, the plunger mechanism 260 can be housed within a distal lumen 270 of a tubular portion 272 of the distal enclosure 212 For example, the biasing means 264 may be a spring. The biasing means 264 can be interposed between an interior structure or wall 274 of the distal lumen 270 and a distal surface or structure 276 of the plunger head 262. The plunger head 262 can move proximally within the distal lumen 270 in order to continue to exert a proximally oriented force on the first end portion 110 of the support frame 102 until the support frame 102 exits the distal lumen 270. Thereafter, in accordance with some embodiments, the support frame 102 can self-expand until the second end portion 112 is pulled out of a proximal lumen 280 of a tubular portion 282 of the proximal enclosure 210 as the support frame 102 continues to expand. The expanded state of the support frame 102 is illustrated in Figures 8C and 8D.

Some embodiments can also provide self-aligning features to allow the components of the delivery assembly to be moved from a releasing state (where the components of the valve prosthesis are released from engagement with the delivery assembly) to a nested or stowed state in which the delivery assembly has an aligned outer surfaces or outer surfaces that abut at a seam outer profile that can tend to reduce the likelihood of snagging on the vasculature as the delivery assembly is retrieved from the patient's vasculature. For example, optionally, after the support frame 102 has been expanded and released from the distal carrier assembly 206, the plunger head 262 can advantageously facilitate repositioning and realignment of the proximal and distal enclosures 210, 212 of the distal carrier assembly 206 in preparation for removal of the delivery device 200 from the patient.

For example, the plunger head 262 can comprise a conical or tapered proximal portion 286. The conical proximal portion 286 can be configured to not contact only the first end portion of the support frame 102 during delivery, but can also help center a distal end portion 290 of the tubular portion 282 of the proximal enclosure 210 relative to a longitudinal axis of the delivery device 200 and help align the distal end portion 290 with a proximal end portion 292 of the tubular portion 272 of the distal enclosure 212.

Further, in some embodiments, the plunger head 262 can extend proximally at least partially out of or from the tubular portion 272 of the distal enclosure 212. For example, the plunger head 262 can comprise an outer circumferential surface 294 that can contact not only an inner surface 296 of the tubular portion 272, but can also contact an inner surface 298 of the tubular portion 282 when the tubular portion 282 is distally advanced over the conical proximal portion 286 of the plunger head 262. As such, distal end portion 290 of the proximal enclosure 210 can positioned in an abutting contact position 299 with the proximal end portion 292 of the distal enclosure 212, as shown in Figures 8D. Thus, the plunger mechanism 260 can facilitate not only be expansion of the support frame 102, but can also facilitate the self-alignment and repositioning of the proximal and distal enclosures 210, 212 in order to ensure that neither the proximal enclosure 210 nor the distal enclosure 212 snags or becomes caught on any structure during retrieval of the delivery device 200.

In accordance with at least one embodiment disclosed herein, the delivery device can optionally comprise a plunger mechanism having features that can expand radially beyond the outer diameter of the tubular portion 272 of the distal enclosure 212. Further, the plunger mechanism can also be configured in a manner to engage a proximal end of the tubular portion 272 of the distal enclosure 212. Thus, instead of the plunger mechanism 260 illustrated in the embodiment shown in Figures 8A-8D, the delivery device can optionally comprise a nose cone protector that can facilitate expansion of the support frame and advantageously provide a contact surface or ramp that tapers from a first diameter to a second, larger diameter in a distal direction to avoid or reduce any catching or engagement of the delivery device 200 when being proximally withdrawn into the delivery catheter. Further, the nose cone protector can increase the column strength of the nose cone or the distal carrier assembly 206. In this manner, the nose cone protector can help avoid breakage or dislocation of the nose cone or distal carrier assembly 206 when the clinician is exerting a proximal retraction force on the delivery assembly 200 to retract the delivery device 200 into the delivery catheter.

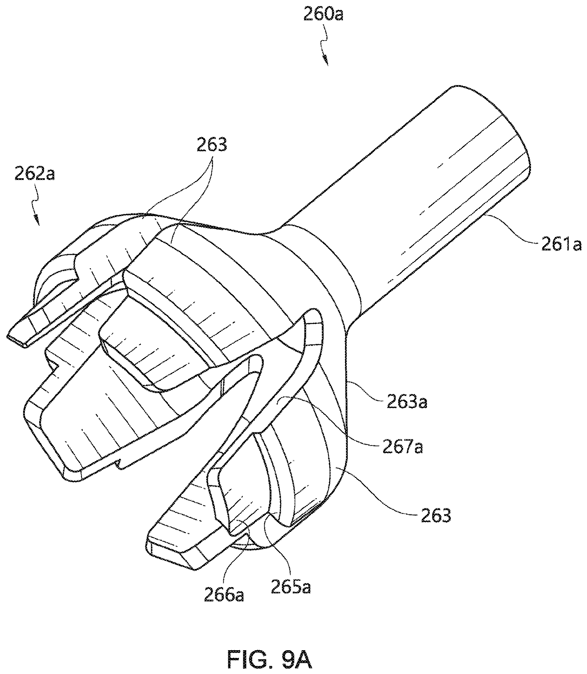

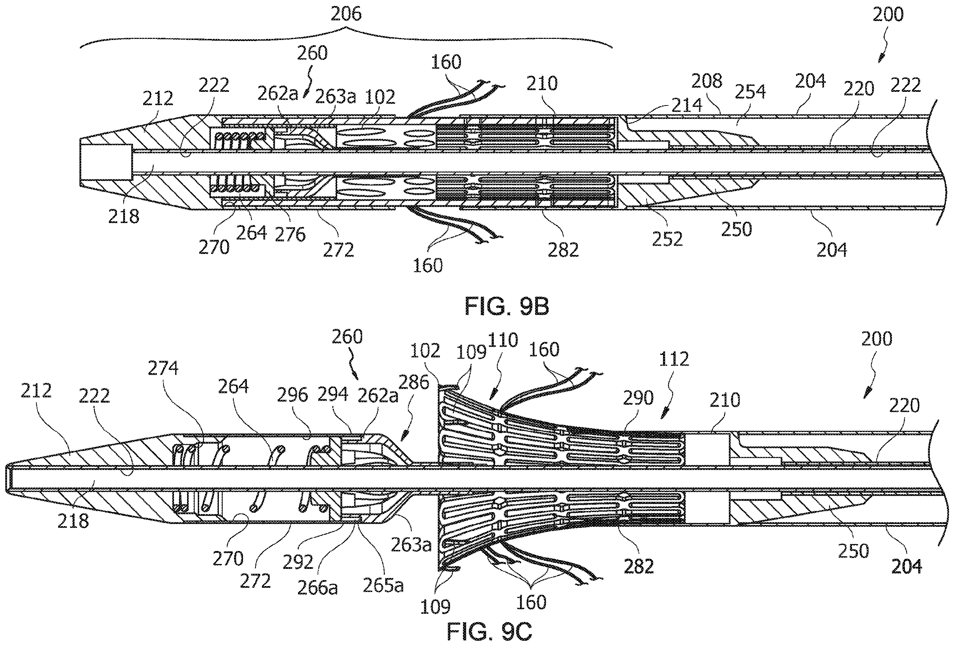

For example, Figures 9A-9F illustrate a nose cone protector 260a that can facilitate expansion of the support frame 102, similar to the plunger mechanism 260 illustrated in Figures 8A-8D. The nose cone protector 260a can move longitudinally from a compressed state (shown in Figure 9B) to an extended state (shown in Figure 9C). The nose cone protector 260a can be biased by a spring or other device in order to move automatically from the compressed state to the extended state. However, the nose cone protector 260a can also be manually actuated by the clinician, in some embodiments.

As shown in Figure 9A, the nose cone protector 260a can comprise a plunger head 262a having petals 263 that each extend radially from the nose cone protector 260a and comprise ramped surfaces 263a. The ramped surfaces 263a can be configured to engage a distal end of the support frame 102 and urge the support frame 102 out of the distal enclosure 212, similar to the plunger mechanism 260 discussed above, which can facilitate expansion of the support frame 102. In some embodiments, the nose cone protector 260a can comprise polyether ether ketone (PEEK) or other thermoplastics.

In at least one embodiment, the petals 263 can be configured to deflect radially inwardly while the nose cone protector 260a is positioned within the lumen of the distal enclosure 212, but to expand radially outwardly when at least a portion of the petals 263 exits the distal enclosure 212, as discussed below. Thus, the petals 263 can be resiliently biased toward a radially expanded position.

Additionally, the nose cone protector 260a can comprise a central lumen 261a through which the core member 220 of the delivery device 200 can extend. In this regard, the nose cone protector 260a can be permitted to slide along the core member of the delivery device.

Further, the nose cone protector 260a can be used with the distal carrier assembly 206 to ensure that neither the proximal enclosure 210 nor the distal enclosure 212 snags or becomes caught on the delivery catheter or any anatomical structure during retrieval of the delivery device 200. In at least one embodiment, the ramped surfaces 263a of the nose cone protector 260a can also facilitate self-alignment and repositioning of the proximal and distal enclosures 210, 212 similar to that provided by the plunger mechanism 260.

In use, because the nose cone protector 260a provides a smooth, ramped surface that the delivery device 200 can contact against any protuberances of the anatomical structure or delivery catheter, the nose cone protector 260a can thereby advantageously minimize the pulling or retraction force required by the clinician during retraction of the delivery device 200 into the delivery catheter. Of course, another beneficial effect of the nose cone protector 260a is to prevent or reduce any trauma to the blood vessel.

As illustrated, the nose cone protector 260a can be biased to spring radially outward to engage against the proximal end of the distal enclosure 212 in its extended state. In the extended state, the petals 263 can spread apart at separations 267a. Further, the petals 263 can each comprise an engagement tooth 266a that has an outer surface that is radially offset from an outer surface of its respective petal 263, thereby permitting the petal 263 to expand radially beyond an inner diameter of the distal enclosure 212. The teeth 266a can have a contact surface that is radially offset from an outer surface of the petals 263. Further, when the teeth 266 engage the proximal end of the distal enclosure 212, the outer surfaces of the petals 263 can extend radially beyond the outer surface of the distal enclosure 212.

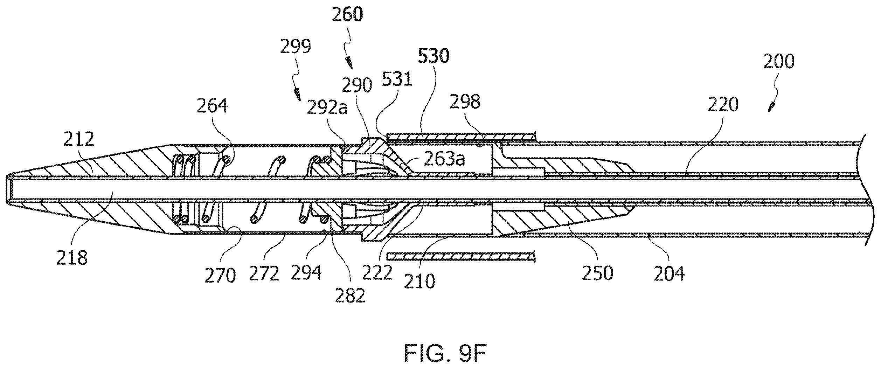

In some embodiments, a distal engagement surface 265a of each petal 263 can abut the proximal end of the distal enclosure 212. As illustrated, the ramped surface 263a of the nose cone protector 260a can align and facilitate repositioning of the proximal and distal enclosures 210, 212 relative to each other in anticipation of proximally withdrawing the delivery device 200 into the delivery catheter 530. Additionally, the ramped surface 263a of the nose cone protector 260a can provide an angled surface that will not tend to snag or engage a distal end 531 of the delivery catheter 530 (see Figure 9F).

As illustrated in Figure 9B, the nose cone protector 260a can be housed within a distal lumen 270 of a tubular portion 272 of the distal enclosure 212. The nose cone protector 260a can be coupled to and urged proximally out of the distal enclosure 212 via a biasing means 264. As described with respect to the plunger mechanism 260, the biasing means 264 may be a spring. The petals 263 can be radially compressed to be housed within the distal lumen 270. The biasing means 264 can be interposed between an interior structure or wall 274 of the distal lumen 270 and a distal surface or structure 276 of the plunger head 262a. The plunger head 262a can move proximally within the distal lumen 270 in order to continue to exert a proximally oriented force on the first end portion 110 of the support frame 102 until the support frame 102 exits the distal lumen 270. Thereafter, in accordance with some embodiments, the support frame 102 can self-expand until the second end portion 112 is pulled out of a proximal lumen 280 of a tubular portion 282 of the proximal enclosure 210 as the support frame 102 continues to expand. The expanded state of the support frame 102 is illustrated in Figures 9C-9F.

As illustrated in Figures 9C and 9D, as the nose cone protector 260a advances (Figure 9C shows the nose cone protector 260a in a semi-expanded state) and the distal engagement surface 265a extends past the proximal end of the distal lumen 270 (Figure 9D shows the nose cone protector 260a in a fully expanded state), the petals 263 can radially expand to engage against the distal lumen 270. Optionally, the proximal end of the nose cone protector 260a can be axially retained by collar engaged against the central lumen 261a to maintain the distal engagement surface 265a in axial abutment with the proximal end of the distal enclosure 212.

In addition to contacting the first end portion of the support frame 102 during delivery, as shown in Figure 9E, the ramped surface 263a can also help center a distal end portion 290 of the tubular portion 282 of the proximal enclosure 210 relative to a longitudinal axis of the delivery device 200 and help align the distal end portion 290 with a proximal end portion 292 of the tubular portion 272 of the distal enclosure 212.

Further, as shown in Figure 9F, in some embodiments, the ramped surface 263a allows the proximal sheath component 204 to be concentrically aligned with the distal enclosure 212, minimizing the retraction force required to retract the proximal and distal enclosure 210, 212 into the proximal sheath component. In some embodiments, the ramped surface 263a can have a ramp angle of approximately 45 degrees, or may range from between about 10 degrees to about 80 degrees, from between about 20 degrees to about 70 degrees, from between about 30 degrees to about 60 degrees, or from between about 40 degrees to about 50 degrees.

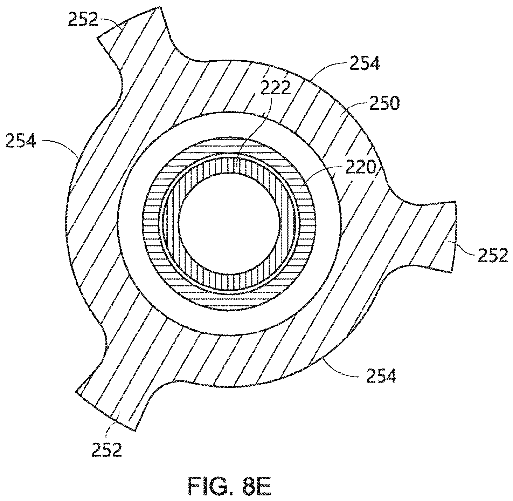

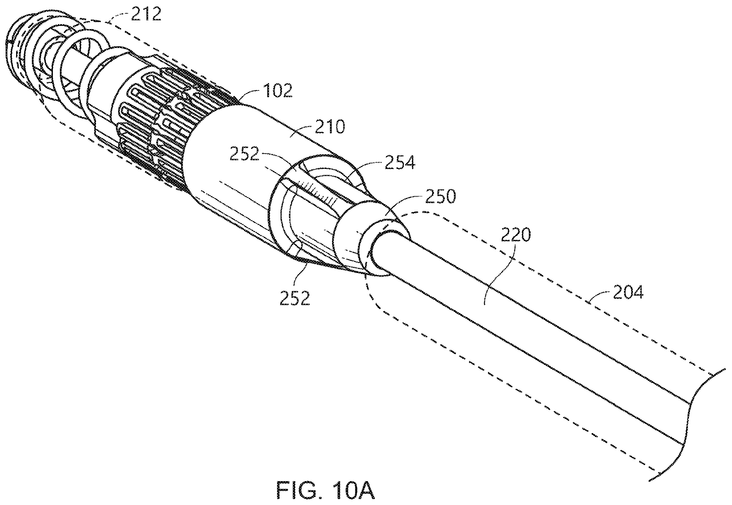

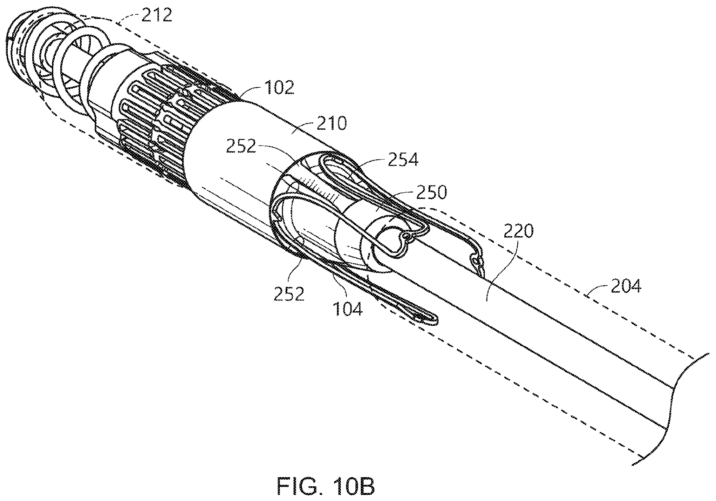

Referring to Figures 8E, 10A, and 10B, the proximal section 250 of the proximal enclosure 210 can include features that facilitate engagement and alignment of the proximal section 250 with the valve anchor. As illustrated in Figures 8E and 10A, the proximal section 250 of the proximal enclosure 210 has circumferential nodes 252 and circumferential cavities 254. Each of the circumferential cavities 254 may be defined by a space between two of the circumferential nodes 252. As illustrated in Figure 10B, respective U-shaped members of the valve anchor 104 may be received in the circumferential cavities 254, between the circumferential nodes 252.

For example, when the valve anchor 104 is loaded and the proximal sheath component 204 covers the valve anchor 104, each U-shaped member of the valve anchor 104 may be positioned in a corresponding circumferential cavity 254, between the proximal section 250 and the proximal sheath component 204. The cross-sectional of the proximal section 250 of the proximal enclosure 210 illustrated in Figure 8E (taken along lines 8E-8E of Figure 8D) also shows that each of the circumferential cavities 254 may be defined by a space between two of the circumferential nodes 252, such that the valve anchor 104 may be received or positioned in the circumferential cavities 254.

The circumferential nodes 252 and the circumferential cavities 254 may facilitate realignment of the proximal sheath component 204 relative to the proximal enclosure 210 along a longitudinal axis of the delivery device 200. The proximal section 250 may be conical or tapered in a proximal direction, thereby facilitating alignment of the proximal sheath component 204 relative to the proximal enclosure 210 along a longitudinal axis of the delivery device 200. In the examples illustrated in Figures 8E, 10A, and 10B, the proximal section 250 has three circumferential nodes 252 and three circumferential cavities 254. However, in another example, a different number of the circumferential nodes 252 and/or a different number of the circumferential cavities 254 may be implemented.

As discussed above, the delivery device 200 provide several benefits, such as a compact passing profile that allows the delivery device 200 to move through the vasculature with facility, reliable control and positioning of the valve anchor while for within the native valve annulus and sinuses, predictable relative positioning of the support frame 102 and the valve anchor 104 via the link mechanism 160, and snag-free retrieval of the delivery device 200.

Some of these features and benefits will be illustrated with respect to the delivery stages shown in Figures 11A-11F. These figures illustrate the use of the delivery device 200 in a human heart 300. The heart 300 can comprise an aorta 301 having an aortic arch 302 and an aortic valve 304. The aorta valve 304 can comprise a plurality of native valve leaflets 306 and separate the aorta 301 from the left ventricle 310. In accordance with some embodiments, the delivery device 200 can be advanced retrograde through the aorta 301 until reaching and being positioned through the native valve leaflets 306 of the aortic valve 304.

With reference to Figure 11A, during delivery of the valve prosthesis 100 to the native valve site, the valve anchor 104 and the support frame 102 can be positioned in tandem, as an axially displaced unit (with or without partial or full overlapping between the anchor and the frame) along the longitudinal axis of the delivery device 200. This configuration, as opposed to a concentric arrangement, allows a more radially compact configuration of the components of the valve prosthesis 100, creating a much smaller cross-section and facilitating a catheter-based delivery. This can improve the flexibility of the delivery device 200, enabling the delivery device 200 to be advanced over a guidewire through the tortuous geometries of the circulatory system, and in particular, the aortic arch 302. Indeed, even with guidewire-directed delivery devices, the aortic arch 302 represents a difficult obstacle due to its sudden and high-degree of curvature. Often, this is a limiting constraint for some surgeries or delivery devices. However, in accordance with the various benefits and advantages of some embodiments disclosed herein, as illustrated in Figure 11A, the delivery device 200 can be advanced over the aortic arch 302 to a target location in the region of the aortic valve 304.

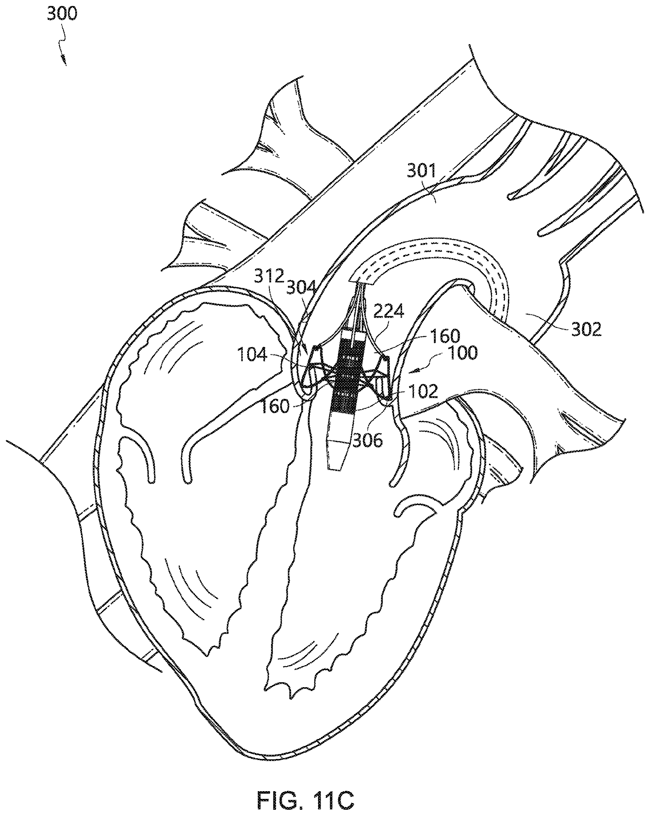

Further, as illustrated in Figure 11B, the proximal sheath component 204 can be proximally retracted relative to the prosthesis 100, thereby permitting the valve anchor 104 to expand. Advantageously, the valve anchor 104 can be expanded adjacent to the aortic valve 304 to maintain a minimal profile of the delivery device 200 to permit clinicians to locate the desired landing site without blocking blood flow (preventing the need for rapid pacing), prevent snagging or catching on patient anatomy or calcification, and permit manipulation and rotation of the delivery device 200 to allow the valve anchor 104 to be located and positioned in the aortic sinuses. The graspers 224 of the delivery device 200 can continue to be engaged with the engagement areas 150 of the valve anchor 104 after the valve anchor 104 has initially expanded. This can allow the clinician to manipulate the position of the valve anchor 104 relative to the aortic valve 304 and its sinus structure 312. As discussed above, the base portions of the valve anchor 104 can be positioned within the sinuses of the aortic valve 304 in order to seat the valve anchor 104 in a proper position within the aortic valve 304, as illustrated in Figure 11C. In the proper position, the native valve leaflets 306 will be positioned in a space between the support frame 102 and the valve anchor 104.

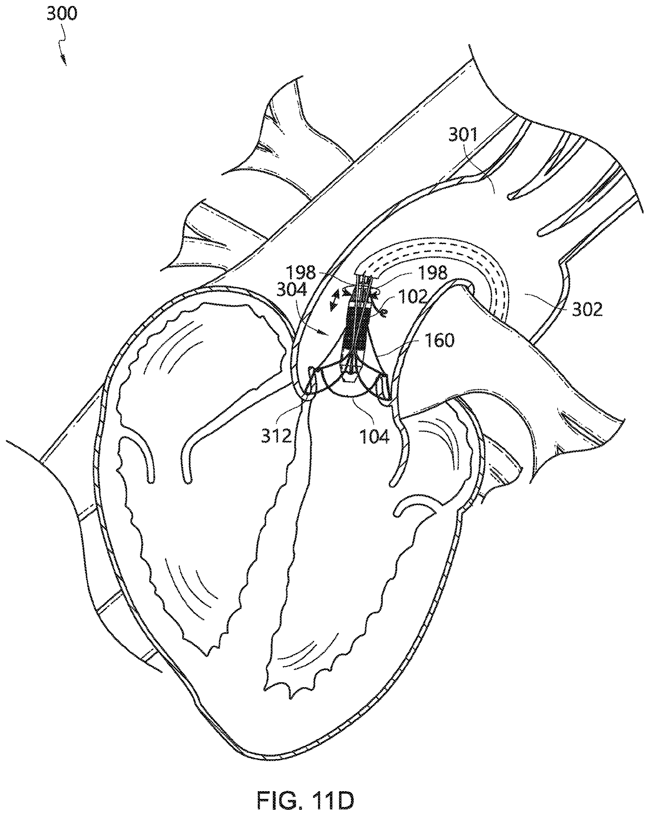

Further, as illustrated in Figures 11B and 11C, the link mechanism 160 is coupled to both the valve anchor 104 and the support frame 102 in a released position which allows the link mechanism 160 to slide freely along the valve anchor 104. Advantageously, in some embodiments, this flexible connection can allow for the valve anchor 104 to rotate, permitting the valve anchor 104 to self-align with the native cusps of the heart for optimal locating and landing. Referring now to Figure 11D, the connection between the link mechanism 160 and the tension members 198 can be released by proximally retracting the support frame 102 relative to the valve anchor 104 while maintaining the valve anchor 104 seated within the sinus structure 312 of the aortic valve 304. This proximal movement of the support frame 102 relative to the valve anchor 104 can cause the link mechanism 160 to become taut, thereby stretching and causing the tension members 198 to release the link mechanism 160. Further, the proximal movement of the support frame 102 relative to the valve anchor 104 causes the link mechanism 160 to become engaged in the engagement areas 150 of the valve anchor 104.

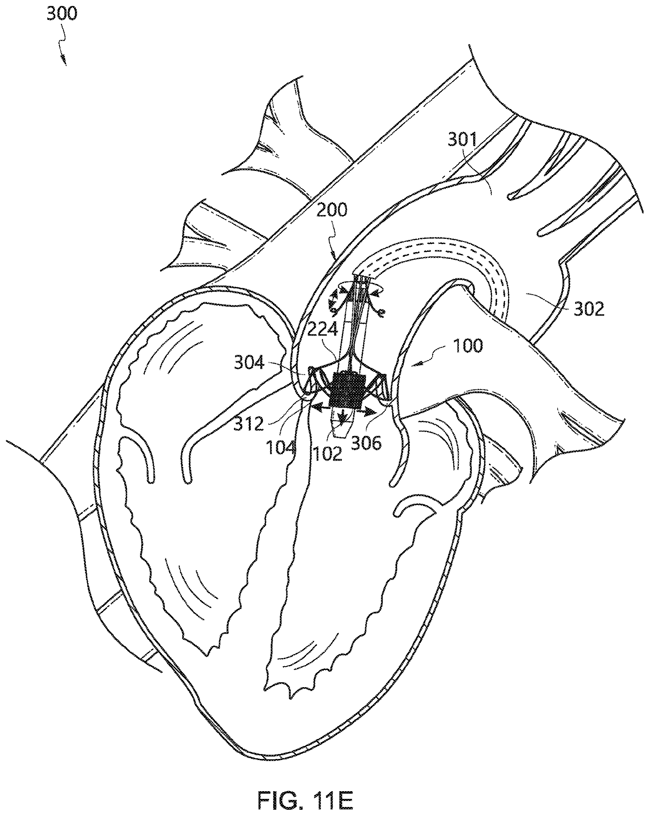

After releasing the link mechanism 160 from the tension members 198 and engaging the link mechanism 160 with the respective engagement areas 150 of the valve anchor 104, the link mechanism 160 is in the retained position. Figure 11E illustrates that in the retained position, the support frame 102 can be distally advanced relative to the valve anchor 104 until the support frame 102 is positioned within the aortic valve 304. In accordance with some embodiments, the link mechanism 160 will become taut when the support frame 102 is in a desirable position relative to the valve anchor 104.

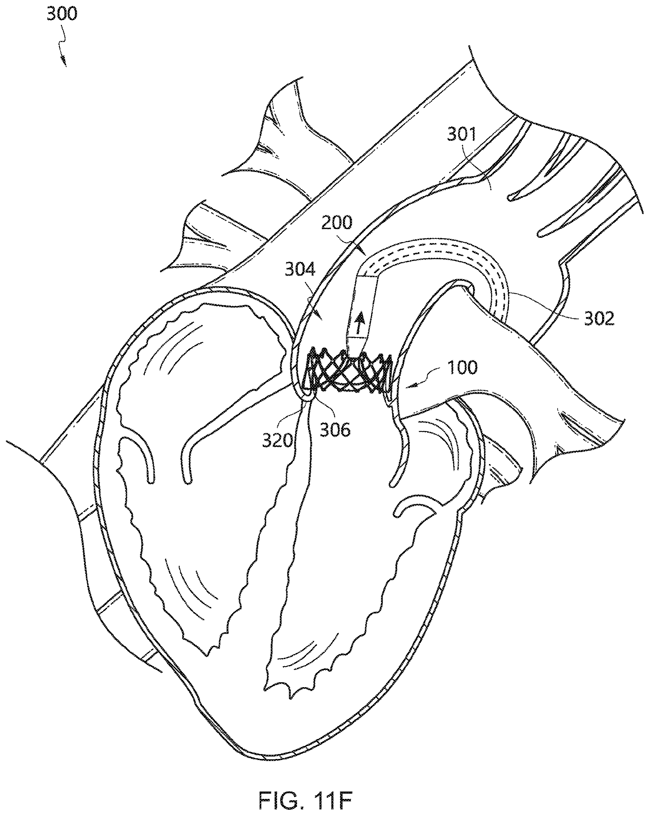

For example, the length of the link mechanism 160 can ensure that the valve anchor 104 can advance distally only to a certain predetermined position within the support frame 102 when the link mechanism 160 is engaged with the engagement areas 150 of the valve anchor 104, as discussed herein. Once the valve anchor 104 is in the desired position, the support frame 102 can be released from the distal carrier assembly and expanded into apposition with the native valve leaflets 306 and the internal aspects of the valve anchor 104, thus sandwiching the native valve leaflets 306 between the support frame 102 and the valve anchor 104, as shown in Figure 11F. Advantageously, by sandwiching the native valve leaflets 306 between the support frame 102 and the valve anchor 104, the valve prosthesis 100 can have reduced reliance on radial force retention. Further, by sandwiching the native valve leaflets 306 between the support frame 102 and the valve anchor 104 the likelihood of the native valve leaflets 306 blocking the opening of the coronary artery is reduced, which may be beneficial for patients with low coronary ostia distance, and in patients with existing valve prosthesis, whom may need a new valve prosthesis inside the existing valve prosthesis (valve-in-valve application). The support frame 102 and the valve anchor 104 can thus expand into contact with the aortic valve 304, exerting a chronic outward force against the native valve leaflets 306 and aortic valve annulus 320.

In addition, as discussed herein, after expanding and releasing the support frame 102, the hooks of the graspers 224 can be disengaged from the valve anchor 104 to release the valve anchor 104 from the delivery device 200. Thereafter, the proximal sheath component and the components of the distal carrier assembly can be packed together, as discussed herein, to permit the delivery device 200 to be withdrawn from the aorta 301 and retrieved from the patient

Thereafter, the prosthetic valve leaflets of the prosthesis 100 can begin to function in the manner desired and provide the same operation as a native valve.

In some embodiments, a delivery device 200' can be used to deliver and position the support frame 102 and the valve anchor 104 utilizing an antegrade, apical, or transapical approach. A transapical approach will be illustrated with respect to the delivery stages shown in Figures 12A-12F. Similar to Figures 11A-11F, these figures illustrate the use of the delivery device 200' in a human heart 300, and more specifically, an aortic valve 304 of an aorta 301. In accordance with some embodiments, the delivery device 200' can be advanced antegrade through an apex 311 until reaching and being positioned through the native valve leaflets 306 of the aortic valve 304. Compared to the transfemoral retrograde approach illustrated in Figures 11A-11F, the valve anchor 104 can be positioned to receive the control member or the grasper from an apical approach and to be seated in the sinus structure of the aortic valve 304 from an apical approach.

With reference to Figure 12A, during delivery of the valve prosthesis 100 to the native valve site, after placement of the delivery catheter 530, the delivery device 200' can be advanced toward the target area. The valve anchor 104 and the support frame 102 can be positioned in series (in tandem) along the longitudinal axis of the delivery device 200'. This can improve the flexibility of the delivery device 200', enabling the delivery device 200' to be advanced over a guidewire through the tortuous geometries of the circulatory system, and in particular, the apex 311. In accordance with the various benefits and advantages of some embodiments disclosed herein, as illustrated in Figure 12A, the delivery device 200' can be advanced through the apex 311 to a target location in the region of the aortic valve 304.

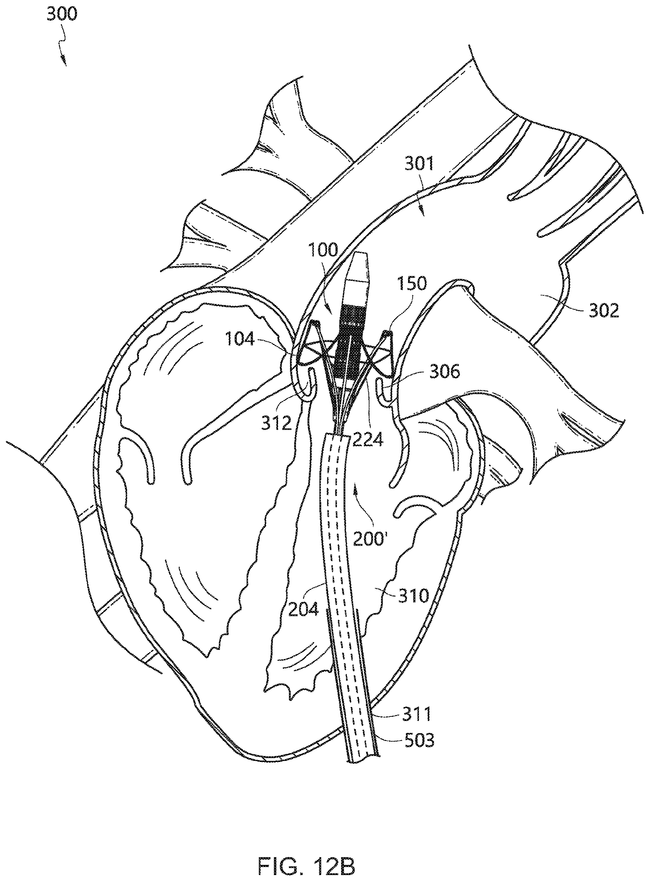

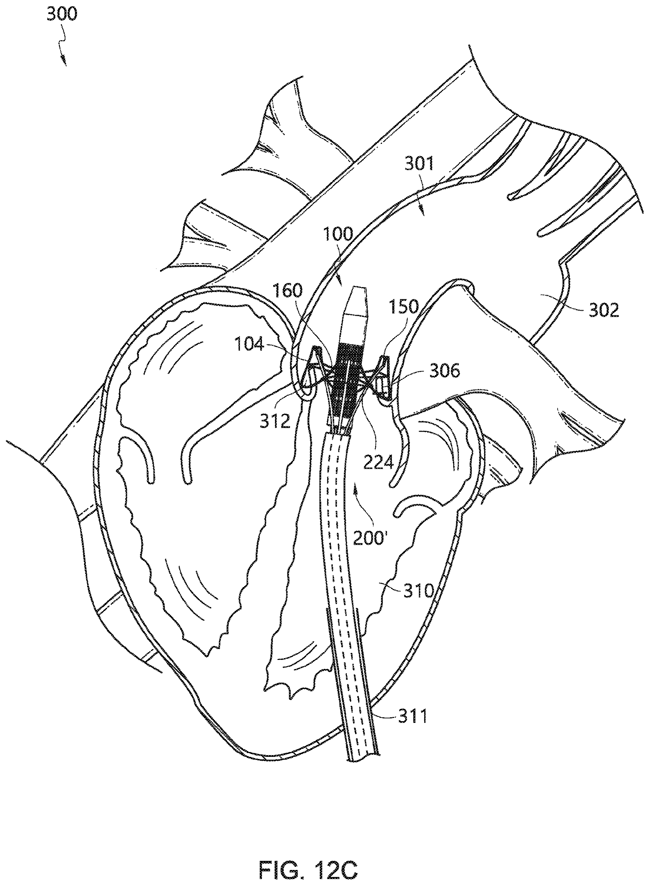

Further, as illustrated in Figure 12B, the proximal sheath component 204 can be proximally retracted relative to the prosthesis 100, thereby permitting the valve anchor 104 to expand. Advantageously, the valve anchor 104 can be expanded adjacent to the aortic valve 304 to maintain a minimal profile of the delivery device 200' to permit clinicians to locate the desired landing site without blocking blood flow (preventing the need for rapid pacing), prevent snagging or catching on patient anatomy or calcification, and permit manipulation and rotation of the delivery device 200' to allow the valve anchor 104 to be located and positioned in the aortic sinuses. The graspers 224 of the delivery device 200' can continue to be engaged with the engagement areas 150 of the valve anchor 104 after the valve anchor 104 has initially expanded. As shown, the graspers 224 of the delivery device 200' (used in the transapical approach) are coupled to the engagement areas 150 of the valve anchor 104 from a direction reverse that of the delivery device 200 (used in the transfemoral approach, shown in Figures 11A-11E); in the transapical approach, the graspers 224 can extend through or within an inner lumen of the valve anchor 104 whereas the in the transfemoral approach, they do not. However, the interconnection of the graspers 224 and the valve anchor 104 can be reversed in either approach provided that the valve anchor 104 can be properly seated within the native valve sinus structure 312. The graspers 224 can allow the clinician to manipulate the position of the valve anchor 104 relative to the aortic valve 304 and its sinus structure 312. As discussed above, the base portions of the valve anchor 104 can be positioned within the sinuses of the aortic valve 304 in order to seat the valve anchor 104 in a proper position within the aortic valve 304, as illustrated in Figure 12C. In the proper position, the native valve leaflets 306 will be positioned in a space between the support frame 102 and the valve anchor 104.

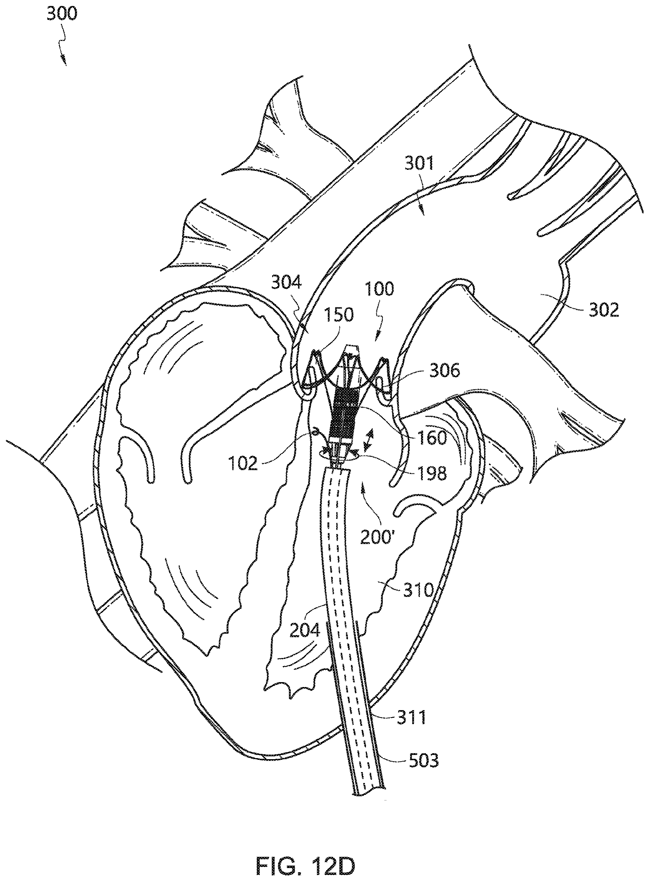

Further, as illustrated in Figures 12B and 12C, the link mechanism 160 is coupled to both the valve anchor 104 and the support frame 102 in a released position which allows the link mechanism 160 to slide freely along the valve anchor 104. Referring now to Figure 12D, the connection between the link mechanism 160 and the tension members 198 can be released by proximally retracting the support frame 102 relative to the valve anchor 104 while maintaining the valve anchor 104 seated within the sinus structure 312 of the aortic valve 304. This proximal movement of the support frame 102 relative to the valve anchor 104 can cause the link mechanism 160 to become taut, thereby stretching and causing the tension members 198 to release the link mechanism 160. Further, the proximal movement of the support frame 102 relative to the valve anchor 104 causes the link mechanism 160 to become engaged in the engagement areas 150 of the valve anchor 104.

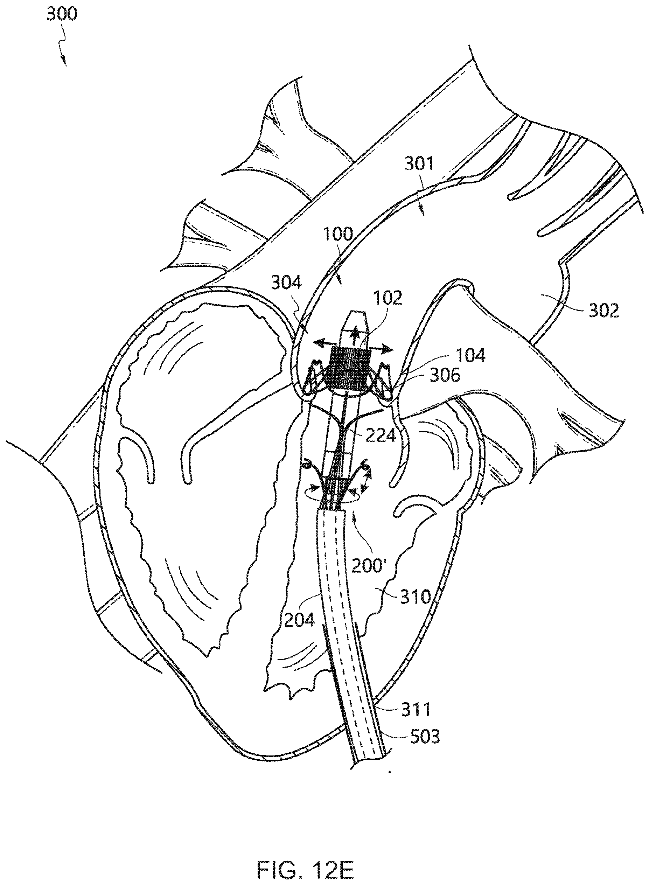

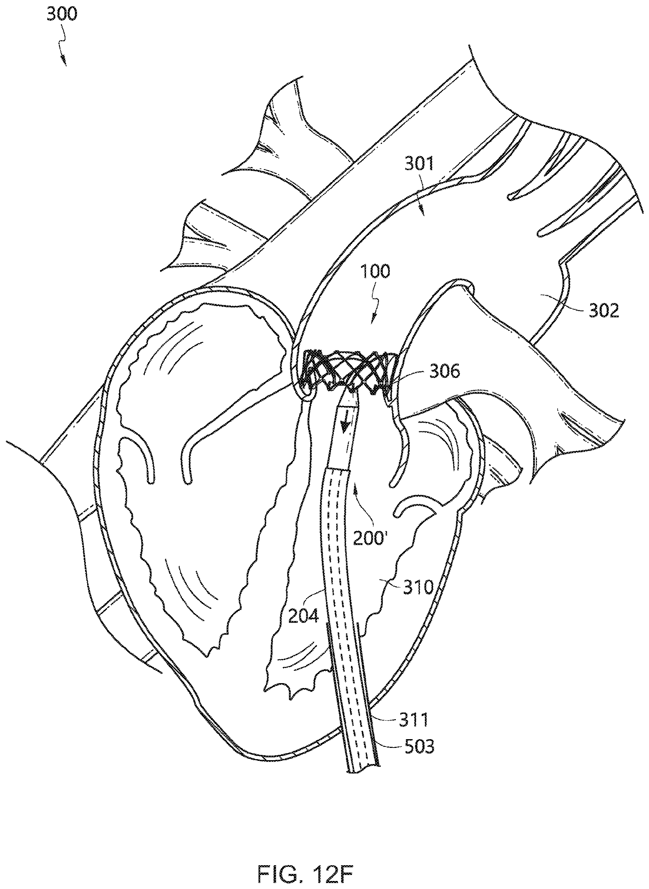

After releasing the link mechanism 160 from the tension members 198 and engaging the link mechanism 160 with the respective engagement areas 150 of the valve anchor 104, the link mechanism 160 is in the retained position. Figure 12E illustrates that in the retained position, the support frame 102 can be distally advanced relative to the valve anchor 104 until the support frame 102 is positioned within the aortic valve 304. In accordance with some embodiments, the link mechanism 160 will become taut when the support frame 102 is in a desirable position relative to the valve anchor 104. Once the valve anchor 104 is in the desired position, the support frame 102 can be released from the distal carrier assembly and expanded into apposition with the native valve leaflets 306 and the internal aspects of the valve anchor 104, thus sandwiching the native valve leaflets 306 between the support frame 102 and the valve anchor 104, as shown in Figure 12F. Advantageously, by sandwiching the native valve leaflets 306 between the support frame 102 and the valve anchor 104, the valve prosthesis 100 can have reduced reliance on radial force retention. Further, by sandwiching the native valve leaflets 306 between the support frame 102 and the valve anchor 104 the likelihood of the native valve leaflets 306 blocking the opening of the coronary artery is reduced, which may be beneficial for patients with low coronary ostia distance, and in patients with existing valve prosthesis, whom may need a new valve prosthesis inside the existing valve prosthesis (valve-in-valve application). The support frame 102 and the valve anchor 104 can thus expand into contact with the aortic valve 304, exerting a chronic outward force against the native valve leaflets 306 and aortic valve annulus 320.

In addition, as discussed herein, after expanding and releasing the support frame 102, the hooks of the graspers 224 can be disengaged from the valve anchor 104 to release the valve anchor 104 from the delivery device 200'. Thereafter, the proximal sheath component and the components of the distal carrier assembly can be packed together, as discussed herein, to permit the delivery device 200' to be withdrawn from the apex 311 and retrieved from the patient. The incision through the apex 311 can then be closed using a technique known in the art.

Thereafter, the prosthetic valve leaflets of the prosthesis 100 can begin to function in the manner desired and provide the same operation as a native valve.

According to some embodiments, the present disclosure also provides a handle actuator that can be used to control the operation of the presently disclosed delivery device and allow a clinician to reliably and accurately control the delivery of the valve prosthesis. Figures 13A-13H illustrate features and operation of the handle actuator, according to some embodiments, for delivering a valve prosthesis using a handle actuator 500. As noted above, the delivery can be performed using a transcatheter approach.

Figure 13A illustrates the handle actuator 500. The handle actuator 500 can control one or more functions of the delivery device (e.g., the delivery device 200 discussed herein) for delivering of a valve prosthesis (e.g., the heart valve prosthesis 100 discussed herein). As shown in Figure 13A, the handle actuator 500 can comprise a plurality of actuators or movable elements, such as knobs or buttons. The movable elements can permit a clinician to control one or more operations of the delivery device 200. The handle actuator 500 can comprise a control handle 510 having a longitudinal axis 512. The handle actuator 500 may be also referred to as a control unit. In some embodiments, the handle actuator 500 may be coupled to the second core member 222 (shown, e.g., in Figures 8A-8D). The control handle 510 can support the actuators and be held by the clinician during the procedure

Figure 13A illustrates that the handle actuator 500 can comprise a steering knob or first movable element 520, an anchor release knob or second movable element 522, a nosecone/valve release knob or third movable element 524, and a nose cone toggle lock or fourth movable element 526. The first movable element 520, the second movable element 522, the third movable element 524, and the fourth movable element 526 may be also referred to as the first control element 520, the second control element 522, the third control element 524, and the fourth control element 526.

Optionally, in some embodiments, one or more of the movable elements, such as the second movable element 522 and/or the third movable element 524, can include a button or slider safety switch 529 that prevent the unintentional rotation of the moveable elements. The safety switch 529 can be configured as resilient button or slider mechanisms that can be actuated to release a lock that provides resistance to rotational or translational movement of the respective movable element. In some embodiments, the movable elements can have a raised feature that provides a visual indication of rotation and facilitates tactile engagement and actuation by the clinician.

In some embodiments, the handle actuator 500 can further include a deair line 514. The deair line 514 can be in fluid communication with the volume within the proximal sheath component 204. Optionally, the deair line 514 can be used to remove or draw air or other gases from the proximal sheath component 204 to remove any air or gas bubbles.

The handle actuator 500 can be coupled to the delivery device 200. At least a portion of the delivery device 200, such as a catheter portion or a distal tip portion 501 thereof, can be steerable in order to facilitate distal advancement and/or coaxial alignment of the delivery device 200 within the vasculature and native anatomy. In this manner, the delivery device 200 can be steered and navigated to the target location within the native valve structure. Thereafter, the process steps performed using the handle actuator 500 can generally mirror the procedure discussed above with regard to the delivery of the valve prosthesis 100 using the delivery device 200.

In a first step, the first movable element 520 can be controlled (e.g., by rotating the first movable element 520) to steer a distal tip portion 501 of the delivery device 200 in order to maneuver the delivery device 200 through the vasculature. For example, the first movable element 520 may include a knob that can be rotated about the longitudinal axis 512 in order to cause the distal tip portion 501 to bend in order to maneuver the delivery device 200.

In some embodiments, the distal tip portion 501 may include the proximal sheath component 204 that can be moved by controlling the first movable element 520. Thus, in some embodiments, the first movable element 520 may be controlled to bend the distal tip portion 501 by moving the proximal sheath component 204. In some embodiments, by moving the proximal sheath component 204, the first movable element 520 may bend the second core member 222.

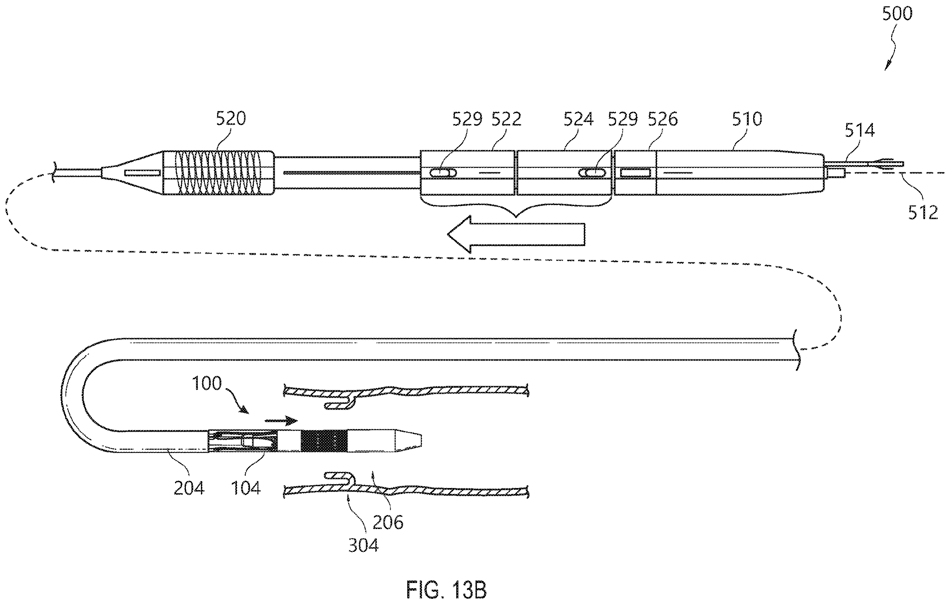

In such embodiments, in some examples, the first movable element 520 may be controlled to move the proximal sheath component 204, thereby axially deflecting the distal carrier assembly 206 and the second core member 222. The delivery device 200 can be steered using the first movable element 520 until reaching the target location within the aortic valve 304, as shown in Figure 13B. In some embodiments, the delivery device 200 can be advanced along a guidewire toward the target location. Further, whether a guidewire is used or not, the distal tip portion 501 can be steered to navigate to the delivery device 200 to the target location. The distal tip portion 501 can be bent by rotating the first movable element 520 in either direction. The first movable element 520 can be operatively coupled to the delivery device 200 via a steering control cable.

Optionally, the delivery can be performed using a transcatheter approach. For example, the delivery device 200 can be maneuvered through an introducer sheath (not shown) toward the target area. Further, optionally, in at least one embodiment, the delivery device 200 can be advanced over a guidewire toward the target area.

As also shown in Figure 13B, the second movable element 522 and the third movable element 524 can be moved together (e.g., by sliding or translating the second movable element 522 and the third movable element 524) to cause the distal carrier assembly 206 of the delivery device 200 to be distally advanced relative to the proximal sheath component 204. For example, the second movable element 522 and the third movable element 524 may include knobs that can be translated in a first direction (e.g., distally) to cause the delivery device 200 to be distally advanced relative to the proximal sheath component 204. The proximal sheath component 204 can be maintained at a generally constant position while the valve prosthesis 100 is advanced relative to the proximal sheath component 204 (i.e., the proximal sheath component 204 is retracted proximally relative to the valve prosthesis 100). This proximal relative motion of the proximal sheath component 204 can be continued until the valve anchor 104 of the valve prosthesis 100 is exposed.

The second movable element 522 and the third movable element 524 can be operatively coupled to a first control cable to retract the proximal sheath component 204 relative to the distal carrier assembly. In some embodiments, the proximal sheath component 204 can be operatively coupled to the second movable element 522 and the third movable element 524 via a control wire.

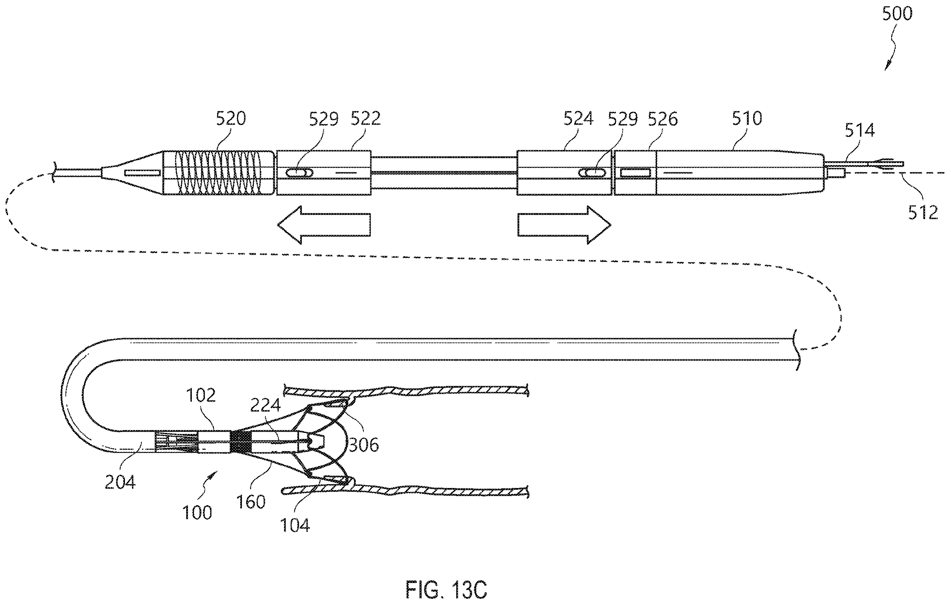

Figure 13C illustrates that linearly moving the second movable element 522 and the third movable element 524 away from one another can retract the proximal sheath component 204 sufficiently to permit the U-shaped members of the valve anchor 104 to expand within the aortic valve 304. In this position, as discussed above, the graspers 224 can be engaged with the peak portions of the valve anchor 104 to permit the clinician to manipulate the position of the valve anchor 104 relative to the native valve leaflets 306 and aortic sinuses 312. The clinician can continue the positional adjustments of the valve anchor 104 until achieving a desired positioning of the valve anchor 104.

In some embodiments, the continued advancement of the third movable element 524 in the distal direction can cause the valve anchor 104 to continue to move distally relative to the support frame 102, thereby causing the link mechanism 160 to slide along the U-shaped members of the valve anchor 104. In some embodiments, this motion can be driven via the graspers 224. As illustrated, the support frame 102, the link mechanism 160, and the valve anchor 104 are in the released position, which allows the link mechanism 160 to slide freely. Eventually, the link mechanism 160 can slide down to be engaged in the engagement areas of the valve anchor 104. The link mechanism will then be in the retained position. In some embodiments, the graspers 224 can be operatively coupled to the third movable element 524 via a control wire.

In some embodiments, the third movable element 524 can be translated in a proximal direction while maintaining the support frame 102 in the desired position, in order to cause the valve anchor 104 to be advanced distally relative to the support frame 102. As discussed herein, the valve anchor 104 can be advanced distally or otherwise moved relative to the support frame 102 until the support frame 102 is in position within the valve anchor 104. In some embodiments, the desired position can be predetermined and achieved once the link mechanism 160 (which is captured within the engagement areas of the valve anchor 104) becomes taut. Thereafter, the support frame 102 is properly positioned within the valve anchor 104 and can be expanded.

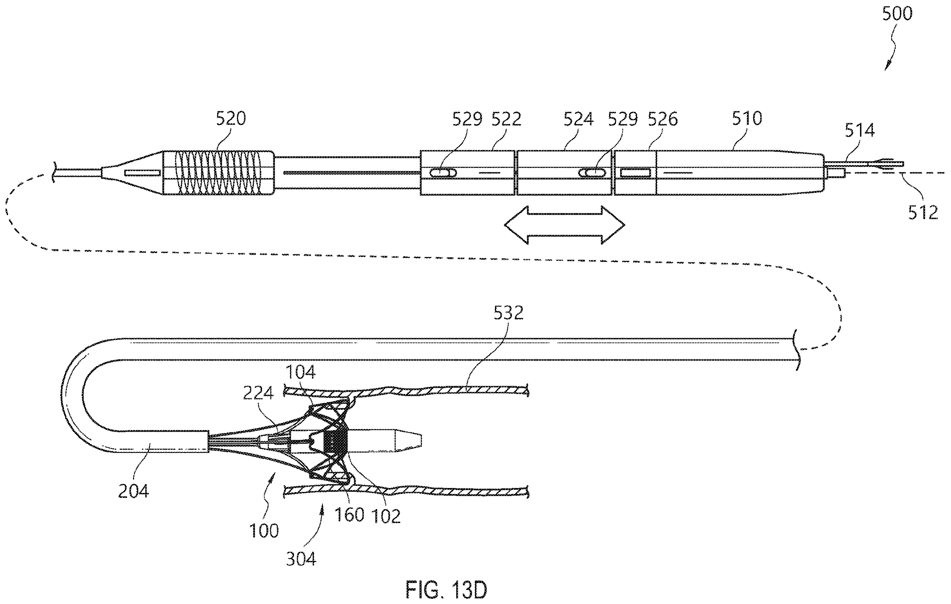

Referring to Figure 13D, the valve anchor 104 is shown in an expanded and rotationally aligned position within the aortic valve 304. During the positioning of the valve anchor 104, the valve anchor 104 can be advanced distally along the longitudinal axis relative to the support frame 102 and become concentric with the support frame 102. This distal advancement of the valve anchor can be achieved by sliding the third movable element 524 along the control handle 510 in an axial direction. This motion is allowed in part due to the movable connection of the link mechanism 160 that interconnects the support frame 102 with the valve anchor 104. Upon reaching the position shown in Figure 13D, the valve anchor has been advanced in a distal direction until U-shaped members of the valve anchor 104 are in contact with the aortic sinuses of the defective native valve leaflets 306, between the leaflets 306 and vessel wall 532.

In some embodiments, the fourth movable element 526 can comprise a safety switch that engages to lock the axial position of the second movable element 522 and/or the third movable element 524 relative to the handle actuator 500.

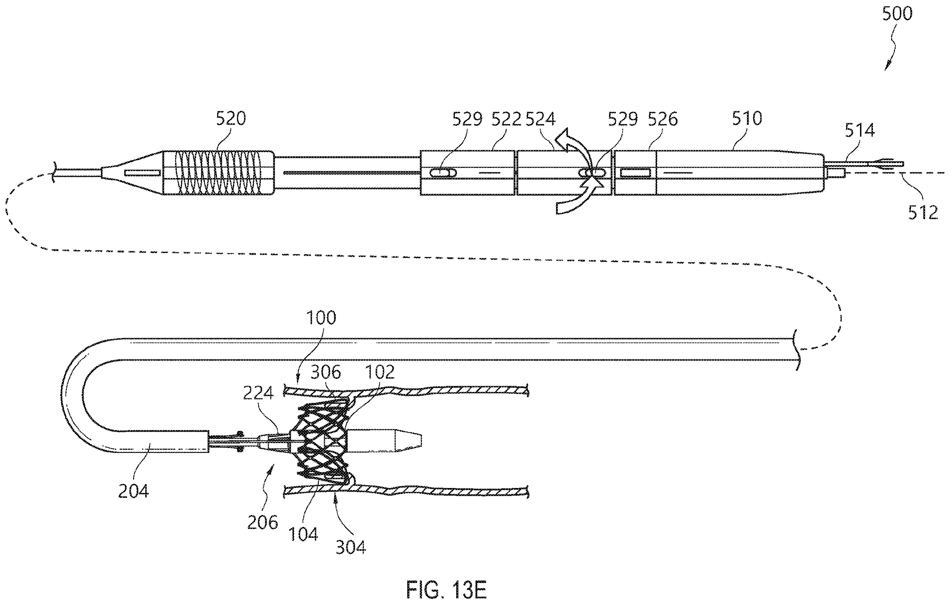

After the support frame 102 is properly positioned within the valve anchor 104, the third movable element 524 may be controlled (e.g., by rotating the third movable element 524) in order to separate the proximal and distal enclosures of the distal carrier assembly 206. For example, Figure 13E illustrates that the third movable element 524 may have a knob that can be rotated in a first direction (e.g., clockwise) in order to separate the proximal and distal enclosures of the distal carrier assembly 206, thereby permitting the support frame 102 to expand within the valve anchor 104 In some embodiments, the proximal enclosure of the distal carrier assembly 206 can be operatively coupled to the third movable element 524 via a control wire.

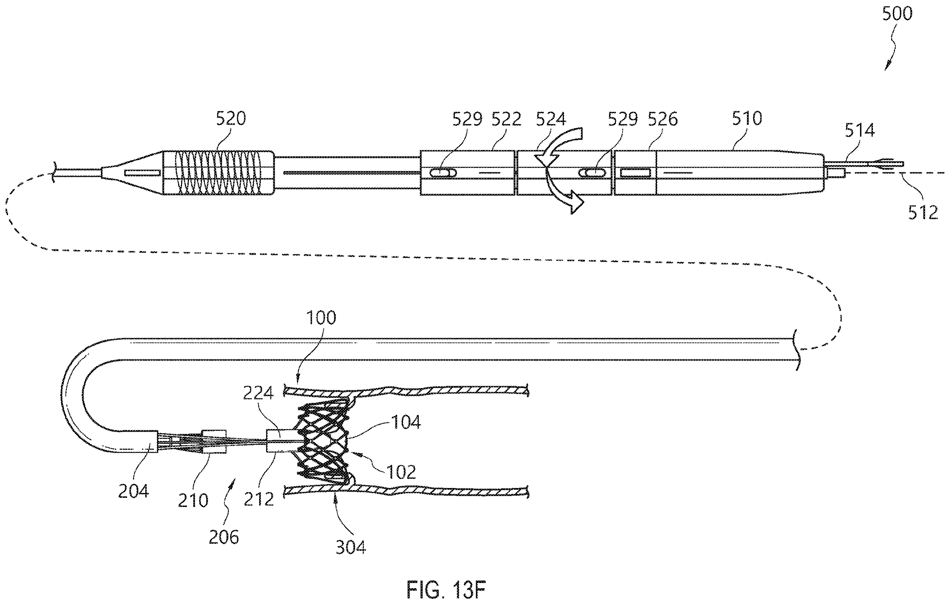

Next, as shown in Figure 13F, after the support frame 102 has been expanded within the valve anchor 104 to sandwich the leaflets 306 between the support frame 102 and the valve anchor 104, the proximal and distal enclosures 210, 212 of the distal carrier assembly 206 can be moved together (for example, until they are in contact, as shown in Figure 8D). The proximal movement of the distal enclosure 212 relative to the proximal enclosure 210 can be achieved by rotating the third movable element 524 in a second direction (e.g., counterclockwise) in order to cause the proximal and distal enclosures 210, 212 to converge toward each other.

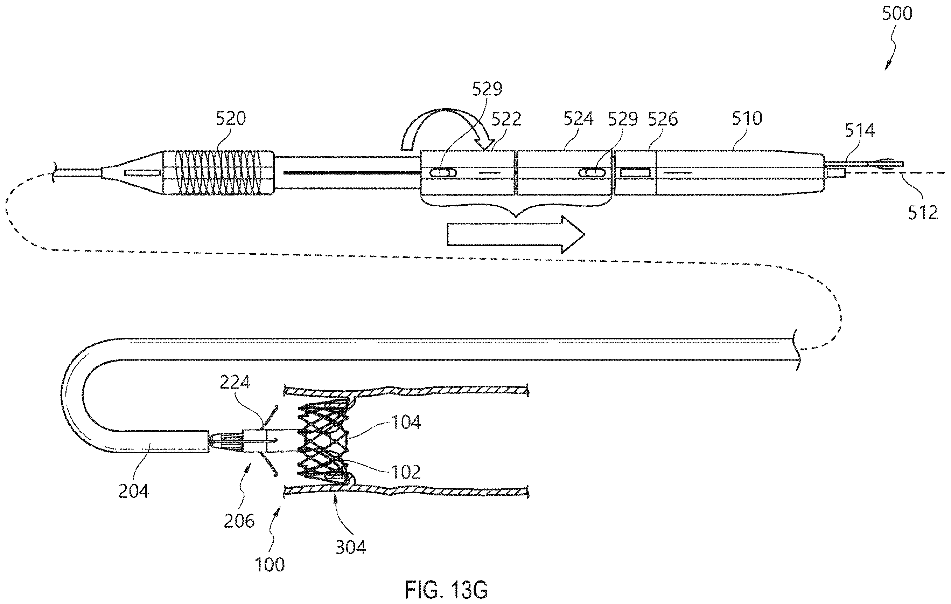

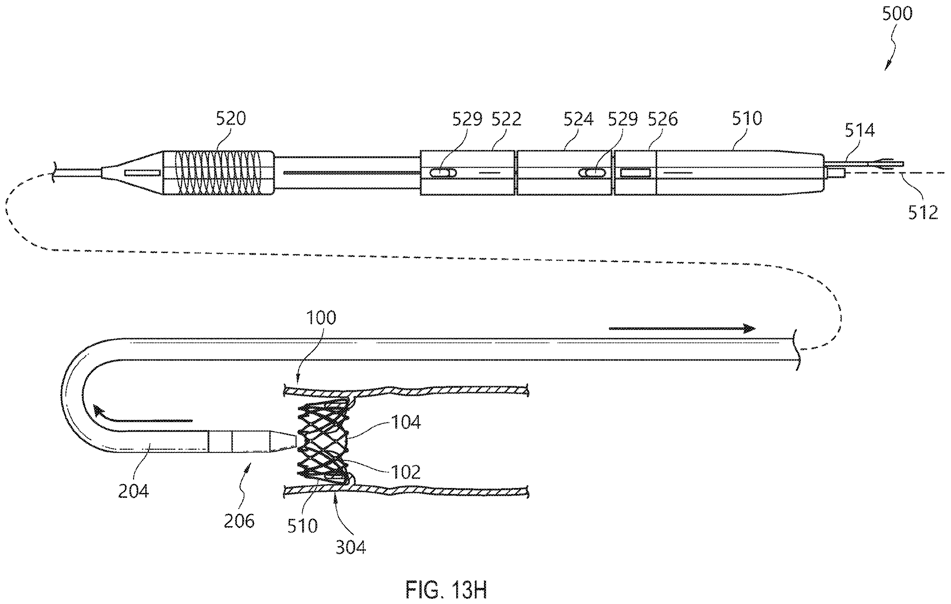

Referring now to Figure 13G, after the support frame 102 has been positioned, expanded, and landed within the aortic valve 304, the valve anchor 104 can be detached from the handle actuator 500. The graspers 224 can be released by controlling the second movable element 522 (e.g., by rotating the second movable element 522 in a first direction). For example, the second movable element 522 may include a knob that can be rotated to release the graspers 224. In some embodiments, the graspers 224 can be proximally withdrawn into the proximal sheath component 204 by linearly moving the second movable element 522 and the third movable element 524 along the handle actuator 500. Thereafter, the catheter 530 and the delivery device 200 can be removed from the patient, as shown in Figure 13H.

Actions performed with the handle actuator 500 to control the support frame 102 and the valve anchor 104 can be altered, modified, substituted, or replaced with another function or feature, in some embodiments. For example, instead of rotation, a movable element can be pressed (e.g., as a button), released, or otherwise actuated. Optionally, the third movable element is rotatable to distally engage the grasper mechanism from the valve anchor. In some embodiments, the handle actuator can include additional movable elements, such as a fourth movable element. Optionally, the fourth control element is rotatable to axially translate the proximal enclosure of the distal carrier assembly relative to the core member for exposing the valve frame. In some embodiments, rotation of the fourth movable element in a first direction axially translates the distal enclosure in a proximal direction, while rotation of the fourth movable element in a second direction axially translates the distal enclosure in a distal direction. In some embodiments, rotation of the fourth movable element axially separates the proximal enclosure from the distal enclosure and/or draws the proximal enclosure toward the distal enclosure. Certain features of the handle, which can be implemented with the handle discussed in the present disclosure, are also further described for example, in U.S. Provisional Application No. 62/756,556, the entirety of which is incorporated herein by reference.

Further, feedback provided to a clinician, such as tactile, auditory, and visual feedback can be altered, modified, substituted, or replaced with another function or feature. In some embodiments, the handle actuator 500 can be manually operated. However, the handle actuator 500 can be motor operated. In some embodiments, the handle actuator 500 can provide electronic control of actuators that are located adjacent to the support frame 102 and the valve anchor 104.

According to some embodiments, the present disclosure optionally provides a membrane that can be used with the presently disclosed valve prosthesis to reduce the diameter of the support frame in a compressed configuration. Figures 14-19 illustrate aspects of the membrane 608 described herein.

Figure 14 illustrates a support frame 102 of a valve prosthesis 600 with a membrane 608 coupled thereto, according to some embodiments. In the embodiment shown in Figure 14, the membrane 608 is positioned or disposed within a lumen or against an inner surface of the expanded support frame 102. As previously described, in some embodiments, the membrane 608 can serve as a sealing component and can be attached to the inside surface, the outside surface, and/or enclose the support frame 102.

In some embodiments, the membrane 608 can be affixed or otherwise attached to the support frame 102 via a plurality of sutures 604. The sutures 604 can attach the membrane 608 to the wire structure of the support frame 102 by passing through the membrane 608 and wrapping around portions of the support frame 102. In the illustrated embodiment of Figure 14, the sutures 604 can be closely spaced (e.g., 2-4 sutures 604 per side of a parallelogram cell or diamond-shaped cell of the support frame 102) to create a tight seal between the membrane 608 and the support frame 102.

In some embodiments, the lateral ends of the membrane 608 can be attached at a seam 602 to form a generally cylindrical shape. As illustrated in Figure 14, axial ends of the membrane 608 can be wrapped around the ends of the support frame 102 at the first end portion 110 and/or the second end portion 112. In some embodiments, the axial ends of the membrane 608 can wrap around the first end portion 110 and/or the second end portion 112 to at least partially cover both the inside surface and the outside surface of the support frame 102. For example, the peaks 606 of the membrane 608 can be wrapped over the peaks 130 of the second end 112 of the support frame 102. Advantageously, by wrapping the axial ends of the membrane 608 around the first and/or second ends 110, 112, the valve prosthesis 600 can be better sealed against the native heart valve. Further, the sutures 604 can be more densely spaced along the axial ends of the membrane 608 than where the membrane 608 is coupled to the support frame 102 intermediate the axial ends of the membrane 608.

As previously described, valve leaflets can be coupled to the membrane 608. In some embodiments, the membrane 608 can restrict blood flow in areas around the valve leaflets so that blood flow occurs only between the valve leaflets through the lumen of the prosthesis 600, as in a healthy native heart valve.

In some embodiments, at the second end portion 112, an axial end of the membrane 608 can be shaped to cover the major peaks 130 and valleys 132 of the second end 112. In some embodiments, the membrane 608 can be shaped to cover the second apices or minor peaks 136 within the valleys 132 between the major peaks 130. Advantageously, the configuration of the minor peaks 136 between the major peaks 130 can allow improved access to and prevent obstructions of the coronary ostia compared to prior art valve prostheses.

For example, referring to Figure 15A, a prior art valve prosthesis 600' is shown within an aorta 696'. The support frame 602' is disposed within the aortic valve annulus 692'. As shown, the support frame 602' and membrane 608' may block or obstruct the coronary ostia 694' disposed a distance 693' away from the valve annulus 692' due to the geometry of the support frame 602' and the membrane 608'.

In contrast, Figure 15B shows an embodiment of the valve prosthesis 600 that advantageously permits blood flow to adjacent coronary ostia 694. As discussed above, and as shown in Figure 15B, the difference in height between the major peaks 130 and the minor peaks 136 of the valve prosthesis 600 facilitates access to the coronary ostia 694 while allowing for desired operation of the valve prosthesis 600.

In some embodiments, the minor peaks 136 of the valve prosthesis 600 can be low enough to allow a variety of sizes and locations of the coronary ostia 694 with respect to the native valve annulus 692 location of a patient. Advantageously, in some embodiments, the minor peaks 136 of the valve prosthesis 600 allow for access to coronary ostia 694 that are less than 10 mm, less than 8 mm, or less than 6 mm in coronary ostia height, which are typically excluded by conventional available prostheses. In some embodiments, the minor peaks 136, and optionally along with one or more other features described herein, allow for access to coronary ostia 694 that are disposed at a lower axial distance 693 relative to the valve annulus 692. For example, the minor peaks 136 can allow for access to coronary ostia 694 disposed at a coronary ostia height or lower axial distance 693 of less than 6 mm between the inferior edge of the coronary artery ostium 694 and the aortic annular plane. Furthermore, in some embodiments, the valve prosthesis 600 can be arranged to be disposed lower in the valve annulus 692 to allow greater access to the coronary ostia 694. By providing minor peaks 136 between the major peaks 130 of the valve prosthesis 600, and optionally used with one or more other features discussed herein, access to the coronary ostia 694 is preserved allowing for future procedures that may require access to the coronary ostia 694, such as coronary stenting.

Figure 16 illustrates a method of forming or manufacturing the membrane for use with the valve prosthesis, which may be a portion of the method of forming the valve prosthesis. In some embodiments, one or more membranes 608 can be cut from a membrane fabric 601. The membrane fabric 601 can be a fabric formed from woven fiber, such as woven polyester. As shown in Figure 17, the membrane fabric 601 is woven together with fibers in a warp direction 608a and a weft direction 608b that are oriented transverse, and in some cases, perpendicular, relative to each other. In some embodiments, a fabric may resist stretching in the warp and weft directions 608a, 608b while allowing stretching and compliance in directions oblique to or biased from the warp and weft directions 608a, 608b.

Referring back to Figure 16, one or more membranes 608 can be cut from the membrane fabric 601 using templates that are generally in the shape of the membranes 608. One or more templates can be placed on the membrane fabric 601 to cut out the membranes 608. While the membrane 608 is shown in a flat orientation in Figure 16, the support frame 102 and the projected longitudinal axis 120 thereof is shown for reference.

The templates can be oriented at an angle relative to the membrane fabric 601. A bias angle 608c can be defined between the edge of the membrane fabric 601 and the projected longitudinal axis 120. For reference, the bias angle 608c is shown between the edge of the membrane fabric 601 and an offset axis 120' that is parallel to the longitudinal axis 120. As discussed further below, the bias angle 608c can be from about 30 degrees to about 60 degrees, such as about 35 degrees, about 40 degrees, about 45 degrees, about 50 degrees, or about 55 degrees.

By orientating the templates at a bias angle 608c relative to the membrane fabric 601, the resulting membrane 608 is cut on the bias with the bias angle 608c with respect to the warp and weft directions 608a, 608b of the membrane fabric 601. In some embodiments, the membrane 608 can be cut at the bias angle 608c by spiral wrapping the membrane fabric onto the support frame and cutting the membrane fabric 601.

Figure 18 illustrates a resultant membrane 608 manufactured using the process shown in Figures 16 and 17. In the depicted embodiment, the warp and weft of the fabric of the membrane 608 is oriented at a bias or diagonal with respect to the direction of a longitudinal axis of the prosthesis. In some embodiments, the bias angle of the membrane 608 can be generally oriented such that the warp or the weft of the material is aligned with expandable elements (e.g., the cells, parallelogram cells, or diamond-shaped cells) of the support frame 102. Advantageously, in some embodiments, the membrane 608 may have increased conformability and may not resist movement or expansion of the support frame 102, thereby reducing stress and bunching of the membrane 608 when the membrane 608 and prosthesis are in a compressed configuration. In some embodiments, the reduction of stress on both the membrane 608 and the support frame 102 can facilitate both assembly and loading of the valve prosthesis.

Indeed, development of some embodiments of the prosthesis has shown that the unique orientation and configuration of the membrane 608 described herein can permit the membrane 608 to more easily radially compress and axially elongate in tandem with the support frame 102, thus permitting the membrane 608 and the support frame 102 to operate as a single unit, in some embodiments. Similarly, in some embodiments, by orientating the membrane 608 along a bias angle, the membrane 608 can more readily elongate along longitudinal axis 120 to obtain a smaller cross-sectional profile, which can prevent flaring, bunching, or pleating, thereby minimizing the cross-sectional profile of the valve prosthesis in a compressed configuration.

Figure 19 is a plan view of the warp 608a and the weft 608b of the membrane 608 relative to the longitudinal axis 120 of the support frame 102. In some embodiments, the warp 608a and weft 608b of the woven membrane 608 can be oriented relative to the longitudinal axis 120 at a bias angle 608c between 0 and 90 degrees. In some embodiments, the woven membrane 608 can be oriented at a bias angle 608c between 15 and 75 degrees relative to the longitudinal axis 120 In some embodiments, the woven membrane 608 can be oriented at a bias angle 608c between 30 and 60 degrees relative to the longitudinal axis 120. In some embodiments, the woven membrane 608 can be oriented at a bias angle 608c of approximately 45 degrees relative to the longitudinal axis 120.

In some embodiments, the warp and weft of the woven membrane 108 can be oriented relative to the longitudinal axis at a bias angle between 0 and 90 degrees. In some embodiments, the woven membrane 108 can be oriented at a bias angle between about 15 and about 75 degrees relative to the longitudinal axis. In some embodiments, the woven membrane 108 can be oriented at a bias angle between about 30 degrees and about 60 degrees relative to the longitudinal axis. In some embodiments, the woven membrane 108 can be oriented at a bias angle of about 45 degrees relative to the longitudinal axis.

### Illustration of Subject Technology as Clauses

Various examples of aspects of the disclosure are described as clause sets having numbered clauses (1, 2, 3, etc.) for convenience. These are provided as examples, and do not limit the subject technology. Identifications of the figures and reference numbers are provided below merely as examples and for illustrative purposes, and the clauses are not limited by those identifications.

### Clause Set 1: Valve Prosthesis

Clause 1. A valve prosthesis comprising: a valve anchor having at least one U-shaped member extending about a longitudinal axis of the valve anchor, the U-shaped member having a peak portion and a base portion, the peak portion having an engagement area, the valve anchor being expandable from a compressed configuration for engaging a native valve structure; an expandable valve frame having a compressed configuration, an expanded configuration, and first and second end portions, the valve frame being configured to expand within the valve anchor at the native valve structure; and a link mechanism interconnecting the valve anchor with the valve frame, the link mechanism being coupled to the valve frame and being slidably coupled to the U-shaped member, the link mechanism being slidable along the U-shaped member from the base portion to be captured within the peak portion engagement area, wherein the link mechanism limits axial movement of the valve frame relative to the valve anchor when captured within the peak portion engagement area.

Clause 2. The valve prosthesis of Clause 1, wherein (i) in a delivery position, the valve anchor and the valve frame are in the compressed configuration, the valve frame is positioned distal to the valve anchor, and the link mechanism extends from the valve anchor base portion across the valve frame first end portion toward the valve frame second end portion, (ii) in an intermediate expanded position, the valve anchor is in an expanded configuration, the valve frame is in the compressed configuration, the valve frame is positioned proximal to the valve anchor, and the link mechanism extends from the valve anchor peak portion toward the valve frame first end portion, and (iii) in an overlapping position, the valve anchor and the valve frame longitudinally overlap each other, the valve frame is positioned within the valve anchor, and the link mechanism extends from the valve anchor peak portion across the valve frame toward the valve frame second end portion.

Clause 3. The valve prosthesis of any preceding Clause, wherein when the link mechanism is engaged in the engagement area, the valve anchor is permitted to expand toward the expanded configuration.

Clause 4. The valve prosthesis of any preceding Clause, wherein when the valve anchor and the valve frame are in the compressed configuration, the link mechanism has a longitudinal extent of between about 110% and about 170% of a longitudinal length of the valve anchor.

Clause 5. The valve prosthesis of any preceding Clause, wherein when the valve anchor and the valve frame are in the expanded configuration, the link mechanism has a longitudinal extent of between about 70% and about 130% of a longitudinal length of the valve anchor.

Clause 6. The valve prosthesis of any preceding Clause, wherein the link mechanism comprises a suture.

Clause 7. The valve prosthesis of Clause 6, the valve anchor comprises a plurality of U-shaped members, the suture having a weave pattern in which the suture extends from (i) a first circumferential position at the valve frame, (ii) then to a first leg of a first U-shaped member, (iii) then to a second leg of a second U-shaped member, the first U-shaped member being interconnected to the second U-shaped member, (iv) and then to the first circumferential position.

Clause 8. The valve prosthesis of Clause 7, wherein the second U-shaped member comprises a link motion limiter extending from a medial location of the base portion thereof.

Clause 9. The valve prosthesis of Clause 8, wherein the link mechanism comprises a plurality of sutures.

Clause 10. The valve prosthesis of Clause 9, wherein the weave pattern of a second suture of the plurality of sutures extends (i) from a first leg of the second U-shaped member to (ii) a second leg of a third U-shaped member and, (iii) then to a second circumferential position at the valve frame.

Clause 11. The valve prosthesis of Clause 6, the valve anchor comprises a plurality of U-shaped members, the suture having a weave pattern in which the suture extends from (i) a first circumferential position at the valve frame, (ii) then to a first leg of a first U-shaped member, (iii) then to a second leg of a second U-shaped member, the first U-shaped member being interconnected to the second U-shaped member, (iv) and then to a second circumferential position at the valve frame, different from the first circumferential position.

Clause 12. The valve prosthesis of Clause 11, wherein the suture comprises a continuous suture loop interwoven with the valve frame and the valve anchor.

Clause 13. The valve prosthesis of Clause 12, wherein the weave pattern of the continuous suture loop further extends (i) from the first leg of the first U-shaped member to the second leg of the second U-shaped member on a first side of a link motion limiter of the second U-shaped member, wherein the link motion limiter extends from a medial location of the base portion thereof, (ii) then to the second circumferential position at the valve frame, (iii) then to a first leg of the second U-shaped member on a second side of the link motion limiter, (iv) then to a second leg of a third U-shaped member, and (v) then to a third circumferential position at the valve frame.

Clause 14. The valve prosthesis of Clause 13, wherein the first, second, and third U-shaped members each comprises a respective link motion limiter, and wherein in the weave pattern of the continuous suture loop, the link mechanism is coupled to a given U-shaped member on both sides of the link motion limiter.

Clause 15. The valve prosthesis of Clause 12, the valve anchor comprises a plurality of U-shaped members, the link mechanism being interwoven with three U-shaped members of the valve anchor, and wherein a loop length of the continuous suture loop is between about 80% to about 120% of a sum of (i) a compressed circumference of the valve frame and (ii) six times a longitudinal length of the valve frame.

Clause 16. The valve prosthesis of Clause 6, wherein the valve anchor comprises first, second, and third U-shaped members, each of the first, second, and third U-shaped members having a link motion limiter extending from a medial location of the base portion thereof, and wherein the link mechanism comprises a continuous suture loop extending (i) from a given U-shaped member on a first side of the link motion limiter, (ii) then to a given circumferential position at the valve frame, and (iii) then to the given U-shaped member on a second side of the link motion limiter.

Clause 17. The valve prosthesis of any preceding Clause, wherein the peak portions of adjacent U-shaped members are coupled together to form a respective engagement area.

Clause 18. The valve prosthesis of any preceding Clause, wherein the engagement area of the peak portion comprises double peaks having a cove shape disposed therebetween for receiving and retaining the link mechanism therein.

Clause 19. A method for delivering a prosthetic heart valve prosthesis to a native valve structure of a patient, the method comprising: introducing the valve prosthesis into the patient at the implantation site, the valve prosthesis including a valve anchor having a U-shaped member with a peak portion and a base portion, an expandable valve frame, and a link mechanism interconnecting the valve anchor and the valve frame, the valve anchor being restrained in a compressed configuration within a proximal sheath, the valve frame being restrained in a compressed configuration within a distal carrier assembly; permitting expansion of the base portion of the valve anchor; distally urging the base portion of the valve anchor into engagement with a native valve structure; proximally retracting the valve frame relative to the valve anchor to slide the link mechanism proximally toward an engagement area of the peak portion to urge the link mechanism into the engagement area, thereby capturing the link mechanism therein and restricting a range of movement of the valve frame relative to the valve anchor; releasing the peak portion of the valve anchor to permit the valve anchor to expand against the native valve structure; and permitting expansion of the valve frame within a lumen of the valve anchor.

Clause 20. The method of Clause 19, wherein prior to the permitting expansion of the valve frame, the method further comprises distally advancing the valve frame into the valve anchor.

Clause 21. The method of Clause 20, wherein the distally advancing the valve frame into the valve anchor comprises distally advancing the valve frame until further distal movement of the valve frame relative to the valve anchor is restricted by the link mechanism.

Clause 22. The method of Clause 19-21, wherein after the proximally retracting the valve frame, the method further comprises distally advancing the valve frame, with the link mechanism being engaged at the engagement area of the peak portion of the valve anchor, until the link mechanism is taut and further distally advancing the valve frame to pull the valve anchor distally relative to the native valve structure.

Clause 23. The method of Clause 19-22, wherein after the proximally retracting the valve frame, the method further comprises rotating the valve frame, with the link mechanism being engaged at the engagement area of the peak portion of the valve anchor, to rotationally adjust a position of the valve anchor relative to the native valve structure.

Clause 24. A valve prosthesis comprising: a valve anchor having at least one U-shaped member extending about a longitudinal axis of the valve anchor, the U-shaped member having a peak portion and a base portion, the valve anchor being expandable from a compressed configuration for engaging a native valve structure; an expandable valve frame having a compressed configuration, and an expanded configuration, the valve frame being configured to expand within the valve anchor at the native valve structure; and a link mechanism interconnecting the valve anchor with the valve frame, the link mechanism being coupled to the valve frame and being coupled to the U-shaped member, wherein the link mechanism limits axial movement of the valve frame relative to the valve anchor.

Clause 25. A valve prosthesis comprising: a valve anchor having at least one U-shaped member extending about a longitudinal axis of the valve anchor, the U-shaped member having a peak portion and a base portion, the peak portion having an engagement area, the base portion having a clasper tang extending from a medial location thereof, the valve anchor being expandable from a compressed configuration for engaging a native valve structure.

Clause 26. The valve prosthesis of Clause 25, wherein the clasper tang is configured to be coupled to a grasper mechanism of a delivery device.

Clause 27. The valve prosthesis of Clause 25 or 26, further comprising: an expandable valve frame having a compressed configuration, an expanded configuration, and first and second end portions, the valve frame being configured to expand within the valve anchor at the native valve structure.

Clause 28. The valve prosthesis of Clause 27, further comprising: a link mechanism interconnecting the valve anchor with the valve frame, the link mechanism being coupled to the valve frame and being slidably coupled to the U-shaped member, the link mechanism being slidable along the U-shaped member from the base portion to be captured within the peak portion engagement area, wherein the link mechanism limits axial movement of the valve frame relative to the valve anchor when captured within the peak portion engagement area.

Clause 29. The valve prosthesis of Clause 28, wherein the link mechanism comprises a suture.

Clause 30. The valve prosthesis of Clause 29, the valve anchor comprises a plurality of U-shaped members, the suture having a weave pattern in which the suture extends from (i) a first circumferential position at the valve frame, (ii) then to a first leg of a first U-shaped member, (iii) then to a second leg of a second U-shaped member, the first U-shaped member being interconnected to the second U-shaped member, (iv) and then to the first circumferential position.

Clause 31. The valve prosthesis of Clause 30, wherein the clasper tang is configured to limit motion of the suture.

Clause 32. The valve prosthesis of Clause 30, wherein the plurality of U-shaped members each comprises a respective clasper tang, and wherein in the weave pattern of the suture, the link mechanism is coupled to a given U-shaped member on both sides of the clasper tang.

Clause 33. A valve prosthesis comprising: a valve anchor having at least one U-shaped member extending about a longitudinal axis of the valve anchor, the valve anchor being expandable from a compressed configuration for engaging a native valve structure; an expandable valve frame having a compressed configuration, an expanded configuration, and first and second end portions, the valve frame being configured to expand within the valve anchor at the native valve structure; and a link mechanism interconnecting the valve anchor with the valve frame, the link mechanism being fixedly coupled to the valve frame and to the U-shaped member, wherein the link mechanism limits axial movement of the valve frame relative to the valve anchor.

Clause 34. The valve prosthesis of Clause 33, wherein when the valve anchor and the valve frame are in the compressed configuration, the link mechanism has a longitudinal extent of between about 110% and about 170% of a longitudinal length of the valve anchor.

Clause 35. The valve prosthesis of Clause 34, wherein the link mechanism elongates to the longitudinal extent.

Clause 36. The valve prosthesis of Clause 34, wherein the link mechanism stretches to the longitudinal extent.

Clause 37. The valve prosthesis of Clause 34, wherein the link mechanism deforms to the longitudinal extent.

Clause 38. The valve prosthesis of Clause 33-37, wherein the link mechanism comprises silicone or polyurethane.

Clause 39. The valve prosthesis of Clause 33-38, wherein when the valve anchor and the valve frame are in the expanded configuration, the link mechanism has a longitudinal extent of between about 70% and about 130% of a longitudinal length of the valve anchor.

Clause 40. The valve prosthesis of Clause 33-39, wherein the link mechanism comprises a suture.

Clause 41. The valve prosthesis of Clause 40, the valve anchor comprises a plurality of U-shaped members, the suture having a weave pattern in which the suture extends from (i) a first circumferential position at the valve frame, (ii) then to a first leg of a first U-shaped member, (iii) then to a second leg of a second U-shaped member, the first U-shaped member being interconnected to the second U-shaped member, (iv) and then to the first circumferential position.

Clause 42. The valve prosthesis of Clause 33-41, wherein the link mechanism is fixedly coupled to a leg of the valve anchor.

Clause 43. The valve prosthesis of Clause 33-42, wherein the link mechanism comprises a coiled portion.

Clause 44. The valve prosthesis of Clause 33-43, wherein the link mechanism comprises a laser-cut portion.

Clause 45. The valve prosthesis of Clause 33-44, further comprising a one-way interconnect mechanism, the one-way interconnect mechanism allowing proximal movement of the link mechanism

Clause 46. The valve prosthesis of Clause 45, wherein the one-way interconnect mechanism prevents distal advancement of the link mechanism relative to the one-way interconnect mechanism.

Clause 47. The valve prosthesis of Clause 45, wherein the one-way interconnect mechanism comprises a mechanism body with an aperture, the aperture permitting the link mechanism to pass therethrough.

Clause 48. The valve prosthesis of Clause 47, wherein the link mechanism ratchets through the one-way interconnect mechanism.

Clause 49. A method for delivering a prosthetic heart valve prosthesis to a native valve structure of a patient, the method comprising: introducing the valve prosthesis into the patient at the implantation site, the valve prosthesis including a valve anchor having a U-shaped member, an expandable valve frame, and a link mechanism fixedly coupled to the valve anchor and the valve frame, the valve anchor being restrained in a compressed configuration within a proximal sheath, the valve frame being restrained in a compressed configuration within a distal carrier assembly; permitting expansion of the valve anchor; distally urging a base portion of the valve anchor into engagement with a native valve structure; restricting a range of movement of the valve frame relative to the valve anchor via the link mechanism; releasing a peak portion of the valve anchor to permit the valve anchor to expand against the native valve structure; and permitting expansion of the valve frame within a lumen of the valve anchor.

Clause 50. The method of Clause 49, wherein prior to the permitting expansion of the valve frame, the method further comprises distally advancing the valve frame into the valve anchor.

Clause 51. The method of Clause 50, wherein the distally advancing the valve frame into the valve anchor comprises distally advancing the valve frame until further distal movement of the valve frame relative to the valve anchor is restricted by the link mechanism.

Clause 52. The method of Clause 49-51, wherein after the proximally retracting the valve frame, the method further comprises distally advancing the valve frame, applying tension to the link mechanism until the link mechanism is taut and further distally advancing the valve frame to pull the valve anchor distally relative to the native valve structure.

Clause 53. The method of Clause 49-52, wherein after the proximally retracting the valve frame, the method further comprises rotating the valve frame and applying tension to the link mechanism to rotationally adjust a position of the valve anchor relative to the native valve structure.

Clause 54. The method of Clause 49-53, further comprising reducing a longitudinal extent of the link mechanism.

Clause 55. The method of Clause 54, further comprising coiling the link mechanism to reduce the longitudinal extent of the link mechanism.

Clause 56. The method of Clause 54, further comprising proximally moving the link mechanism through a one-way interconnect to reduce the longitudinal extent of the link mechanism.

Clause 57. The method of Clause 56, further comprising preventing distal advancement of the link mechanism through the one-way interconnect.

### Clause Set 2: Additional features of the Valve Prosthesis

Clause 1. A valve prosthesis comprising: a support frame comprising a plurality of cells arranged to define a bottom edge of the support frame, a plurality of major peak portions opposite the bottom edge, and at least one minor peak portion disposed longitudinally intermediate the bottom edge and the plurality of major peak portions; and a membrane attached to the support frame.

Clause 2. The valve prosthesis of Clause 1, wherein the membrane comprises a first end portion corresponding to the bottom edge of the support frame, a plurality of membrane major peak portions corresponding to the plurality major peak portions of the support frame, and at least one membrane minor peak portion corresponding to the at least one minor peak portion of the support frame.

Clause 3. The valve prosthesis of Clause 2, wherein one or more membrane major peak portions of the plurality of membrane major peak portions are wrapped around corresponding one or more major peak portions of the plurality of major peak portions of the support frame to cover a part of an inner surface and a part of an outer surface of the support frame at the corresponding one or more major peak portions of the support frame.

Clause 4. The valve prosthesis of Clause 3, wherein one or more membrane minor peak portions of the at least one membrane minor peak portion are wrapped around corresponding one or more minor peak portions of the at least one minor peak portion of the support frame to cover a part of an inner surface and a part of an outer surface of the support frame at the corresponding one or more minor peak portions of the support frame.

Clause 5. The valve prosthesis of any preceding Clause, wherein the at least one minor peak portion is disposed at a lower axial distance from the bottom edge compared to the plurality of major peak portions.

Clause 6. The valve prosthesis of any preceding Clause, wherein the membrane encloses the support frame with one or more openings along a longitudinal axis of the support frame.

Clause 7. The valve prosthesis of any preceding Clause, wherein the membrane is disposed within a lumen of the support frame to be attached to an inner surface of the support frame.

Clause 8. The valve prosthesis of any preceding Clause, wherein the membrane is laminated onto inner and outer surfaces of the support frame.

Clause 9. The valve prosthesis of any preceding Clause, wherein the membrane is attached to the support frame via a plurality of sutures.

Clause 10. The valve prosthesis of Clause 9, wherein the plurality of sutures are passed through the membrane and are wrapped around a plurality of portions of the support frame.

Clause 11. The valve prosthesis of Clause 9, wherein a first set of the plurality of sutures along a plurality of top portions corresponding to the plurality of major peak portions and the at least one minor peak portion of the support frame is more densely spaced than a second set of the plurality of sutures coupled to portions of the support frame intermediate the plurality of top portions and a plurality of base portions.

Clause 12. The valve prosthesis of any preceding Clause, wherein membrane fabric of the membrane is formed from woven fiber.

Clause 13. The valve prosthesis of Clause 12, wherein the membrane fabric is formed by weaving fibers in a warp direction and a weft direction that are oriented transverse relative to each other.

Clause 14. The valve prosthesis of Clause 13, wherein the membrane fabric resists stretching in the warp direction and the weft direction of the fibers.

Clause 15. The valve prosthesis of Clause 13, wherein the membrane fabric stretches in one or more directions oblique to the warp direction or the weft direction of the fibers.

Clause 16. The valve prosthesis of any preceding Clause, further comprising: a plurality of valve leaflets coupled to the membrane.

Clause 17. The valve prosthesis of Clause 16, wherein the membrane is coupled to the plurality of valve leaflets to restrict fluid flow around the plurality of valve leaflets and to direct the fluid flow to an area between the plurality of valve leaflets.

Clause 18. A method of manufacturing a membrane for a support frame of a valve prosthesis, the method comprising: creating a template on a membrane fabric in a shape of one or more membranes to be attached to a support frame of a valve prosthesis; orientating the template at a bias angle relative to the membrane fabric; and generating one or more membranes from the membrane fabric using the template.

Clause 19. The method of Clause 18, wherein the membrane fabric is formed from woven fiber.

Clause 20. The method of Clause 19, wherein the membrane fabric is formed by weaving fibers in a warp direction and a weft direction that are oriented transverse relative to each other.

Clause 21. The method of Clause 20, wherein the membrane fabric resists stretching in the warp direction and the weft direction of the fibers.

Clause 22. The method of Clause 20, wherein the membrane fabric stretches in one or more directions oblique to the warp direction or the weft direction of the fibers.

Clause 23. The method of Clause 18-22, wherein the bias angle is about 30 degrees to about 60 degrees.

Clause 24. A valve prosthesis, comprising: a support frame having a longitudinal axis; and a membrane coupled to the support frame, the membrane comprising: a plurality of first fibers arranged in a warp direction, and a plurality of second fibers arranged in a weft direction that is transverse relative to the warp direction, the plurality of first fibers and the plurality of second fibers being woven together, the warp and weft directions being oblique relative to the longitudinal axis of the support frame.

Clause 25. The valve prosthesis of Clause 24, wherein the warp and weft directions are aligned with expandable elements of the support frame, the expandable elements being expandable during a transition between a compressed configuration and an expanded configuration of the support frame.

Clause 26. A method of manufacturing a valve prosthesis, the method comprising: aligning a membrane along a support frame having a longitudinal axis, the membrane comprising: a plurality of first fibers arranged in a warp direction, and a plurality of second fibers arranged in a weft direction that is transverse relative to the warp direction, the plurality of first fibers and the plurality of second fibers being woven together, the warp and weft directions being oblique relative to the longitudinal axis of the support frame; and attaching the membrane to the support frame.

Clause 27. The method of Clause 26, wherein the membrane is attached to the support frame via a plurality of sutures, the plurality of sutures permitting movement of the membrane relative to the support frame when the membrane is attached to the support frame.

Clause 28. The method of Clause 27, wherein the support frame is made of a wire structure, and wherein the membrane is attached to the support frame by passing the plurality of sutures through the membrane and wrapping around a plurality of portions of the wire structure of the support frame.

Clause 29. The method of Clause 26-28, wherein the support frame includes one or more peak portions and one or more base portions, and the membrane includes one or more membrane peak portions corresponding to the one or more peak portions of the support frame and the one or more membrane base portions corresponding to the one or more base portions of the support frame.

Clause 30. The method of Clause 29, further comprising: wrapping the one or more membrane peak portions over the one or more peak portions of the support frame, respectively.

Clause 31. The method of Clause 26, further comprising: forming a cylindrical shape with the membrane by attaching one lateral end of the membrane to another lateral end of the membrane, the cylindrical shape corresponding to a shape of the support frame.

Clause 32. The method of Clause 26, wherein the warp and weft directions are aligned with expandable elements of the support frame, the expandable elements being expandable during a transition between a compressed configuration and an expanded configuration of the support frame.

Clause 33. A method for delivering a prosthetic heart valve prosthesis to a native valve structure of a patient at an implantation site, the method comprising: introducing the valve prosthesis into the patient at the implantation site, the valve prosthesis including a valve anchor and an expandable valve frame comprising a bottom edge, a plurality of major peak portions, and at least one minor peak portion disposed longitudinally intermediate the bottom edge and the plurality of major peak portions; permitting expansion of the valve anchor; distally urging a portion of the valve anchor into engagement with a native valve structure; permitting the valve anchor to expand against the native valve structure; rotating the valve frame to rotationally align the minor peak portion with an ostia at the implantation site; and permitting expansion of the valve frame within a lumen of the valve anchor.

Clause 34. The method of Clause 33, wherein the minor peak portion is disposed at a lower axial distance relative to the ostia.

Clause 35. The method of Clause 34, wherein the distally advancing the valve frame into the valve anchor comprises distally advancing the valve frame until further distal movement of the valve frame relative to the valve anchor is restricted by a link mechanism.

Clause 36. The method of Clause 33-35, wherein prior to the permitting expansion of the valve frame, the method further comprises distally advancing the valve frame into the valve anchor.

### Clause Set 3: Delivery Device

A valve prosthesis delivery device for delivering a valve prosthesis, the valve prosthesis comprising a valve frame and a valve anchor, the delivery device comprising: an elongate core member extending along a longitudinal axis of the system; a proximal sheath component slidably coupled to the core member and comprising a proximal sheath lumen, the proximal sheath component being configured to receive at least a portion of the valve anchor within the proximal sheath lumen, the proximal sheath being proximally retractable relative to the core member for permitting expansion of the valve anchor; and a distal carrier assembly comprising a distal enclosure and a proximal enclosure, the distal enclosure being coupled to the core member and comprising a distal lumen, the distal enclosure being configured to receive a distal portion of the valve frame within the distal lumen in a compressed configuration, the proximal enclosure being coupled to the core member proximal to the distal enclosure and comprising a proximal lumen, the proximal enclosure being configured to receive a proximal portion of the valve frame within the proximal lumen in a compressed configuration.

Clause 2. The delivery device of Clause 1, further comprising a plunger mechanism and a biasing means disposed within the distal lumen of the distal carrier assembly, the plunger mechanism being moveable between a distal position and a proximal position within the distal lumen, the plunger mechanism being biased toward the proximal position by the biasing means for urging the valve frame out of the distal lumen.

Clause 3. The delivery device of Clause 2, wherein the plunger mechanism comprises a plunger head that is moveable between the distal position and the proximal position within the distal lumen, the plunger head being biased toward the proximal position by the biasing means.

Clause 4. The delivery device of Clause 3, wherein in the proximal position, the plunger head extends at least partially proximally out of the distal lumen.

Clause 5. The delivery device of Clause 3, wherein in the proximal position, an outer surface of the plunger head is in contact with both the inner surface of the distal lumen and an inner surface of the proximal lumen when the proximal enclosure is distally advanced over the plunger head.

Clause 6. The delivery device of Clause 3, further comprising the valve prosthesis, wherein when the valve frame is positioned within the distal lumen, the plunger head is urged to the distal position.

Clause 7. The delivery device of Clause 3, wherein the biasing means comprises a spring.

Clause 8. The delivery device of Clause 7, wherein the biasing means comprises a spring, the spring being interposed between a proximal face of the distal enclosure and a distal face of the plunger head.

Clause 9. The delivery device of Clause 2, wherein the plunger mechanism comprises a proximal portion that tapers in a proximal direction.

Clause 10. The delivery device of Clause 9, wherein the proximal portion comprises a conical shape.

Clause 11. The delivery device of Clause 9, wherein in the proximal position, the plunger mechanism extends at least partially proximally out of the distal lumen, the proximal enclosure being distally advanceable over the tapered proximal portion of the plunger mechanism to align an outer surface of the distal enclosure with an outer surface of the proximal enclosure for facilitating removal of the delivery device from a patient.

Clause 12. The delivery device of any preceding Clause, wherein the distal enclosure comprises a distal nose cone that tapers in a distal direction.

Clause 13. The delivery device of any preceding Clause, further comprising the valve prosthesis, wherein in a loaded configuration, a proximal end of the distal enclosure is spaced longitudinally apart from a distal end of the proximal enclosure to permit a longitudinal section of the valve frame to remain exposed in the loaded configuration.

Clause 14. The delivery device of any preceding Clause, further comprising the valve prosthesis.

Clause 15. The delivery device of any preceding Clause, wherein the proximal enclosure comprises a proximal section having an outer diameter smaller than an inner diameter of the proximal lumen, and wherein when a valve anchor is loaded onto the delivery device, the valve anchor extends between the proximal section and the proximal sheath in a loaded configuration.

Clause 16. The delivery device of Clause 15, wherein the proximal section tapers in a proximal direction.

Clause 17. The delivery device of Clause 15, wherein the proximal section has a plurality of circumferential nodes, the plurality of circumferential nodes extending proximally from a proximal abutment surface of the proximal enclosure.

Clause 18. The delivery device of Clause 17, wherein the plurality of circumferential nodes comprises three circumferential nodes that are evenly circumferentially spaced apart from each other.

Clause 19. The delivery device of Clause 17, wherein the valve anchor comprises a plurality of U-shaped members, and wherein the proximal section has a plurality of circumferential cavities each configured to receive a respective U-shaped member of the valve anchor when the valve anchor is loaded onto the delivery device in a compressed configuration.

Clause 20. The delivery device of Clause 19, wherein, when the valve anchor is loaded onto the delivery device, each of the U-shaped members is positioned adjacent to a respective one of the plurality of circumferential cavities between the proximal section and the proximal sheath component.

Clause 21. The delivery device of Clause 20, wherein in a delivery configuration, the proximal enclosure is spaced apart from the distal enclosure by a gap to permit a portion of the valve frame to be exposed through the gap.

Clause 22. The delivery device of Clause 21, wherein the valve prosthesis includes a link mechanism interconnecting the valve anchor and the valve frame, and wherein the link mechanism extends through the gap, the link mechanism being moveable relative to the valve frame after releasing the valve anchor from the proximal sheath component.

Clause 23. A valve prosthesis delivery device for delivering a valve prosthesis, the valve prosthesis comprising a valve frame and a valve anchor, the delivery device comprising: an elongate core member extending along a longitudinal axis of the system; a sheath component slidably coupled to the core member and comprising a sheath lumen, the sheath component being configured to receive at least a portion of the valve anchor within the sheath lumen, the sheath being moveable relative to the core member for permitting expansion of the valve anchor; and a carrier assembly comprising a distal enclosure and a proximal enclosure, the enclosure being coupled to the core member and comprising a distal lumen, the enclosure being configured to receive a distal portion of the valve frame within the distal lumen in a compressed configuration, the proximal enclosure being coupled to the core member proximal to the enclosure and comprising a proximal lumen, the proximal enclosure being configured to receive a proximal portion of the valve frame within the proximal lumen in a compressed configuration.

Clause 24. The delivery device of Clause 23, wherein the sheath component is positioned proximally relative to the carrier assembly.

Clause 25. The delivery device of Clause 23, comprising any of the features recited in any of Clauses 1-21.

Clause 26. A method for delivering a prosthetic heart valve prosthesis to a native valve structure of a patient, the valve prosthesis comprising a valve frame and a valve anchor, the method comprising: introducing the valve prosthesis into the patient at an implantation site via a valve prosthesis delivery device, the system comprising a proximal sheath component and a distal carrier assembly, the proximal sheath component receiving at least a portion of the valve anchor in a proximal sheath lumen, and the distal carrier assembly comprising a distal enclosure and a proximal enclosure, the distal enclosure being configured to receive at least a distal portion of the valve frame; proximally retracting the proximal sheath in a proximal direction to permit expansion of the valve anchor; and expanding the distal carrier assembly to permit expansion of the valve frame.

Clause 27. The method of Clause 26, further comprising distally urging a base portion of the valve anchor into engagement with a native valve structure.

Clause 28. The method of Clause 26 or 27, further comprising proximally retracting the valve frame relative to the valve anchor to engage a link mechanism therebetween and restricting a range of movement of the valve frame relative to the valve anchor.

Clause 29. The method of Clause 28, further comprising distally advancing the valve frame.

Clause 30. The method of Clause 26, wherein the expanding longitudinally separating the distal enclosure from the proximal enclosure to permit expansion of the distal portion of the valve frame.

Clause 31. The method of Clause 26-30, wherein the expanding comprises distally advancing the distal enclosure relative to the proximal enclosure to permit expansion of the distal portion of the valve frame.

Clause 32. The method of Clause 26-31, wherein the expanding comprises proximally retracting the proximal enclosure relative to the distal enclosure to permit expansion of the proximal portion of the valve frame.

Clause 33. The method of Clause 26-32, wherein the expanding comprises proximally retracting the distal enclosure relative to the proximal enclosure and distally advancing the proximal enclosure relative to the distal enclosure to move the distal carrier assembly to a retrieval configuration for retracting the valve prosthesis delivery device from the patient.

Clause 34. The method of Clause 26-33, further comprising extending a plunger mechanism at least partially proximally out of the distal enclosure toward the proximal enclosure, aligning an outer surface of the distal enclosure with an outer surface of the proximal enclosure.

Clause 35. The method of Clause 26-34, wherein the proximal enclosure comprises a proximal section having an outer diameter smaller than the proximal enclosure, and wherein when a valve anchor is loaded onto the delivery device, the valve anchor extends between the proximal section and the proximal sheath in a loaded configuration.

Clause 36. The method of Clause 35, wherein the proximal section tapers in a proximal direction.

Clause 37. A method for delivering a prosthetic heart valve prosthesis to a native valve structure of a patient, the method comprising: introducing the valve prosthesis into the patient at an implantation site via a valve prosthesis delivery device, the valve prosthesis comprising a valve frame coupled to a valve anchor via a link mechanism, the valve prosthesis being carried by the delivery device; permitting expansion of the valve anchor; and with the valve anchor expanded, proximally retracting the valve frame within and relative to the valve anchor to proximally slide the link mechanism along the valve anchor toward an engagement region of the valve anchor for capturing the link mechanism in the engagement region for restricting a range of movement of the valve frame relative to the valve anchor.

Clause 38. The method of Clause 37, wherein the delivery device comprises a proximal sheath component and a distal carrier assembly, the proximal sheath component receiving at least a portion of the valve anchor in a proximal sheath lumen, and wherein the permitting expansion of the valve anchor comprises proximally retracting the proximal sheath in a proximal direction relative to the valve anchor to permit expansion of the valve anchor.

Clause 39. The method of Clause 38, the distal carrier assembly comprising a distal enclosure and a proximal enclosure.

Clause 40. The method of Clause 38, further comprising distally advancing the valve frame into the valve anchor.

Clause 41. The method of Clause 38, further comprising expanding the distal carrier assembly to permit expansion of the valve frame.

Clause 42. The method of Clause 38, wherein the distally advancing the valve frame into the valve anchor comprises distally advancing the valve frame until further distal movement of the valve frame relative to the valve anchor is restricted by the link mechanism.

Clause 43. The method of Clause 37-42, further comprising distally advancing the valve frame to pull the valve anchor distally relative to the native valve structure.

Clause 44. The method of Clause 37-43, further comprising rotating the valve frame, to rotationally adjust a position of the valve anchor relative to the native valve structure.

Clause 45. The method of Clause 37-44, wherein the permitting expansion of the valve frame comprises proximally retracting a proximal sheath of a delivery device to permit expansion of a base portion of the valve anchor.

Clause 46. The method of Clause 45, wherein a peak portion of the valve anchor is coupled to a grasper mechanism of a delivery device, and wherein distally urging the base portion of the valve anchor comprises distally advancing the grasper mechanism to convey a distal force to the valve anchor.

Clause 47. The method of Clause 45, wherein a base portion of the valve anchor is coupled to a grasper mechanism of a delivery device, and wherein distally urging the base portion of the valve anchor comprises distally advancing the grasper mechanism to convey a distal force to the valve anchor.

Clause 48. The method of Clause 38, wherein the permitting expansion of the valve frame comprises proximally retracting a proximal enclosure of the distal carrier assembly to expose a proximal portion of the valve frame.

Clause 49. The method of Clause 48, wherein the permitting expansion of the valve frame further comprises permitting a plunger mechanism to proximally urge a distal portion of the valve frame out of a distal enclosure of the distal carrier assembly to facilitate release of the valve frame from the distal carrier assembly.

Clause 50. The method of Clause 49, wherein the distal carrier assembly comprises a distal lumen and the plunger mechanism has a plunger head and biasing means disposed within the distal lumen, the plunger head being biased in a proximal direction by the biasing means, and wherein the permitting expansion of the valve frame comprises permitting the plunger head to move proximally within the distal lumen and to exert a proximally oriented force on the valve frame to release the valve frame from the distal lumen.

Clause 51. The method of Clause 49, wherein after the valve frame has been released, the method further comprises distally advancing the proximal enclosure over a proximal conical portion of the plunger mechanism to align an outer surface of the proximal enclosure with an outer surface of the distal enclosure of the distal carrier assembly.

Clause 52. The method of Clause 51, wherein the distally advancing the proximal enclosure over a proximal conical portion comprises abutting a distal end of the proximal enclosure against a proximal end of the distal enclosure.

### Clause Set 4: Additional Features of the Delivery Device

Clause 1. A valve prosthesis delivery device for delivering a valve prosthesis, the valve prosthesis comprising a valve frame and a valve anchor, the delivery device comprising: an elongate core member extending along a longitudinal axis of the system; a proximal sheath component slidably coupled to the core member and comprising a proximal sheath lumen, the proximal sheath component being configured to receive at least a portion of the valve anchor within the proximal sheath lumen, the proximal sheath being proximally retractable relative to the core member for permitting expansion of the valve anchor; a distal carrier assembly comprising a distal enclosure and a proximal enclosure, the distal enclosure being coupled to the core member and comprising a distal lumen, the distal enclosure being configured to receive a distal portion of the valve frame within the distal lumen in a compressed configuration, the proximal enclosure being coupled to the core member proximal to the distal enclosure and comprising a proximal lumen, the proximal enclosure being configured to receive a proximal portion of the valve frame within the proximal lumen in a compressed configuration; and a plunger mechanism and a biasing means disposed within the distal lumen of the distal carrier assembly, the plunger mechanism being moveable between a distal position and a proximal position within the distal lumen, the plunger mechanism being biased toward the proximal position by the biasing means for urging the valve frame out of the distal lumen.

Clause 2. The delivery device of Clause 1, wherein the plunger mechanism comprises a plunger head that is moveable between the distal position and the proximal position within the distal lumen, the plunger head being biased toward the proximal position by the biasing means.

Clause 3. The delivery device of Clause 2, wherein in the proximal position, the plunger head extends at least partially proximally out of the distal lumen.

Clause 4. The delivery device of Clause 2, wherein in the proximal position, an outer surface of the plunger head is in contact with both the inner surface of the distal lumen and an inner surface of the proximal lumen when the proximal enclosure is distally advanced over the plunger head.

Clause 5. The delivery device of Clause 2, further comprising the valve prosthesis, wherein when the valve frame is positioned within the distal lumen, the plunger head is urged to the distal position.

Clause 6. The delivery device of Clause 2, wherein the biasing means comprises a spring.

Clause 7. The delivery device of Clause 6, wherein the biasing means comprises a spring, the spring being interposed between a proximal face of the distal enclosure and a distal face of the plunger head.

Clause 8. The delivery device of any preceding Clause wherein the plunger mechanism comprises a proximal portion that tapers in a proximal direction.

Clause 9. The delivery device of Clause 8, wherein the plunger mechanism comprises a plunger head with a plurality of petals extending from the plunger head, wherein the plurality of petals each form a ramped surface.

Clause 10. The delivery device of Clause 9, wherein the plurality of petals are configured to deflect radially inward within the distal lumen.

Clause 11. The delivery device of Clause 9, wherein the plurality of petals are configured to deflect radially outward when a portion of the plurality of petals exits the distal enclosure.

Clause 12. The delivery device of Clause 11, wherein in the proximal position, the plunger mechanism extends at least partially proximally out of the distal lumen, the proximal enclosure being distally advanceable over the ramped surfaces of the plurality of petals to align an outer surface of the distal enclosure with an outer surface of the proximal enclosure for facilitating removal of the delivery device from a patient.

Clause 13. A valve prosthesis delivery device for delivering a valve prosthesis, the valve prosthesis comprising a valve frame and a valve anchor, the delivery device comprising: an elongate core member extending along a longitudinal axis of the system; a sheath component slidably coupled to the core member and comprising a sheath lumen, the sheath component being configured to receive at least a portion of the valve anchor within the sheath lumen, the sheath being moveable relative to the core member for permitting expansion of the valve anchor; a plurality of grasper components disposed within the sheath component and extending along the elongate core member, wherein a distal end of the plurality of grasper components each engage with the valve anchor for permitting expansion of the valve anchor; and a distal hub disposed near a distal end of the elongate core member, wherein the distal hub is coupled to the plurality of grasper components to align each of the distal ends of the plurality of grasper components in a common plane as the sheath component is bent.

Clause 14. The valve prosthesis delivery device of Clause 13, wherein the distal hub is bonded to a tubular enclosure of each of the plurality of grasper components.

Clause 15. The valve prosthesis delivery device of Clause 13 or 14, wherein the distal hub comprises a plurality of passages to permit the plurality of grasper components to pass therethrough.

Clause 16. The valve prosthesis delivery device of Clause 13-15, wherein the distal hub comprises a core member passage to permit the elongate core member to pass therethrough.

Clause 17. The valve prosthesis delivery device of Clause 16, wherein the core member passage is offset from a central axis of the distal hub.

Clause 18. The valve prosthesis delivery device of Clause 13-17, wherein the distal hub is located approximately 1 to 4 inches from the distal end of at least one of the plurality of grasper components.

Clause 19. The valve prosthesis delivery device of Clause 13-18, wherein the distal hub is movable relative to the elongate core member.

Clause 20. The valve prosthesis delivery device of Clause 13-19, wherein the distal hub comprises a generally cylindrical shape.

### Clause Set 5: Handle for the Delivery Device

A valve delivery device comprising: a distal assembly comprising: a core member extending along a longitudinal axis of the assembly; a distal carrier assembly comprising a distal enclosure and a proximal enclosure, the distal enclosure being coupled to the core member and configured to cover a distal portion of a valve frame of a valve prosthesis, the proximal enclosure being slidably coupled to the core member and configured to cover a proximal portion of the valve frame: a proximal sheath, proximal to the distal carrier assembly, extending along the core member and configured to cover at least a portion of a valve anchor of the valve prosthesis; and a control unit comprising a control handle, coupled to the core member, and configured to control an operation of the distal assembly, the control unit further comprising: a first control element coupled to the control handle and configured to deflect the distal carrier assembly for steering the delivery device during advancement into a body lumen; a second control element coupled to the control handle and configured to actuate the proximal sheath for proximally retracting the proximal sheath relative to the distal carrier assembly and the valve anchor for exposing the valve anchor; and a third control element coupled to the control handle and configured to actuate a grasper mechanism for longitudinally moving the valve anchor relative to the core member and for controlling engagement with the valve anchor and the distal carrier assembly for permitting expansion of the valve frame.

Clause 2. The delivery device of Clause 1, wherein the core member comprises a hollow shaft.

Clause 3. The delivery device of Clause 2, wherein the shaft comprises a shaft lumen.

Clause 4. The delivery device of any preceding Clause, wherein actuation of the first control element axially deflects the distal carrier assembly relative to the core member.

Clause 5. The delivery device of any preceding Clause, wherein the first control element is actuatable via rotation about a longitudinal axis of the control unit.

Clause 6. The delivery device of any preceding Clause, wherein the first control element comprises a knob that is rotatable about a longitudinal axis of the control unit.

Clause 7. The delivery device of any preceding Clause, wherein the second control element is actuatable via rotation about a longitudinal axis of the control unit.

Clause 8. The delivery device of Clause 7, wherein rotation of the second control element distally disengages the grasper mechanism from the valve anchor.

Clause 9. The delivery device of any preceding Clause, wherein the second control element comprises a knob that is rotatable about a longitudinal axis of the control unit and translation along the control handle.

Clause 10. The delivery device of any preceding Clause, wherein the third control element is actuatable via translation along the control handle

Clause 11. The delivery device of Clause 10, wherein distal translation of the third control element distally advances the grasper mechanism relative to the proximal sheath to facilitate manipulation of the valve anchor.

Clause 12. The delivery device of any preceding Clause, wherein the third control element comprises a knob that is rotatable about and translatable along a longitudinal axis of the control unit to control the grasper mechanism

Clause 13. The delivery device of any preceding Clause, wherein the grasper mechanism comprises a plurality of grasper arms coupled to a respective engagement area of the valve anchor.

Clause 14. The delivery device of any preceding Clause, wherein the valve anchor and the valve frame are movably attached and moveable between a disengaged position and an engaged position, the disengaged position allowing variable range of movement between the valve frame and the valve anchor, the engaged position providing a fixed range of movement between the valve frame and the valve anchor.

Clause 15. The delivery device of any preceding Clause, wherein rotation of the third control element axially translates the proximal enclosure of the distal carrier assembly relative to the core member for exposing the valve frame.

Clause 16. The delivery device of Clause 15, wherein the distal enclosure is disposed distal to the proximal enclosure, wherein the rotation of the third control element axially translates the proximal enclosure in a proximal direction relative to the distal enclosure.

Clause 17. The delivery device of Clause 15, wherein the distal carrier assembly comprises a plunger mechanism and the distal enclosure of the distal carrier assembly has a distal lumen, the plunger mechanism being slidable within the distal lumen of the distal enclosure to urge the distal portion of the valve frame proximally out of the distal lumen after the proximal enclosure is moved proximally relative to the distal enclosure.

Clause 18. The delivery device of Clause 15, wherein rotation of the third control element in a first direction axially translates the distal enclosure in a first direction relative to the core member.

Clause 19. The delivery device of Clause 18, wherein the first direction is a proximal relative to the core member.

Clause 20. The delivery device of Clause 18, wherein rotation of the third control element in a second direction axially translates the distal enclosure in a second direction relative to the core member.

Clause 21. The delivery device of Clause 20, wherein the second direction is a distal relative to the core member.

Clause 22. The delivery device of Clause 20, wherein the rotation of the third control element in the first direction axially separates the proximal enclosure from the distal enclosure, and wherein rotation of the third control element in the second direction axially draws the proximal enclosure toward the distal enclosure.

Clause 23. The delivery device of any preceding Clause, wherein the first, second, and third control elements are positioned along an outer surface of the control handle.

Clause 24. The delivery device of any preceding Clause, wherein at least one of the first, second, or third control elements comprises a knob.

Clause 25. The delivery device of any preceding Clause, wherein the first control element is positioned distal to the second control element along the control handle.

Clause 26. The delivery device of any preceding Clause, further comprising the valve prosthesis having the valve frame and the valve anchor.

Clause 27. The delivery device of any preceding Clause, wherein in a loaded configuration, a proximal end of the distal enclosure is longitudinally spaced apart from a distal end of the proximal enclosure.

Clause 28. A method of controlling a valve delivery device for delivering a valve prosthesis using a control unit, comprising: controlling a first control element coupled to the control unit to cause a portion of a core member of the delivery device to bend, wherein a proximal sheath of the valve delivery device extends along the core member and is configured to cover at least a portion of a valve anchor of the valve prosthesis; controlling a second control element coupled to the control unit to proximally retract the proximal sheath relative to the valve anchor to expose the valve anchor and to control engagement with the valve anchor; and controlling a third control element coupled to the control unit to move the valve anchor relative to the core member and to actuate a distal carrier assembly of the valve delivery device for permitting expansion of a valve frame of the valve prosthesis, the distal carrier assembly covering at least a portion of the valve frame to maintain the valve frame in a compressed configuration.

Clause 29. The method of Clause 28, wherein the controlling of the first control element to cause the portion of the core member of the delivery device to bend comprises controlling the first control element to axially deflect the distal carrier assembly relative to the core member to bend the portion of the core member of the delivery device.

Clause 30. The method of Clause 28 or 29, wherein the first control element is controllable via rotation about a longitudinal axis of the control unit.

Clause 31. The method of Clause 28-30, wherein the first control element comprises a knob that is rotatable about a longitudinal axis of the control unit.

Clause 32. The method of Clause 28-31, wherein the second control element is controllable via rotation about a longitudinal axis of the control unit.

Clause 33. The method of Clause 28-32, wherein the second control element comprises a knob that is rotatable about a longitudinal axis of the control unit.

Clause 34. The method of Clause 33, wherein rotation of the second control element distally disengages a grasper mechanism from the valve anchor, the grasper mechanism being used for longitudinally moving the valve anchor relative to the core member and for controlling engagement with the valve anchor.

Clause 35. The method of Clause 34, wherein the second control element comprises a knob that is rotatable about and translatable along a longitudinal axis of the control unit to control the grasper mechanism.

Clause 36. The method of Clause 34, wherein the grasper mechanism comprises a plurality of grasper arms coupled to a respective engagement area of the valve anchor.

Clause 37. The method of Clause 28-36, wherein the valve anchor and the valve frame are movably attached and moveable between a disengaged position and an engaged position, the disengaged position allowing variable range of movement between the valve frame and the valve anchor, the engaged position providing a fixed range of movement between the valve frame and the valve anchor.

Clause 38. The method of Clause 28-37, wherein the third control element is controllable via translation of the third control element along a control handle coupled to the third control element.

Clause 39. The method of Clause 38, wherein distal translation of the third control element distally advances a grasper mechanism relative to the proximal sheath to facilitate manipulation of the valve anchor, the grasper mechanism being used for longitudinally moving the valve anchor relative to the core member and for controlling engagement with the valve anchor.

Clause 40. The method of Clause 28-39, wherein the third control element is controllable via rotation.

Clause 41. The method of Clause 40, wherein the rotation of the third control element axially translates a proximal enclosure of the distal carrier assembly relative to the core member for exposing the valve frame, the proximal enclosure being slidably coupled to the core member and configured to cover a proximal portion of the valve frame.

Clause 42. The method of Clause 41, wherein the distal carrier assembly comprises a distal enclosure disposed distal to the proximal enclosure, the distal enclosure being coupled to the core member and configured to cover a distal portion of a valve frame of a valve prosthesis, wherein the rotation of the third control element axially translates the proximal enclosure in a proximal direction relative to the distal enclosure.

Clause 43. The method of Clause 42, wherein the distal carrier assembly comprises a plunger mechanism and a distal enclosure having a distal lumen, the plunger mechanism being slidable within the distal lumen of the distal enclosure to urge the distal portion of the valve frame proximally out of the distal lumen after the proximal enclosure is moved proximally relative to the distal enclosure.

Clause 44. The method of Clause 41, wherein rotation of the third control element in a first direction axially translates the distal enclosure in a first direction relative to the core member.

Clause 45. The method of Clause 44, wherein the first direction is a proximal relative to the core member.

Clause 46. The method of Clause 44, wherein rotation of the third control element in a second direction axially translates the distal enclosure in a second direction relative to the core member.

Clause 47. The method of Clause 46, wherein the second direction is a distal relative to the core member.

Clause 48. The method of Clause 46, wherein the rotation of the third control element in the first direction axially separates the proximal enclosure from the distal enclosure, and wherein rotation of the third control element in the second direction axially draws the proximal enclosure toward the distal enclosure.

Clause 49. The method of Clause 28-48, wherein the first, second, and third control elements are positioned along an outer surface of a control handle.

Clause 50. The method of Clause 49, wherein at least one of the first, second, or third control elements comprises a knob.

Clause 51. The method of Clause 49, wherein the first control element is positioned distal to the second control element along the control handle.

Clause 52. The method of Clause 28-51, further comprising a valve prosthesis having the valve frame and the valve anchor.

Clause 53. The method of Clause 28-52, wherein the distal carrier assembly comprises a distal enclosure and a proximal enclosure, and wherein in a loaded configuration, a proximal end of the distal enclosure is longitudinally spaced apart from a distal end of the proximal enclosure.

Clause 54. A control unit for controlling a valve delivery device to deliver a valve prosthesis, the control unit comprising: a control handle coupled to a core member of the valve delivery device and configured to control an operation of the valve delivery device; a first control element configured to cause a portion of a core member of the delivery device to bend, wherein a proximal sheath of the valve delivery device extends along the core member and is configured to cover at least a portion of a valve anchor of the valve prosthesis; a second control element configured to proximally retract the proximal sheath relative to the valve anchor to expose the valve anchor and to control engagement with the valve anchor; and a third control element configured to move the valve anchor relative to the core member and to actuate a distal carrier assembly of the valve delivery device for permitting expansion of a valve frame of the valve prosthesis by actuating distal carrier assembly covering at least a portion of the valve frame to maintain the valve frame in a compressed configuration.

Clause 55. The control unit of Clause 54, wherein the core member comprises a hollow shaft.

Clause 56. The control unit of Clause 55, wherein the shaft comprises a shaft lumen.

Clause 57. The control unit of Clause 54-56, wherein the controlling of the first control element to cause the portion of the core member of the delivery device to bend comprises controlling the first control element to axially deflect the distal carrier assembly relative to the core member to bend the portion of the core member of the delivery device

Clause 58. The control unit of Clause 54-57, wherein the first control element is controllable via rotation about a longitudinal axis of the control unit.

Clause 59. The control unit of Clause 54-58, wherein the first control element comprises a knob that is rotatable about a longitudinal axis of the control unit.

Clause 60. The control unit of Clause 54-59, wherein the second control element is controllable via rotation about a longitudinal axis of the control unit.

Clause 61. The control unit of Clause 54-60, wherein the second control element comprises a knob that is rotatable about a longitudinal axis of the control unit.

Clause 62. The control unit of Clause 61, wherein rotation of the second control element distally disengages a grasper mechanism from the valve anchor, the grasper mechanism being used for longitudinally moving the valve anchor relative to the core member and for controlling engagement with the valve anchor.

Clause 63. The control unit of Clause 62, wherein the second control element comprises a knob that is rotatable about and translatable along a longitudinal axis of the control unit to control the grasper mechanism.

Clause 64. The control unit of Clause 62, wherein the grasper mechanism comprises a plurality of grasper arms coupled to a respective engagement area of the valve anchor.

Clause 65. The control unit of Clause 54-64, wherein the valve anchor and the valve frame are movably attached and moveable between a disengaged position and an engaged position, the disengaged position allowing variable range of movement between the valve frame and the valve anchor, the engaged position providing a fixed range of movement between the valve frame and the valve anchor.

Clause 66. The control unit of Clause 54-65, wherein the third control element is controllable via translation of the third control element along the control handle.

Clause 67. The control unit of Clause 66, wherein distal translation of the third control element distally advances a grasper mechanism relative to the proximal sheath to facilitate manipulation of the valve anchor, the grasper mechanism being used for longitudinally moving the valve anchor relative to the core member and for controlling engagement with the valve anchor.

Clause 68. The control unit of Clause 54-67, wherein the third control element is controllable via rotation about a longitudinal axis of the control unit.

Clause 69. The control unit of Clause 68, wherein the rotation of the third control element axially translates a proximal enclosure of the distal carrier assembly relative to the core member for exposing the valve frame, the proximal enclosure being slidably coupled to the core member and configured to cover a proximal portion of the valve frame.

Clause 70. The control unit of Clause 69, wherein the distal carrier assembly comprises a distal enclosure disposed distal to the proximal enclosure, the distal enclosure being coupled to the core member and configured to cover a distal portion of a valve frame of a valve prosthesis, wherein the rotation of the third control element axially translates the proximal enclosure in a proximal direction relative to the distal enclosure.

Clause 71. The control unit of Clause 70, wherein the distal carrier assembly comprises a plunger mechanism and a distal enclosure having a distal lumen, the plunger mechanism being slidable within the distal lumen of the distal enclosure to urge the distal portion of the valve frame proximally out of the distal lumen after the proximal enclosure is moved proximally relative to the distal enclosure.

Clause 72. The control unit of Clause 69, wherein rotation of the third control element in a first direction axially translates the distal enclosure in a first direction relative to the core member.

Clause 73. The control unit of Clause 72, wherein the first direction is a proximal relative to the core member.

Clause 74. The control unit of Clause 72, wherein rotation of the third control element in a second direction axially translates the distal enclosure in a second direction relative to the core member.

Clause 75. The control unit of Clause 74, wherein the second direction is a distal relative to the core member.

Clause 76. The control unit of Clause 74, wherein the rotation of the third control element in the first direction axially separates the proximal enclosure from the distal enclosure, and wherein rotation of the third control element in the second direction axially draws the proximal enclosure toward the distal enclosure.

Clause 77. The control unit of Clause 54-76, wherein the first, second, and third control elements are positioned along an outer surface of the control handle.

Clause 78. The control unit of Clause 54-77, wherein at least one of the first, second, or third control elements comprises a knob.

Clause 79. The control unit of Clause 54-78, wherein the first control element is positioned distal to the second control element along the control handle.

Clause 80. The control unit of Clause 54-79, further comprising a valve prosthesis having the valve frame and the valve anchor.

Clause 81. The control unit of Clause 54-80, wherein the distal carrier assembly comprises a distal enclosure and a proximal enclosure, and wherein in a loaded configuration, a proximal end of the distal enclosure is longitudinally spaced apart from a distal end of the proximal enclosure.

### Clause Set 6: Methods for Delivery

Clause 1. A method for delivering a prosthetic heart valve prosthesis to a native valve structure of a patient, the valve prosthesis comprising a valve frame and a valve anchor, the method comprising: introducing the valve prosthesis into the patient at an implantation site via a valve prosthesis delivery device, the device comprising a proximal sheath component and a distal carrier assembly, the proximal sheath component receiving at least a portion of the valve anchor in a proximal sheath lumen, and the distal carrier assembly comprising a distal enclosure and a proximal enclosure, the distal enclosure being configured to receive at least a distal portion of the valve frame; advancing the valve prosthesis to the implantation site via an aorta; proximally retracting the proximal sheath in a proximal direction to permit expansion of the valve anchor; expanding the distal carrier assembly to permit expansion of the valve frame; and distally urging a base portion of the valve anchor into engagement with a native valve structure.

Clause 2. The method of Clause 1, further comprising advancing the valve prosthesis in a retrograde direction.

Clause 3. The method of any preceding Clause, further comprising proximally retracting the valve frame relative to the valve anchor to engage a link mechanism therebetween and restricting a range of movement of the valve frame relative to the valve anchor.

Clause 4. The method of Clause 3, further comprising distally advancing the valve frame.

Clause 5. The method of any preceding Clause, wherein the expanding longitudinally separating the distal enclosure from the proximal enclosure to permit expansion of the distal portion of the valve frame.

Clause 6. The method of any preceding Clause, wherein the expanding comprises distally advancing the distal enclosure relative to the proximal enclosure to permit expansion of the distal portion of the valve frame.

Clause 7. The method of any preceding Clause, wherein the expanding comprises proximally retracting the proximal enclosure relative to the distal enclosure to permit expansion of a proximal portion of the valve frame.

Clause 8. The method of any preceding Clause, wherein the expanding comprises proximally retracting the distal enclosure relative to the proximal enclosure and distally advancing the proximal enclosure relative to the distal enclosure to move the distal carrier assembly to a retrieval configuration for retracting the valve prosthesis delivery device from the patient.

Clause 9. A method for delivering a prosthetic heart valve prosthesis to a native valve structure of a patient, the valve prosthesis comprising a valve frame and a valve anchor, the method comprising: introducing the valve prosthesis into the patient at an implantation site via a valve prosthesis delivery device, the device comprising a proximal sheath component and a distal carrier assembly, the proximal sheath component receiving at least a portion of the valve anchor in a proximal sheath lumen, and the distal carrier assembly comprising a distal enclosure and a proximal enclosure, the distal enclosure being configured to receive at least a distal portion of the valve frame; advancing the valve prosthesis to the implantation site via an apex of a heart of the patient; proximally retracting the proximal sheath in a proximal direction to permit expansion of the valve anchor; expanding the distal carrier assembly to permit expansion of the valve frame; and proximally urging a base portion of the valve anchor into engagement with a native valve structure.

Clause 10. The method of Clause 9, further comprising advancing the valve prosthesis in a antegrade direction.

Clause 11. The method of Clause 9 or 10, further comprising proximally retracting the valve frame relative to the valve anchor to engage a link mechanism therebetween and restricting a range of movement of the valve frame relative to the valve anchor.

Clause 12. The method of Clause 11, further comprising distally advancing the valve frame.

Clause 13. The method of Clause 9-12, wherein the expanding longitudinally separating the distal enclosure from the proximal enclosure to permit expansion of the distal portion of the valve frame.

Clause 14. The method of Clause 9-13, wherein the expanding comprises distally advancing the distal enclosure relative to the proximal enclosure to permit expansion of the distal portion of the valve frame.

Clause 15. The method of Clause 9-14, wherein the expanding comprises proximally retracting the proximal enclosure relative to the distal enclosure to permit expansion of the proximal portion of the valve frame.

Clause 16. The method of Clause 9-15, wherein the expanding comprises proximally retracting the distal enclosure relative to the proximal enclosure and distally advancing the proximal enclosure relative to the distal enclosure to move the distal carrier assembly to a retrieval configuration for retracting the valve prosthesis delivery device from the patient.

### Further Considerations

In some embodiments, any of the clauses herein may depend from any one of the independent clauses or any one of the dependent clauses. In some embodiments, any of the clauses (e.g., dependent or independent clauses) may be combined with any other one or more clauses (e.g., dependent or independent clauses). In some embodiments, a claim may include some or all of the words (e.g., steps, operations, means or components) recited in a clause, a sentence, a phrase or a paragraph. In some embodiments, a claim may include some or all of the words recited in one or more clauses, sentences, phrases or paragraphs. In some embodiments, some of the words in each of the clauses, sentences, phrases or paragraphs may be removed. In some embodiments, additional words or elements may be added to a clause, a sentence, a phrase or a paragraph. In some embodiments, the subject technology may be implemented without utilizing some of the components, elements, functions or operations described herein. In some embodiments, the subject technology may be implemented utilizing additional components, elements, functions or operations.

The foregoing description is provided to enable a person skilled in the art to practice the various configurations described herein. While the subject technology has been particularly described with reference to the various figures and configurations, it should be understood that these are for illustration purposes only and should not be taken as limiting the scope of the subject technology.

There may be many other ways to implement the subject technology. Various functions and elements described herein may be partitioned differently from those shown without departing from the scope of the subject technology. Various modifications to these configurations will be readily apparent to those skilled in the art, and generic principles defined herein may be applied to other configurations. Thus, many changes and modifications may be made to the subject technology, by one having ordinary skill in the art, without departing from the scope of the subject technology.

It is understood that the specific order or hierarchy of steps in the processes disclosed is an illustration of exemplary approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

As used herein, the term "distal" can denote a location or direction that is away from a point of interest, such as a control unit or region of the delivery system that will be used to deliver a valve prosthesis to a native valve annulus. Additionally, the term "proximal" can denote a location or direction that is closer to a point of interest, such as a control unit or region of the delivery system that will be used to deliver a valve prosthesis.

As used herein, the phrase "at least one of" preceding a series of items, with the term "and" or "or" to separate any of the items, modifies the list as a whole, rather than each member of the list (i.e., each item). The phrase "at least one of" does not require selection of at least one of each item listed: rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Terms such as "top," "bottom," "front," "rear" and the like as used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Furthermore, to the extent that the term "include," "have," or the like is used in the description or the claims, such term is intended to be inclusive in a manner similar to the term "comprise" as "comprise" is interpreted when employed as a transitional word in a claim.

The word "exemplary" is used herein to mean "serving as an example, instance, or illustration." Any embodiment described herein as "exemplary" is not necessarily to be construed as preferred or advantageous over other embodiments.

A reference to an element in the singular is not intended to mean "one and only one" unless specifically stated, but rather "one or more." Pronouns in the masculine (e.g., his) include the feminine and neuter gender (e.g., her and its) and vice versa. The term "some" refers to one or more. Underlined and/or italicized headings and subheadings are used for convenience only, do not limit the subject technology, and are not referred to in connection with the interpretation of the description of the subject technology. All structural and functional equivalents to the elements of the various configurations described throughout this disclosure that are known or later come to be known to those of ordinary skill in the art are expressly incorporated herein by reference and intended to be encompassed by the subject technology. Moreover, nothing disclosed herein is intended to be dedicated to the public regardless of whether such disclosure is explicitly recited in the above description.

Although the detailed description contains many specifics, these should not be construed as limiting the scope of the subject technology but merely as illustrating different examples and aspects of the subject technology. It should be appreciated that the scope of the subject technology includes other embodiments not discussed in detail above. Various other modifications, changes and variations may be made in the arrangement, operation and details of the method and apparatus of the subject technology disclosed herein without departing from the scope of the present disclosure. Unless otherwise expressed, reference to an element in the singular is not intended to mean "one and only one" unless explicitly stated, but rather is meant to mean "one or more." In addition, it is not necessary for a device or method to address every problem that is solvable (or possess every advantage that is achievable) by different embodiments of the disclosure in order to be encompassed within the scope of the disclosure. The use herein of "can" and derivatives thereof shall be understood in the sense of "possibly" or "optionally" as opposed to an affirmative capability.

## Claims

1. A delivery system for delivering a prosthetic heart valve prosthesis to a native valve sinus of a patient, the valve prosthesis comprising a mesh frame and a sinus locator, the delivery system comprising:
a grasper configured to be coupled to a proximal portion of the sinus locator of the valve prosthesis; and
a distal sheath configured to receive a distal portion of the mesh frame, the distal sheath having a proximal edge that overlaps the mesh frame; and
a proximal sheath configured to receive a proximal portion of the mesh frame, the proximal sheath having a distal edge that overlaps the mesh frame and that is spaced apart from the proximal edge of the distal sheath to form a longitudinal gap that permits a section of the mesh frame to be exposed therethrough between the proximal and distal edges,
wherein the delivery system supports the prosthesis in a delivery configuration wherein (i) the sinus locator is engaged with the grasper and (ii) the proximal and distal sheaths support the mesh frame in a collapsed state with a medial portion of the mesh frame being exposed through the longitudinal gap, the delivery system being configured to permit expansion of the sinus locator and to subsequently permit expansion of the mesh frame within the sinus locator upon separation of the proximal sheath from the distal sheath, the grasper being configured to disengage from the proximal portion of the sinus locator for releasing the valve prosthesis.

2. The system of Claim 1, wherein the grasper comprises a tubular member configured to receive the proximal portion of the sinus locator therewithin when engaged with the sinus locator.

3. The system of any of the preceding Claims, wherein the longitudinal gap exposes about 1/4 of the longitudinal length of the mesh frame therethrough.

4. The system of any of the preceding Claims, wherein the medial portion of the mesh frame comprises about 1/4 of the longitudinal length of the mesh frame.

5. The system of any of the preceding Claims, further comprising a plunger configured to urge the distal portion of the mesh frame out of the distal sheath.

6. The system of Claim 5, wherein the plunger is configured to urge the distal portion of the mesh frame out of the distal sheath while the distal sheath is stationary relative to the proximal sheath.

7. The system of any of Claims 5 or 6, wherein the plunger comprises a spring configured to expand from a compressed state to an extended state, thereby proximally urging the distal portion of the mesh frame.

8. A system for delivering a prosthetic heart valve prosthesis to a native valve sinus of a patient, the system comprising:
a valve prosthesis comprising a mesh frame and a sinus locator; and
a valve prosthesis delivery device having a grasper component, a proximal sheath, and a distal sheath, wherein, in a delivery configuration, (i) the grasper component engages with a proximal portion of the sinus locator, (ii) the proximal sheath radially restrains a proximal portion of the mesh frame, and (iii) the distal sheath radially restrains a distal portion of the mesh frame and the proximal and distal sheaths are longitudinally spaced apart from each other to form a longitudinal gap therebetween to expose a medial portion of the mesh frame through the longitudinal gap when in the delivery configuration, the proximal and distal sheaths being actuatable to widen the longitudinal gap for permitting the mesh frame to be released therefrom.

9. The system of Claim 8, wherein the proximal sheath is longitudinally movable relative to the grasper component while the grasper component remains engaged with the proximal portion of the sinus locator.

10. The system of any of Claims 8 or 9, wherein the proximal and distal sheaths comprise tubular components.

11. The system of any of Claims 8 to 10, further comprising a plunger configured to urge the distal portion of the mesh frame out of the distal sheath.

12. The system of Claim 11, wherein the plunger is configured to urge the distal portion of the mesh frame out of the distal sheath while the distal sheath is stationary relative to the proximal sheath.

13. The system of any of Claims 11 or 12, wherein the plunger comprises a spring configured to expand from a compressed state to an extended state, thereby proximally urging the distal portion of the mesh frame.

14. The system of any of Claims 8 or 13, wherein the grasper component comprises a tubular member configured to receive the proximal portion of the sinus locator therewithin when engaged with the sinus locator.

15. The system of any of Claims 8 or 13, wherein the longitudinal gap exposes about 1/4 of the longitudinal length of the mesh frame therethrough.

16. The system of any of Claims 8 or 13, wherein the medial portion of the mesh frame comprises about 1/4 of the longitudinal length of the mesh frame.
